# EUROPEAN PATENT SPECIFICATION

(11) **EP 2 764 362 B1**
(45) Date of publication and mention of the grant of the patent: **05.09.2018**
(21) Application number: 12839048.1
(22) Date of filing: 02.10.2012
(51) Int. Cl.: G01N 33/533, G01N 21/76

(54) **DYE CONJUGATED PEPTIDES FOR FLUORESCENT IMAGING**
FARBSTOFFKONJUGIERTE PEPTIDE FÜR BILDGEBUNG MIT FLUORESZENZ
PEPTIDES CONJUGUÉS À UN COLORANT POUR IMAGERIE PAR FLUORESCENCE

(30) Priority: 03.10.2011 US 201161542539 P; 30.05.2012 US 201213483761; 16.07.2012 US 201213549906; 20.08.2012 US 201213589575
(43) Date of publication of application: 13.08.2014
(73) Proprietor: Immunomedics, Inc., Morris Plains, NJ 07950 (US)
(72) Inventor: REGINO, Celeste Aida S., Morris Plains New Jersey 07950 (US); MCBRIDE, William J., Morris Plains New Jersey 07950 (US); CHANG, Chien-Hsing, Morris Plains New Jersey 07950 (US); GOLDENBERG, David M., Morris Plains New Jersey 07950 (US)
(74) Representative: Bohmann, Armin K.
(86) International application number: PCT/US2012/058462
(87) International publication number: WO 2013/052484

(56) References cited:
- US-A1- 2007 086 942
- US-A1- 2011 158 905
- US-A1- 2011 165 072

### SEQUENCE LISTING

<110> IMMUNOMEDICS, INC.
<120> DYE CONJUGATED PEPTIDES FOR FLUORESCENT IMAGING
<130> IMM336WO1
<140>
   <141>
<150> 13/589,575
   <151> 2012-08-20
<150> 13/549,906
   <151> 2012-07-16
<150> 13/483,761
   <151> 2012-05-30
<150> 61/542,539
   <151> 2011-10-03
<160> 100
<170> PatentIn version 3.5
<210> 1
   <211> 44
   <212> PRT
   <213> Artificial Sequence
<220>
   <223> Description of Artificial Sequence: Synthetic polypeptide
<400> 1
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 2
   <211> 45
   <212> PRT
   <213> Artificial Sequence
<220>
   <223> Description of Artificial Sequence: Synthetic polypeptide
<400> 2
   Cys Gly His Ile Gln Ile Pro Pro Gly Leu Thr Glu Leu Leu Gln Gly
   1               5                   10                  15
   Tyr Thr Val Glu Val Leu Arg Gln Gln Pro Pro Asp Leu Val Glu Phe
               20                  25                  30
   Ala Val Glu Tyr Phe Thr Arg Leu Arg Glu Ala Arg Ala
           35                  40                  45
<210> 3
   <211> 17
   <212> PRT
   <213> Artificial Sequence
<220>
   <223> Description of Artificial Sequence: Synthetic peptide
<400> 3
   Gln Ile Glu Tyr Leu Ala Lys Gln Ile Val Asp Asn Ala Ile Gln Gln
   1               5                   10                  15
   Ala
<210> 4
   <211> 21
   <212> PRT
   <213> Artificial Sequence
<220>
   <223> Description of Artificial Sequence: Synthetic peptide
<400> 4
   Cys Gly Gln Ile Glu Tyr Leu Ala Lys Gln Ile Val Asp Asn Ala Ile
   1               5                   10                  15
   Gln Gln Ala Gly Cys
               20
<210> 5
   <211> 50
   <212> PRT
   <213> Artificial Sequence
<220>
   <223> Description of Artificial Sequence: Synthetic polypeptide
<400> 5
   Ser Leu Arg Glu Cys Glu Leu Tyr Val Gln Lys His Asn Ile Gln Ala
   1               5                   10                  15
   Leu Leu Lys Asp Ser Ile Val Gln Leu Cys Thr Ala Arg Pro Glu Arg
               20                  25                  30
   Pro Met Ala Phe Leu Arg Glu Tyr Phe Glu Arg Leu Glu Lys Glu Glu
                  35                  40                  45
   Ala Lys
       50
<210> 6
   <211> 55
   <212> PRT
   <213> Artificial Sequence
<220>
   <223> Description of Artificial Sequence: Synthetic polypeptide
<400> 6
   Met Ser Cys Gly Gly Ser Leu Arg Glu Cys Glu Leu Tyr Val Gln Lys
   1               5                   10                  15
   His Asn Ile Gln Ala Leu Leu Lys Asp Ser Ile Val Gln Leu Cys Thr
               20                  25                  30
   Ala Arg Pro Glu Arg Pro Met Ala Phe Leu Arg Glu Tyr Phe Glu Arg
           35                  40                  45
   Leu Glu Lys Glu Glu Ala Lys
       50                  55
<210> 7
   <211> 23
   <212> PRT
   <213> Artificial Sequence
<220>
   <223> Description of Artificial Sequence: Synthetic peptide
<400> 7
   Cys Gly Phe Glu Glu Leu Ala Trp Lys Ile Ala Lys Met Ile Trp Ser
   1               5                   10                  15
   Asp Val Phe Gln Gln Gly Cys
               20
<210> 8
   <211> 51
   <212> PRT
   <213> Homo sapiens
<400> 8
   Ser Leu Arg Glu Cys Glu Leu Tyr Val Gln Lys His Asn Ile Gln Ala
   1               5                   10                  15
   Leu Leu Lys Asp Val Ser Ile Val Gln Leu Cys Thr Ala Arg Pro Glu
               20                  25                  30
   Arg Pro Met Ala Phe Leu Arg Glu Tyr Phe Glu Lys Leu Glu Lys Glu
           35                  40                  45
   Glu Ala Lys
       50
<210> 9
   <211> 54
   <212> PRT
   <213> Homo sapiens
<400> 9
   Ser Leu Lys Gly Cys Glu Leu Tyr Val Gln Leu His Gly Ile Gln Gln
   1               5                   10                  15
   Val Leu Lys Asp Cys Ile Val His Leu Cys Ile Ser Lys Pro Glu Arg
               20                  25                  30
   Pro Met Lys Phe Leu Arg Glu His Phe Glu Lys Leu Glu Lys Glu Glu
           35                  40                  45
   Asn Arg Gln Ile Leu Ala
       50
<210> 10
   <211> 44
   <212> PRT
   <213> Homo sapiens
<400> 10
   Ser His Ile Gln Ile Pro Pro Gly Leu Thr Glu Leu Leu Gln Gly Tyr
   1               5                   10                  15
   Thr Val Glu Val Gly Gln Gln Pro Pro Asp Leu Val Asp Phe Ala Val
               20                  25                  30
   Glu Tyr Phe Thr Arg Leu Arg Glu Ala Arg Arg Gln
           35                  40
<210> 11
   <211> 44
   <212> PRT
   <213> Homo sapiens
<400> 11
   Ser Ile Glu Ile Pro Ala Gly Leu Thr Glu Leu Leu Gln Gly Phe Thr
   1               5                   10                  15
   Val Glu Val Leu Arg His Gln Pro Ala Asp Leu Leu Glu Phe Ala Leu
               20                  25                  30
   Gln His Phe Thr Arg Leu Gln Gln Glu Asn Glu Arg
           35                  40
<210> 12
   <211> 44
   <212> PRT
   <213> Artificial Sequence
<220>
   <223> Description of Artificial Sequence: Synthetic polypeptide
<400> 12
   Thr His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 13
   <211> 44
   <212> PRT
   <213> Artificial Sequence
<220>
   <223> Description of Artificial Sequence: Synthetic polypeptide
<400> 13
   Ser Lys Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 14
   <211> 44
   <212> PRT
   <213> Artificial Sequence
<220>
   <223> Description of Artificial Sequence: Synthetic polypeptide
<400> 14
   Ser Arg Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 15
   <211> 44
   <212> PRT
   <213> Artificial Sequence
<220>
   <223> Description of Artificial Sequence: Synthetic polypeptide
<400> 15
   Ser His Ile Asn Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 16
   <211> 44
   <212> PRT
   <213> Artificial Sequence
<220>
   <223> Description of Artificial Sequence: Synthetic polypeptide
<400> 16
   Ser His Ile Gln Ile Pro Pro Ala Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 17
   <211> 44
   <212> PRT
   <213> Artificial Sequence
<220>
   <223> Description of Artificial Sequence: Synthetic polypeptide
<400> 17
   Ser His Ile Gln Ile Pro Pro Gly Leu Ser Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 18
   <211> 44
   <212> PRT
   <213> Artificial Sequence
<220>
   <223> Description of Artificial Sequence: Synthetic polypeptide
<400> 18
   Ser His Ile Gln Ile Pro Pro Gly Leu Thr Asp Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 19
   <211> 44
   <212> PRT
   <213> Artificial Sequence
<220>
   <223> Description of Artificial Sequence: Synthetic polypeptide
<400> 19
   Ser His Ile Gln Ile Pro Pro Gly Leu Thr Glu Leu Leu Asn Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 20
   <211> 44
   <212> PRT
   <213> Artificial Sequence
<220>
   <223> Description of Artificial Sequence: Synthetic polypeptide
<400> 20
   Ser His Ile Gln Ile Pro Pro Gly Leu Thr Glu Leu Leu Gln Ala Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 21
   <211> 44
   <212> PRT
   <213> Artificial Sequence
<220>
   <223> Description of Artificial Sequence: Synthetic polypeptide
<400> 21
   Ser His Ile Gln Ile Pro Pro Gly Leu Thr Glu Leu Leu Gln Gly Tyr
   1               5                   10                  15
   Ser Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 22
   <211> 44
   <212> PRT
   <213> Artificial Sequence
<220>
   <223> Description of Artificial Sequence: Synthetic polypeptide
<400> 22
   Ser His Ile Gln Ile Pro Pro Gly Leu Thr Glu Leu Leu Gln Gly Tyr
   1               5                   10                  15
   Thr Val Asp Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 23
   <211> 44
   <212> PRT
   <213> Artificial Sequence
<220>
   <223> Description of Artificial Sequence: Synthetic polypeptide
<400> 23
   Ser His Ile Gln Ile Pro Pro Gly Leu Thr Glu Leu Leu Gln Gly Tyr
   1               5                   10                  15
   Thr Val Glu Val Leu Lys Gln Gln Pro Pro Asp Leu Val Glu Phe Ala
               20                  25                  30
   Val Glu Tyr Phe Thr Arg Leu Arg Glu Ala Arg Ala
           35                  40
<210> 24
   <211> 44
   <212> PRT
   <213> Artificial Sequence
<220>
   <223> Description of Artificial Sequence: Synthetic polypeptide
<400> 24
   Ser His Ile Gln Ile Pro Pro Gly Leu Thr Glu Leu Leu Gln Gly Tyr
   1               5                   10                  15
   Thr Val Glu Val Leu Arg Asn Gln Pro Pro Asp Leu Val Glu Phe Ala
               20                  25                  30
   Val Glu Tyr Phe Thr Arg Leu Arg Glu Ala Arg Ala
           35                  40
<210> 25
   <211> 44
   <212> PRT
   <213> Artificial Sequence
<220>
   <223> Description of Artificial Sequence: Synthetic polypeptide
<400> 25
   Ser His Ile Gln Ile Pro Pro Gly Leu Thr Glu Leu Leu Gln Gly Tyr
   1               5                   10                  15
   Thr Val Glu Val Leu Arg Gln Asn Pro Pro Asp Leu Val Glu Phe Ala
               20                  25                  30
   Val Glu Tyr Phe Thr Arg Leu Arg Glu Ala Arg Ala
           35                  40
<210> 26
   <211> 44
   <212> PRT
   <213> Artificial Sequence
<220>
   <223> Description of Artificial Sequence: Synthetic polypeptide
<400> 26
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Glu Leu Val Glu Phe Ala
               20                  25                  30
   Val Glu Tyr Phe Thr Arg Leu Arg Glu Ala Arg Ala
           35                  40
<210> 27
   <211> 44
   <212> PRT
   <213> Artificial Sequence
<220>
   <223> Description of Artificial Sequence: Synthetic polypeptide
<400> 27
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Asp Phe Ala
               20                  25                  30
   Val Glu Tyr Phe Thr Arg Leu Arg Glu Ala Arg Ala
           35                  40
<210> 28
   <211> 44
   <212> PRT
   <213> Artificial Sequence
<220>
   <223> Description of Artificial Sequence: Synthetic polypeptide
<400> 28
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Leu
               20                  25                  30
   Val Glu Tyr Phe Thr Arg Leu Arg Glu Ala Arg Ala
           35                  40
<210> 29
   <211> 44
   <212> PRT
   <213> Artificial Sequence
<220>
   <223> Description of Artificial Sequence: Synthetic polypeptide
<400> 29
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ile
               20                  25                  30
   Val Glu Tyr Phe Thr Arg Leu Arg Glu Ala Arg Ala
           35                  40
<210> 30
   <211> 44
   <212> PRT
   <213> Artificial Sequence
<220>
   <223> Description of Artificial Sequence: Synthetic polypeptide
<400> 30
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Val
               20                  25                  30
   Val Glu Tyr Phe Thr Arg Leu Arg Glu Ala Arg Ala
           35                  40
<210> 31
   <211> 44
   <212> PRT
   <213> Artificial Sequence
<220>
   <223> Description of Artificial Sequence: Synthetic polypeptide
<400> 31
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Asp Tyr Phe Thr Arg Leu Arg Glu Ala Arg Ala
           35                  40
<210> 32
   <211> 17
   <212> PRT
   <213> Artificial Sequence
<220>
   <223> Description of Artificial Sequence: Synthetic peptide
<400> 32
   Asn Ile Glu Tyr Leu Ala Lys Gln Ile Val Asp Asn Ala Ile Gln Gln
   1               5                   10                  15
   Ala
<210> 33
   <211> 17
   <212> PRT
   <213> Artificial Sequence
<220>
   <223> Description of Artificial Sequence: Synthetic peptide
<400> 33
   Gln Leu Glu Tyr Leu Ala Lys Gln Ile Val Asp Asn Ala Ile Gln Gln
   1               5                   10                  15
   Ala
<210> 34
   <211> 17
   <212> PRT
   <213> Artificial Sequence
<220>
   <223> Description of Artificial Sequence: Synthetic peptide
<400> 34
   Gln Val Glu Tyr Leu Ala Lys Gln Ile Val Asp Asn Ala Ile Gln Gln
   1               5                   10                  15
   Ala
<210> 35
   <211> 17
   <212> PRT
   <213> Artificial Sequence
<220>
   <223> Description of Artificial Sequence: Synthetic peptide
<400> 35
   Gln Ile Asp Tyr Leu Ala Lys Gln Ile Val Asp Asn Ala Ile Gln Gln
   1               5                   10                  15
   Ala
<210> 36
   <211> 17
   <212> PRT
   <213> Artificial Sequence
<220>
   <223> Description of Artificial Sequence: Synthetic peptide
<400> 36
   Gln Ile Glu Phe Leu Ala Lys Gln Ile Val Asp Asn Ala Ile Gln Gln
   1               5                   10                  15
   Ala
<210> 37
   <211> 17
   <212> PRT
   <213> Artificial Sequence
<220>
   <223> Description of Artificial Sequence: Synthetic peptide
<400> 37
   Gln Ile Glu Thr Leu Ala Lys Gln Ile Val Asp Asn Ala Ile Gln Gln
   1               5                   10                  15
   Ala
<210> 38
   <211> 17
   <212> PRT
   <213> Artificial Sequence
<220>
   <223> Description of Artificial Sequence: Synthetic peptide
<400> 38
   Gln Ile Glu Ser Leu Ala Lys Gln Ile Val Asp Asn Ala Ile Gln Gln
   1               5                   10                  15
   Ala
<210> 39
   <211> 17
   <212> PRT
   <213> Artificial Sequence
<220>
   <223> Description of Artificial Sequence: Synthetic peptide
<400> 39
   Gln Ile Glu Tyr Ile Ala Lys Gln Ile Val Asp Asn Ala Ile Gln Gln
   1               5                   10                  15
   Ala
<210> 40
   <211> 17
   <212> PRT
   <213> Artificial Sequence
<220>
   <223> Description of Artificial Sequence: Synthetic peptide
<400> 40
   Gln Ile Glu Tyr Val Ala Lys Gln Ile Val Asp Asn Ala Ile Gln Gln
   1               5                   10                  15
   Ala
<210> 41
   <211> 17
   <212> PRT
   <213> Artificial Sequence
<220>
   <223> Description of Artificial Sequence: Synthetic peptide
<400> 41
   Gln Ile Glu Tyr Leu Ala Arg Gln Ile Val Asp Asn Ala Ile Gln Gln
   1               5                   10                  15
   Ala
<210> 42
   <211> 17
   <212> PRT
   <213> Artificial Sequence
<220>
   <223> Description of Artificial Sequence: Synthetic peptide
<400> 42
   Gln Ile Glu Tyr Leu Ala Lys Asn Ile Val Asp Asn Ala Ile Gln Gln
   1               5                   10                  15
   Ala
<210> 43
   <211> 17
   <212> PRT
   <213> Artificial Sequence
<220>
   <223> Description of Artificial Sequence: Synthetic peptide
<400> 43
   Gln Ile Glu Tyr Leu Ala Lys Gln Ile Val Glu Asn Ala Ile Gln Gln
   1               5                   10                  15
   Ala
<210> 44
   <211> 17
   <212> PRT
   <213> Artificial Sequence
<220>
   <223> Description of Artificial Sequence: Synthetic peptide
<400> 44
   Gln Ile Glu Tyr Leu Ala Lys Gln Ile Val Asp Gln Ala Ile Gln Gln
   1               5                   10                  15
   Ala
<210> 45
   <211> 17
   <212> PRT
   <213> Artificial Sequence
<220>
   <223> Description of Artificial Sequence: Synthetic peptide
<400> 45
   Gln Ile Glu Tyr Leu Ala Lys Gln Ile Val Asp Asn Ala Ile Asn Gln
   1               5                   10                  15
   Ala
<210> 46
   <211> 17
   <212> PRT
   <213> Artificial Sequence
<220>
   <223> Description of Artificial Sequence: Synthetic peptide
<400> 46
   Gln Ile Glu Tyr Leu Ala Lys Gln Ile Val Asp Asn Ala Ile Gln Asn
   1               5                   10                  15
   Ala
<210> 47
   <211> 17
   <212> PRT
   <213> Artificial Sequence
<220>
   <223> Description of Artificial Sequence: Synthetic peptide
<400> 47
   Gln Ile Glu Tyr Leu Ala Lys Gln Ile Val Asp Asn Ala Ile Gln Gln
   1               5                   10                  15
   Leu
<210> 48
   <211> 17
   <212> PRT
   <213> Artificial Sequence
<220>
   <223> Description of Artificial Sequence: Synthetic peptide
<400> 48
   Gln Ile Glu Tyr Leu Ala Lys Gln Ile Val Asp Asn Ala Ile Gln Gln
   1               5                   10                  15
   Ile
<210> 49
   <211> 17
   <212> PRT
   <213> Artificial Sequence
<220>
   <223> Description of Artificial Sequence: Synthetic peptide
<400> 49
   Gln Ile Glu Tyr Leu Ala Lys Gln Ile Val Asp Asn Ala Ile Gln Gln
   1               5                   10                  15
   Val
<210> 50
   <211> 17
   <212> PRT
   <213> Artificial Sequence
<220>
   <223> Description of Artificial Sequence: Synthetic peptide
<400> 50
   Gln Ile Glu Tyr Val Ala Lys Gln Ile Val Asp Tyr Ala Ile His Gln
   1               5                   10                  15
   Ala
<210> 51
   <211> 17
   <212> PRT
   <213> Artificial Sequence
<220>
   <223> Description of Artificial Sequence: Synthetic peptide
<400> 51
   Gln Ile Glu Tyr Lys Ala Lys Gln Ile Val Asp His Ala Ile His Gln
   1               5                   10                  15
   Ala
<210> 52
   <211> 17
   <212> PRT
   <213> Artificial Sequence
<220>
   <223> Description of Artificial Sequence: Synthetic peptide
<400> 52
   Gln Ile Glu Tyr His Ala Lys Gln Ile Val Asp His Ala Ile His Gln
   1 5 10 15
   Ala
<210> 53
   <211> 17
   <212> PRT
   <213> Artificial Sequence
<220>
   <223> Description of Artificial Sequence: Synthetic peptide
<400> 53
   Gln Ile Glu Tyr Val Ala Lys Gln Ile Val Asp His Ala Ile His Gln
   1 5 10 15
   Ala
<210> 54
   <211> 18
   <212> PRT
   <213> Artificial Sequence
<220>
   <223> Description of Artificial Sequence: Synthetic peptide
<400> 54
   Pro Leu Glu Tyr Gln Ala Gly Leu Leu Val Gln Asn Ala Ile Gln Gln
   1 5 10 15
   Ala Ile
<210> 55
   <211> 18
   <212> PRT
   <213> Artificial Sequence
<220>
   <223> Description of Artificial Sequence: Synthetic peptide
<400> 55
   Leu Leu Ile Glu Thr Ala Ser Ser Leu Val Lys Asn Ala Ile Gln Leu
   1               5                   10                  15
   Ser Ile
<210> 56
   <211> 18
   <212> PRT
   <213> Artificial Sequence
<220>
   <223> Description of Artificial Sequence: Synthetic peptide
<400> 56
   Leu Ile Glu Glu Ala Ala Ser Arg Ile Val Asp Ala Val Ile Glu Gln
   1               5                   10                  15
   Val Lys
<210> 57
   <211> 18
   <212> PRT
   <213> Artificial Sequence
<220>
   <223> Description of Artificial Sequence: Synthetic peptide
<400> 57
   Ala Leu Tyr Gln Phe Ala Asp Arg Phe Ser Glu Leu Val Ile Ser Glu
   1               5                   10                  15
   Ala Leu
<210> 58
   <211> 17
   <212> PRT
   <213> Artificial Sequence
<220>
   <223> Description of Artificial Sequence: Synthetic peptide
<400> 58
   Leu Glu Gln Val Ala Asn Gln Leu Ala Asp Gln Ile Ile Lys Glu Ala
   1               5                   10                  15
   Thr
<210> 59
   <211> 17
   <212> PRT
   <213> Artificial Sequence
<220>
   <223> Description of Artificial Sequence: Synthetic peptide
<400> 59
   Phe Glu Glu Leu Ala Trp Lys Ile Ala Lys Met Ile Trp Ser Asp Val
   1               5                   10                  15
   Phe
<210> 60
   <211> 18
   <212> PRT
   <213> Artificial Sequence
<220>
   <223> Description of Artificial Sequence: Synthetic peptide
<400> 60
   Glu Leu Val Arg Leu Ser Lys Arg Leu Val Glu Asn Ala Val Leu Lys
   1               5                   10                  15
   Ala Val
<210> 61
   <211> 18
   <212> PRT
   <213> Artificial Sequence
<220>
   <223> Description of Artificial Sequence: Synthetic peptide
<400> 61
   Thr Ala Glu Glu Val Ser Ala Arg Ile Val Gln Val Val Thr Ala Glu
   1               5                   10                  15
   Ala Val
<210> 62
   <211> 18
   <212> PRT
   <213> Artificial Sequence
<220>
   <223> Description of Artificial Sequence: Synthetic peptide
<400> 62
   Gln Ile Lys Gln Ala Ala Phe Gln Leu Ile Ser Gln Val Ile Leu Glu
   1 5 10 15
   Ala Thr
<210> 63
   <211> 16
   <212> PRT
   <213> Artificial Sequence
<220>
   <223> Description of Artificial Sequence: Synthetic peptide
<400> 63
   Leu Ala Trp Lys Ile Ala Lys Met Ile Val Ser Asp Val Met Gln Gln
   1 5 10 15
<210> 64
   <211> 24
   <212> PRT
   <213> Artificial Sequence
<220>
   <223> Description of Artificial Sequence: Synthetic peptide
<400> 64
   Asp Leu Ile Glu Glu Ala Ala Ser Arg Ile Val Asp Ala Val Ile Glu
   1 5 10 15
   Gln Val Lys Ala Ala Gly Ala Tyr
   20
<210> 65
   <211> 18
   <212> PRT
   <213> Artificial Sequence
<220>
   <223> Description of Artificial Sequence: Synthetic peptide
<400> 65
   Leu Glu Gln Tyr Ala Asn Gln Leu Ala Asp Gln Ile Ile Lys Glu Ala
   1 5 10 15
   Thr Glu
<210> 66
   <211> 20
   <212> PRT
   <213> Artificial Sequence
<220>
   <223> Description of Artificial Sequence: Synthetic peptide
<400> 66
   Phe Glu Glu Leu Ala Trp Lys Ile Ala Lys Met Ile Trp Ser Asp Val
   1               5                   10                  15
   Phe Gln Gln Cys
               20
<210> 67
   <211> 17
   <212> PRT
   <213> Artificial Sequence
<220>
   <223> Description of Artificial Sequence: Synthetic peptide
<400> 67
   Gln Ile Glu Tyr Leu Ala Lys Gln Ile Pro Asp Asn Ala Ile Gln Gln
   1               5                   10                  15
   Ala
<210> 68
   <211> 25
   <212> PRT
   <213> Artificial Sequence
<220>
   <223> Description of Artificial Sequence: Synthetic peptide
<400> 68
   Lys Gly Ala Asp Leu Ile Glu Glu Ala Ala Ser Arg Ile Val Asp Ala
   1               5                   10                  15
   Val Ile Glu Gln Val Lys Ala Ala Gly
               20                  25
<210> 69
   <211> 25
   <212> PRT
   <213> Artificial Sequence
<220>
   <223> Description of Artificial Sequence: Synthetic peptide
<400> 69
   Lys Gly Ala Asp Leu Ile Glu Glu Ala Ala Ser Arg Ile Pro Asp Ala
   1               5                   10                  15
   Pro Ile Glu Gln Val Lys Ala Ala Gly
               20                  25
<210> 70
   <211> 25
   <212> PRT
   <213> Artificial Sequence
<220>
   <223> Description of Artificial Sequence: Synthetic peptide
<400> 70
   Pro Glu Asp Ala Glu Leu Val Arg Leu Ser Lys Arg Leu Val Glu Asn
   1               5                   10                  15
   Ala Val Leu Lys Ala Val Gln Gln Tyr
               20                  25
<210> 71
   <211> 25
   <212> PRT
   <213> Artificial Sequence
<220>
   <223> Description of Artificial Sequence: Synthetic peptide
<400> 71
   Pro Glu Asp Ala Glu Leu Val Arg Thr Ser Lys Arg Leu Val Glu Asn
   1               5                   10                  15
   Ala Val Leu Lys Ala Val Gln Gln Tyr
               20                  25
<210> 72
   <211> 25
   <212> PRT
   <213> Artificial Sequence
<220>
   <223> Description of Artificial Sequence: Synthetic peptide
<400> 72
   Pro Glu Asp Ala Glu Leu Val Arg Leu Ser Lys Arg Asp Val Glu Asn
   1               5                   10                  15
   Ala Val Leu Lys Ala Val Gln Gln Tyr
               20                  25
<210> 73
   <211> 25
   <212> PRT
   <213> Artificial Sequence
<220>
   <223> Description of Artificial Sequence: Synthetic peptide
<400> 73
   Pro Glu Asp Ala Glu Leu Val Arg Leu Ser Lys Arg Leu Pro Glu Asn
   1               5                   10                  15
   Ala Val Leu Lys Ala Val Gln Gln Tyr
               20                  25
<210> 74
   <211> 25
   <212> PRT
   <213> Artificial Sequence
<220>
   <223> Description of Artificial Sequence: Synthetic peptide
<400> 74
   Pro Glu Asp Ala Glu Leu Val Arg Leu Ser Lys Arg Leu Pro Glu Asn
   1               5                   10                  15
   Ala Pro Leu Lys Ala Val Gln Gln Tyr
               20                  25
<210> 75
   <211> 25
   <212> PRT
   <213> Artificial Sequence
<220>
   <223> Description of Artificial Sequence: Synthetic peptide
<400> 75
   Pro Glu Asp Ala Glu Leu Val Arg Leu Ser Lys Arg Leu Val Glu Asn
   1               5                   10                  15
   Ala Val Glu Lys Ala Val Gln Gln Tyr
               20                  25
<210> 76
   <211> 25
   <212> PRT
   <213> Artificial Sequence
<220>
   <223> Description of Artificial Sequence: Synthetic peptide
<400> 76
   Glu Glu Gly Leu Asp Arg Asn Glu Glu Ile Lys Arg Ala Ala Phe Gln
   1               5                   10                  15
   Ile Ile Ser Gln Val Ile Ser Glu Ala
               20                  25
<210> 77
   <211> 25
   <212> PRT
   <213> Artificial Sequence
<220>
   <223> Description of Artificial Sequence: Synthetic peptide
<400> 77
   Leu Val Asp Asp Pro Leu Glu Tyr Gln Ala Gly Leu Leu Val Gln Asn
   1               5                   10                  15
   Ala Ile Gln Gln Ala Ile Ala Glu Gln
               20                  25
<210> 78
   <211> 25
   <212> PRT
   <213> Artificial Sequence
<220>
   <223> Description of Artificial Sequence: Synthetic peptide
<400> 78
   Gln Tyr Glu Thr Leu Leu Ile Glu Thr Ala Ser Ser Leu Val Lys Asn
   1               5                   10                  15
   Ala Ile Gln Leu Ser Ile Glu Gln Leu
               20                  25
<210> 79
   <211> 25
   <212> PRT
   <213> Artificial Sequence
<220>
   <223> Description of Artificial Sequence: Synthetic peptide
<400> 79
   Leu Glu Lys Gln Tyr Gln Glu Gln Leu Glu Glu Glu Val Ala Lys Val
   1               5                   10                  15
   Ile Val Ser Met Ser Ile Ala Phe Ala
               20                  25
<210> 80
   <211> 25
   <212> PRT
   <213> Artificial Sequence
<220>
   <223> Description of Artificial Sequence: Synthetic peptide
<400> 80
   Asn Thr Asp Glu Ala Gln Glu Glu Leu Ala Trp Lys Ile Ala Lys Met
   1               5                   10                  15
   Ile Val Ser Asp Ile Met Gln Gln Ala
               20                  25
<210> 81
   <211> 25
   <212> PRT
   <213> Artificial Sequence
<220>
   <223> Description of Artificial Sequence: Synthetic peptide
<400> 81
   Val Asn Leu Asp Lys Lys Ala Val Leu Ala Glu Lys Ile Val Ala Glu
   1               5                   10                  15
   Ala Ile Glu Lys Ala Glu Arg Glu Leu
               20                  25
<210> 82
   <211> 25
   <212> PRT
   <213> Artificial Sequence
<220>
   <223> Description of Artificial Sequence: Synthetic peptide
<400> 82
   Asn Gly Ile Leu Glu Leu Glu Thr Lys Ser Ser Lys Leu Val Gln Asn
   1               5                   10                  15
   Ile Ile Gln Thr Ala Val Asp Gln Phe
               20                  25
<210> 83
   <211> 25
   <212> PRT
   <213> Artificial Sequence
<220>
   <223> Description of Artificial Sequence: Synthetic peptide
<400> 83
   Thr Gln Asp Lys Asn Tyr Glu Asp Glu Leu Thr Gln Val Ala Leu Ala
   1               5                   10                  15
   Leu Val Glu Asp Val Ile Asn Tyr Ala
               20                  25
<210> 84
   <211> 25
   <212> PRT
   <213> Artificial Sequence
<220>
   <223> Description of Artificial Sequence: Synthetic peptide
<400> 84
   Glu Thr Ser Ala Lys Asp Asn Ile Asn Ile Glu Glu Ala Ala Arg Phe
   1               5                   10                  15
   Leu Val Glu Lys Ile Leu Val Asn His
               20                  25
<210> 85
   <211> 44
   <212> PRT
   <213> Artificial Sequence
<220>
   <223> Description of Artificial Sequence: Synthetic consensus polypeptide
<220>
   <221> MOD_RES
   <222> (1)..(1)
   <223> Ser or Thr
<220>
   <221> MOD_RES
   <222> (2)..(2)
   <223> His, Lys or Arg
<220>
   <221> MOD_RES
   <222> (4)..(4)
   <223> Gln or Asn
<220>
   <221> MOD_RES
   <222> (8)..(8)
   <223> Gly or Ala
<220>
   <221> MOD_RES
   <222> (10)..(10)
   <223> Thr or Ser
<220>
   <221> MOD_RES
   <222> (11)..(11)
   <223> Glu or Asp
<220>
   <221> MOD_RES
   <222> (14)..(14)
   <223> Gln or Asn
<220>
   <221> MOD_RES
   <222> (15)..(15)
   <223> Gly or Ala
<220>
   <221> MOD_RES
   <222> (17)..(17)
   <223> Thr or Ser
<220>
   <221> MOD_RES
   <222> (19)..(19)
   <223> Glu or Asp
<220>
   <221> MOD_RES
   <222> (22)..(22)
   <223> Arg or Lys
<220>
   <221> MOD_RES
   <222> (23)..(24)
   <223> Gln or Asn
<220>
   <221> MOD_RES
   <222> (27)..(27)
   <223> Asp or Glu
<220>
   <221> MOD_RES
   <222> (30)..(30)
   <223> Glu or Asp
<220>
   <221> MOD_RES
   <222> (32)..(32)
   <223> Ala, Leu, Ile or Val
<220>
   <221> MOD_RES
   <222> (34)..(34)
   <223> Glu or Asp
<220>
   <221> MOD_RES
   <222> (37)..(37)
   <223> Thr or Ser
<220>
   <221> MOD_RES
   <222> (38)..(38)
   <223> Arg or Lys
<220>
   <221> MOD_RES
   <222> (40)..(40)
   <223> Arg or Lys
<220>
   <221> MOD_RES
   <222> (41)..(41)
   <223> Glu or Asp
<220>
   <221> MOD_RES
   <222> (42)..(42)
   <223> Ala, Leu, Ile or Val
<220>
   <221> MOD_RES
   <222> (43)..(43)
   <223> Arg or Lys
<220>
   <221> MOD_RES
   <222> (44)..(44)
   <223> Ala, Leu, Ile or Val
<400> 85
   Xaa Xaa Ile Xaa Ile Pro Pro Xaa Leu Xaa Xaa Leu Leu Xaa Xaa Tyr
   1               5                   10                  15
   Xaa Val Xaa Val Leu Xaa Xaa Xaa Pro Pro Xaa Leu Val Xaa Phe Xaa
               20                  25                  30
   Val Xaa Tyr Phe Xaa Xaa Leu Xaa Xaa Xaa Xaa Xaa
           35                  40
<210> 86
   <211> 17
   <212> PRT
   <213> Artificial Sequence
<220>
   <223> Description of Artificial Sequence: Synthetic consensus peptide
<220>
   <221> MOD_RES
   <222> (1)..(1)
   <223> Gln or Asn
<220>
   <221> MOD_RES
   <222> (2)..(2)
   <223> Ile, Leu or Val
<220>
   <221> MOD_RES
   <222> (3)..(3)
   <223> Glu or Asp
<220>
   <221> MOD_RES
   <222> (4)..(4)
   <223> Tyr, Phe, Thr or Ser
<220>
   <221> MOD_RES
   <222> (5)..(5)
   <223> Leu, Ile or Val
<220>
   <221> MOD_RES
   <222> (7)..(7)
   <223> Lys or Arg
<220>
   <221> MOD_RES
   <222> (8)..(8)
   <223> Gln or Asn
<220>
   <221> MOD_RES
   <222> (11)..(11)
   <223> Asp or Glu
<220>
   <221> MOD_RES
   <222> (12)..(12)
   <223> Asn or Gln
<220>
   <221> MOD_RES
   <222> (15)..(16)
   <223> Gln or Asn
<220>
   <221> MOD_RES
   <222> (17)..(17)
   <223> Ala, Leu, Ile or Val
<400> 86
   Xaa Xaa Xaa Xaa Xaa Ala Xaa Xaa Ile Val Xaa Xaa Ala Ile Xaa Xaa
   1               5                   10                  15
   Xaa
<210> 87
   <211> 44
   <212> PRT
   <213> Artificial Sequence
<220>
   <223> Description of Artificial Sequence: Synthetic consensus polypeptide
<220>
   <221> MOD_RES
   <222> (1)..(1)
   <223> Ser or Thr
<220>
   <221> MOD_RES
   <222> (4)..(4)
   <223> Gln or Asn
<220>
   <221> MOD_RES
   <222> (10)..(10)
   <223> Thr or Ser
<220>
   <221> MOD_RES
   <222> (18)..(18)
   <223> Val, Ile, Leu or Ala
<220>
   <221> MOD_RES
   <222> (23)..(23)
   <223> Gln or Asn
<220>
   <221> MOD_RES
   <222> (33)..(33)
   <223> Val, Ile, Leu or Ala
<220>
   <221> MOD_RES
   <222> (34)..(34)
   <223> Glu or Asp
<220>
   <221> MOD_RES
   <222> (37)..(37)
   <223> Thr or Ser
<220>
   <221> MOD_RES
   <222> (38)..(38)
   <223> Arg or Lys
<220>
   <221> MOD_RES
   <222> (40)..(40)
   <223> Arg or Lys
<220>
   <221> MOD_RES
   <222> (42)..(42)
   <223> Ala, Leu, Ile or Val
<220>
   <221> MOD_RES
   <222> (44)..(44)
   <223> Ala, Leu, Ile or Val
<400> 87
   Xaa His Ile Xaa Ile Pro Pro Gly Leu Thr Glu Leu Leu Gln Gly Tyr
   1               5                   10                  15
   Thr Xaa Glu Val Leu Arg Xaa Gln Pro Pro Asp Leu Val Glu Phe Ala
               20                  25                  30
   Xaa Xaa Tyr Phe Xaa Xaa Leu Xaa Glu Xaa Arg Xaa
           35                  40
<210> 88
   <211> 4
   <212> PRT
   <213> Artificial Sequence
<220>
   <223> Description of Artificial Sequence: Synthetic peptide
<220>
   <223> N-term DOTA
<220>
   <221> MOD_RES
   <222> (1)..(1)
   <223> D-Tyr
<220>
   <221> MOD_RES
   <222> (2)..(2)
   <223> D-Lys(HSG)
<220>
   <221> MOD_RES
   <222> (3)..(3)
   <223> D-Glu
<220>
   <221> MOD_RES
   <222> (4)..(4)
   <223> D-Lys(HSG)
<220>
   <223> C-term CH2CH2-SH
<400> 88
   Tyr Lys Glu Lys
   1
<210> 89
   <211> 55
   <212> PRT
   <213> Artificial Sequence
<220>
   <223> Description of Artificial Sequence: Synthetic polypeptide
<400> 89
   Gly Ser Gly Gly Gly Gly Ser Gly Gly Gly Gly Ser His Ile Gln Ile
   1               5                   10                  15
   Pro Pro Gly Leu Thr Glu Leu Leu Gln Gly Tyr Thr Val Glu Val Leu
               20                  25                  30
   Arg Gln Gln Pro Pro Asp Leu Val Glu Phe Ala Val Glu Tyr Phe Thr
           35                  40                  45
   Arg Leu Arg Glu Ala Arg Ala
       50                  55
<210> 90
   <211> 29
   <212> PRT
   <213> Artificial Sequence
<220>
   <223> Description of Artificial Sequence: Synthetic peptide
<400> 90
   Gly Ser Gly Gly Gly Gly Ser Gly Gly Gly Gly Ser Gln Ile Glu Tyr
   1               5                   10                  15
   Leu Ala Lys Gln Ile Val Asp Asn Ala Ile Gln Gln Ala
               20                  25
<210> 91
   <211> 9
   <212> PRT
   <213> Artificial Sequence
<220>
   <223> Description of Artificial Sequence: Synthetic peptide
<400> 91
   Gly Ser Gly Gly Gly Gly Ser Gly Gly
   1 5
<210> 92
   <211> 4
   <212> PRT
   <213> Artificial Sequence
<220>
   <223> Description of Artificial Sequence: Synthetic peptide
<220>
   <223> N-term NOTA-ITC benzyl
<220>
   <221> MOD_RES
   <222> (1)..(1)
   <223> D-Ala
<220>
   <221> MOD_RES
   <222> (2)..(2)
   <223> D-Lys(HSG)
<220>
   <221> MOD_RES
   <222> (3)..(3)
   <223> D-Tyr
<220>
   <221> MOD_RES
   <222> (4)..(4)
   <223> D-Lys(HSG)
<220>
   <223> C-term NH2
<400> 92
   Ala Lys Tyr Lys
   1
<210> 93
   <211> 4
   <212> PRT
   <213> Artificial Sequence
<220>
   <223> Description of Artificial Sequence: Synthetic peptide
<220>
   <221> MOD_RES
   <222> (1)..(1)
   <223> D-Ala
<220>
   <221> MOD_RES
   <222> (2)..(2)
   <223> D-Lys(HSG)
<220>
   <221> MOD_RES
   <222> (3)..(3)
   <223> D-Tyr
<220>
   <221> MOD_RES
   <222> (4)..(4)
   <223> D-Lys(HSG)
<220>
   <223> C-term NH2
<400> 93
   Ala Lys Tyr Lys
   1
<210> 94
   <211> 4
   <212> PRT
   <213> Artificial Sequence
<220>
   <223> Description of Artificial Sequence: Synthetic peptide
<220>
   <223> N-term NODA-Ga
<220>
   <221> MOD_RES
   <222> (1)..(1)
   <223> D-Ala
<220>
   <221> MOD_RES
   <222> (2)..(2)
   <223> D-Lys(HSG)
<220>
   <221> MOD_RES
   <222> (3)..(3)
   <223> D-Tyr
<220>
   <221> MOD_RES
   <222> (4)..(4)
   <223> D-Lys(HSG)
<220>
   <223> C-term NH2
<400> 94
   Ala Lys Tyr Lys
   1
<210> 95
   <211> 4
   <212> PRT
   <213> Artificial Sequence
<220>
   <223> Description of Artificial Sequence: Synthetic peptide
<220>
   <223> N-term NOTA
<220>
   <221> MOD_RES
   <222> (1)..(1)
   <223> D-Ala
<220>
   <221> MOD_RES
   <222> (2)..(2)
   <223> D-Lys(HSG)
<220>
   <221> MOD_RES
   <222> (3)..(3)
   <223> D-Tyr
<220>
   <221> MOD_RES
   <222> (4)..(4)
   <223> D-Lys(HSG)
<220>
   <223> C-term NH2
<400> 95
   Ala Lys Tyr Lys
   1
<210> 96
   <211> 4
   <212> PRT
   <213> Artificial Sequence
<220>
   <223> Description of Artificial Sequence: Synthetic peptide
<220>
   <223> N-term NOTA
<220>
   <221> MOD_RES
   <222> (1)..(1)
   <223> D-Asp
<220>
   <221> MOD_RES
   <222> (2)..(2)
   <223> D-Lys(HSG)
<220>
   <221> MOD_RES
   <222> (3)..(3)
   <223> D-Tyr
<220>
   <221> MOD_RES
   <222> (4)..(4)
   <223> D-Lys(HSG)
<220>
   <223> C-term NH2
<400> 96
   Asp Lys Tyr Lys
   1
<210> 97
   <211> 3
   <212> PRT
   <213> Artificial Sequence
<220>
   <223> Description of Artificial Sequence: Synthetic peptide
<220>
   <223> N-term C-NETA-succinyl
<220>
   <221> MOD_RES
   <222> (1)..(1)
   <223> D-Lys(HSG)
<220>
   <221> MOD_RES
   <222> (2)..(2)
   <223> D-Tyr
<220>
   <221> MOD_RES
   <222> (3)..(3)
   <223> D-Lys(HSG)
<220>
   <223> C-term NH2
<400> 97
   Lys Tyr Lys
   1
<210> 98
   <211> 10
   <212> PRT
   <213> Artificial Sequence
<220>
   <223> Description of Artificial Sequence: Synthetic peptide
<220>
   <223> N-term NOTA-NH-(CH2)7CO
<220>
   <223> C-term NH2
<400> 98
   Gln Trp Val Trp Ala Val Gly His Leu Met
   1 5 10
<210> 99
   <211> 3
   <212> PRT
   <213> Artificial Sequence
<220>
   <223> Description of Artificial Sequence: Synthetic peptide
<220>
   <223> N-term L-NETA-succinyl
<220>
   <221> MOD_RES
   <222> (1)..(1)
   <223> D-Lys(HSG)
<220>
   <221> MOD_RES
   <222> (2)..(2)
   <223> D-Tyr
<220>
   <221> MOD_RES
   <222> (3)..(3)
   <223> D-Lys(HSG)
<220>
   <223> C-term NH2
<400> 99
   Lys Tyr Lys
   1
<210> 100
   <211> 3
   <212> PRT
   <213> Artificial Sequence
<220>
   <223> Description of Artificial Sequence: Synthetic peptide
<220>
   <223> N-term NODA-MPAA
<220>
   <221> MOD_RES
   <222> (1)..(1)
   <223> D-Lys(HSG)
<220>
   <221> MOD_RES
   <222> (2)..(2)
   <223> D-Tyr
<220>
   <221> MOD_RES
   <222> (3)..(3)
   <223> D-Lys(HSG)
<220>
   <223> C-term NH2
<400> 100
   Lys Tyr Lys
   1

## Description

### Field

The present invention is realted to a DNL complex comprising (i) an anti-hapten antibody or antigen-binding fragment thereof conjugated to an AD moiety from an A-kinase anchor protein (AKAP); and (ii) a DDD moiety from human protein kinase A RIα, RIβ, RIIα or RIIβ, conjugated to an antibody or antigen-binding fragment thereof that binds to a target antigen associated with a disease or condition, wherein two copies of the DDD moiety form a dimer that binds to the AD moiety to form the DNL complex, for use in a method of diagnosising the disease or condition; and a kit for use in a method of diagnosis of a disease or condition, wherein the kit comprises (a) a DNL complex comprising (i) an anti-hapten antibody or antigen-binding fragment thereof conjugated to an AD moiety from an A-kinase anchor protein (AKAP); and (ii) a DDD moiety from human protein kinase A RIα, RIβ, RIIα or RIIβ, conjugated to an antibody or antigen-binding fragment thereof that binds to the target antigen associated with the disease or condition, wherein two copies of the DDD moiety form a dimer that binds to the AD moiety to form the DNL complex; and (b) a targetable construct comprising (iii) at least one hapten; and (iv) a fluorescent probe, wherein the hapten binds to the DNL complex.

### Background

Fluorescent imaging is an important modality for detection and/or diagnosis of disease states. It is of particular use for intraoperative, intraperitoneal, laparoscopic, endoscopic or intravascular detection of diseased tissues, either before, during or after surgical procedures (see, e.g., U.S. Patent Nos. 6,096,289; 6,387,350; 7,201,890). Surgical resection remains a primary curative approach in the management of cancer and other diseases (*Id*.). Intraoperative detection of minimal residual diseased tissues during cytoreductive surgery is an important prognostic factor for post-operative survival, facilitating complete cytoreduction of the diseased tissues (see, e.g., Prasad et al., 2005, J Gastrointest Surg 9:1138-47). Fluorescent imaging is well-suited for such intraoperative or diagnostic procedures, showing high sensitivity and specificity with minimal toxicity.

Previous methods of fluorescent imaging using certain dyes that are accreted by lesions, such as tumors, which are in turn activated by a specific frequency of light, are disclosed in Dougherty et al., Cancer Res. 38:2628, 1978; Dougherty, T. J., Photochem. Photobiol. 45:879, 1987; Jori and Perria, eds., Photodynamic Therapy of Tumors and Other Diseases; Padua: Libreria Progetto, 1985; Profio, Proc. Soc. Photoopt. Instr. Eng. 907:150, 1988; Doiron and Gomer, eds., Porphyrin Localization and Treatment of Tumors; New York: Alan Liss, 1984; Hayata and Dougherty, Lasers and Hematoporphyrin Derivative in Cancer; Tokyo: Igaku-Shoin, 1984; and van den Bergh, Chem. Britain 22:430, 1986. These dyes are injected, for example, systemically, and laser-induced fluorescence can be used by endoscopes to detect sites of cancer which have accreted the light-activated dye. For example, this has been applied to fluorescence bronchoscopic disclosure of early lung tumors (Doiron et al., Chest 76:32, 1979). However, non-specific background or high accretion in non-targeted tissues may complicate detection, diagnosis and imaging in the absence of targeting molecules.

Fluorescent imaging, detection and/or diagnosis may be performed using fluorescently labeled targeting molecules, such as antibodies, antigen-binding antibody fragments, receptor ligands or other tissue-specific or tissue-selective molecules. Fluorescent labeling may occur by direct conjugation of a fluorescent probe to a targeting molecule. Alternatively, a bispecific or multispecific antibody may be indirectly labeled by a pretargeting technique. Pretargeting is a multistep process originally developed to address the slow blood clearance of directly targeting antibodies, which contributes to undesirable toxicity to normal tissues such as bone marrow. With pretargeting, a radionuclide, fluorescent dye or other diagnostic or therapeutic agent is attached to a small delivery molecule (targetable construct) that is cleared within minutes from the blood. A pretargeting bispecific or multispecific antibody, which has binding sites for the targetable construct as well as a target antigen, is administered first, free antibody is allowed to clear from circulation and then the targetable construct is administered. Pretargeting methods are disclosed, for example, in Goodwin et al., U.S. Pat. No. 4,863,713; Goodwin et al., J. Nucl. Med. 29:226, 1988; Hnatowich et al., J. Nucl. Med. 28:1294, 1987; Oehr et al., J. Nucl. Med. 29:728,1988; Klibanov et al., J. Nucl. Med. 29:1951, 1988; Sinitsyn et al., J. Nucl. Med. 30:66,1989; Kalofonos et al., J. Nucl. Med. 31:1791, 1990; Schechter et al., Int. J. Cancer 48:167,1991; Paganelli et al., Cancer Res. 51:5960,1991; Paganelli et al., Nucl. Med. Commun. 12:211, 1991; U.S. Pat. No. 5,256,395; Stickney et al., Cancer Res. 51:6650, 1991; Yuan et al., Cancer Res. 51:3119, 1991; U.S. Pat. Nos. 6,077,499; 7,011,812; 7,300,644; 7,074,405; 6,962.702; 7,387,772; 7,052,872; 7,138,103; 6,090,381; 6,472,511; 6,962,702; and 6,962,702.

Once the dye-labeled molecule has been delivered to the target tissue, its distribution may be imaged by fluorescent detection. For example, fluorescent imaging may be utilized during intraoperative, intravascular or endoscopic procedures, as described in U.S. Patent Nos. 4,932,412; 6,096,289; 6,387,350; 7,201,890. Such imaging methods may be of use, for example, to image the distribution of tumor tissue to facilitate its removal. Fluorescent imaging may also be of use for diagnostic purposes, for example to distinguish between malignant, benign and hyperplastic tissues.

US 2007/0086942 A1 is related to methods and compositions for making and using bioactive assemblies of defined compositions which may have multiple functionalities and/or binding specificities.

US 2011/0158905 A1 is related to compositions and methods of use of dock-and-lock (DNL) complexes, comprising at least two copies of a dimerization and docking domain (DDD) moiety and at least one copy of an anchoring domain (AD) moiety.

US 2011/0165072 A1 is related to compositions and methods of use of anti-pancreatic cancer antibodies or fragments thereof.

While fluorescent imaging is a promising technique for detection and/or diagnosis of diseased tissues, a need exists for improved methods and compositions for fluorescent labeling, targeted delivery and detection of peptides and other molecules of use in fluorescent imaging, detection and/or diagnosis.

### Summary

The problem underlying the present invention is solved by the subject matter of the attached independent claims. Preferred embodiments may be taken from the attached dependent claims.

More specifically, the problem underlying the present invention is solved in a first aspect by a DNL complex comprising (i) an anti-hapten antibody or antigen-binding fragment thereof conjugated to an AD moiety from an A-kinase anchor protein (AKAP); and (ii) a DDD moiety from human protein kinase A RIα, RIβ, RIIα or RIIβ, conjugated to an antibody or antigen-binding fragment thereof that binds to a target antigen associated with a disease or condition, wherein two copies of the DDD moiety form a dimer that binds to the AD moiety to form the DNL complex, for use in a method for diagnosising the disease or condition comprising detecting the target antigen associated with the disease or condition, wherein the method comprises
a) exposing the target antigen to the DNL complex;
b) allowing the DNL complex to bind to the antigen;
c) adding a targetable construct comprising (iii) at least one hapten; and (iv) a fluorescent probe, wherein the hapten binds to the DNL complex; and
d) detecting the fluorescently labeled target antigen by an intraoperative, intraperitoneal, laparoscopic, endoscopic or intravascular procedure.

In an embodiment of the first aspect, the targetable construct is selected from the group consisting of IMP-448, IMP-449, IMP-460, IMP-461, IMP-462, IMP-467, IMP-468, IMP-470, IMP-485 and IMP-499.

In an embodiment of the first aspect, the hapten is HSG or In-DTPA.

In an embodiment of the first aspect, the antigen is a tumor-associated antigen, an autoimmune disease-associated antigen or an antigen produced or displayed by a pathogenic organism.

In an embodiment of the first aspect, the tumor-associated antigen is selected from the group consisting of carbonic anhydrase IX, alpha-fetoprotein, α-actinin-4, A3, antigen specific for A33 antibody, ART-4, B7, Ba 733, BAGE, BrE3-antigen, CA125, CAMEL, CAP-1, CASP-8/m, , CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CXCR4, CXCR7, CXCL12, HIF-la, colon-specific antigen-p (CSAp), CEA (CEACAM5), CEACAM6, c-met, DAM, EGFR, EGFRvIII, EGP-1, EGP-2, ELF2-M, Ep-CAM, Flt-1, Flt-3, folate receptor, G250 antigen, GAGE, gp100, GROB, HLA-DR, HM1.24, human chorionic gonadotropin (HCG) and its subunits, HER2/neu, HMGB-1, hypoxia inducible factor (HIF-1), HSP70-2M, HST-2, Ia, IGF-1R, IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5, MUC13, MUC16, MUM-1/2, MUM-3, NCA66, NCA95, NCA90, pancreatic cancer mucin, placental growth factor, p53, PLAGL2, prostatic acid phosphatase, PSA, PRAME, PSMA, P1GF, ILGF, ILGF-1R, IL-6, IL-25, RS5, RANTES, T101, SAGE, S100, survivin, survivin-2B, TAC, TAG-72, tenascin, TRAIL receptors, TNF-a, Tn antigen, Thomson-Friedenreich antigens, tumor necrosis antigens, TROP-2, VEGFR, ED-B fibronectin, WT-1, 17-1A-antigen, complement factors C3, C3a, C3b, C5a, C5, an angiogenesis marker, bcl-2, bcl-6, Kras, cMET, an oncogene marker and an oncogene product.

In an embodiment of the first aspect, the antibody that binds to a target antigen is selected from the group consisting of hPAM4 (anti-mucin), hA20 (anti-CD20), hA19 (anti-CD19), hIMMU31 (anti-AFP), hLL1 (anti-CD74), hLL2 (anti-CD22), hMu-9 (anti-CSAp), hL243 (anti-HLA-DR), hMN-14 (anti-CEACAM5), hMN-15 (anti-CEACAM6), hRS7 (anti-EGP-1), hMN-3 (anti-CEACAM6), Ab124 (anti-CXCR4), Ab125 (anti-CXCR4), abciximab (anti-glycoprotein IIb/IIIa), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), trastuzumab (anti-ErbB2), abagovomab (anti-CA-125), adecatumumab (anti-EpCAM), atlizumab (anti-IL-6R), benralizumab (anti-CD 125), CC49 (anti-TAG-72), AB-PG1-XG1-026 (anti-PSMA), D2/B (anti-PSMA), tocilizumab (anti-IL-6R), basiliximab (anti-CD25), daclizumab (anti-CD25), efalizumab (anti-CD 11a), GA101 (anti-CD20), muromonab-CD3 (anti-CD3 receptor), natalizumab (anti-a4 integrin), omalizumab (anti-IgE), CDP571 (anti-TNF-a), infliximab (anti-TNF-a), certolizumab (anti-TNF-a), adalimumab (anti-TNF-α), belimumab (anti-B-cell activating factor), Alz 50 (anti-tau protein), gantenerumab (anti-amyloid protein), solanezumab (anti-amyloid protein), P4/D10 (anti-gp120), CR6261 (anti-influenza), exbivirumab (anti-hepatitis B), felvizumab (anti-respiratory syncytial virus), foravirumab (anti-rabies virus), motavizumab (anti-respiratory syncytial virus), palivizumab (anti-respiratory syncytial virus), panobacumab (anti-Pseudomonas), rafivirumab (anti-rabies virus), regavirumab (anti-cytomegalovirus), sevirumab (anti-cytomegalovirus), tivirumab (anti-hepatitis B), and urtoxazumab (anti-E. coli).

In an embodiment of the first aspect, the fluorescent probe is selected from the group consisting of Alexa 350, Alexa 430, AMCA, aminoacridine, BODIPY 630/650, BODIPY 650/665, BODIPY-FL, BODIPY-R6G, BODIPY-TMR, BODIPY-TRX, 5-carboxy-4',5'-dichloro-2',7'-dimethoxy fluorescein, 5-carboxy-2',4',5',7'-tetrachlorofluorescein, 5-carboxyfluorescein, 5-carboxyrhodamine, 6-carboxyrhodamine, 6-carboxytetramethyl amino, Cascade Blue, Cy2, Cy3, Cy5,6-FAM, dansyl chloride, fluorescein, HEX, 6-JOE, NBD (7-nitrobenz-2-oxa-1,3-diazole), Oregon Green 488, Oregon Green 500, Oregon Green 514, Pacific Blue, phthalic acid, terephthalic acid, isophthalic acid, cresyl fast violet, cresyl blue violet, brilliant cresyl blue, para-aminobenzoic acid, erythrosine, phthalocyanines, azomethines, cyanines, xanthines, succinylfluoresceins, rare earth metal cryptates, europium trisbipyridine diamine, a europium cryptate or chelate, diamine, dicyanins, La Jolla blue dye, allopycocyanin, allococyanin B, phycocyanin C, phycocyanin R, thiamine, phycoerythrocyanin, phycoerythrin R, REG, Rhodamine Green, rhodamine isothiocyanate, Rhodamine Red, ROX, TAMRA, TET, TRIT (tetramethyl rhodamine isothiol), Tetramethylrhodamine, and Texas Red.

In an embodiment of the first aspect, the disease is selected from the group consisting of non-Hodgkin's lymphoma, acute lymphoid leukemia, chronic lymphoid leukemia, Burkitt lymphoma, Hodgkin's lymphoma, hairy cell leukemia, acute myeloid leukemia, chronic myeloid leukemia, T-cell lymphoma, T-cell leukemia, multiple myeloma, glioma, Waldenstrom's macroglobulinemia, carcinoma, melanoma, sarcoma, glioma and skin cancer, wherein preferably the carcinoma is a carcinoma of the oral cavity, gastrointestinal tract, pulmonary tract, lung, breast, ovary, prostate, uterus, endometrium, cervix, urinary bladder, pancreas, bone, brain, connective tissue, liver, gall bladder, kidney, skin, central nervous system or testes.

In an embodiment of the first aspect, the disease is selected from the group consisting of acute immune thrombocytopenia, chronic immune thrombocytopenia, dermatomyositis, Sydenham's chorea, myasthenia gravis, systemic lupus erythematosus, lupus nephritis, rheumatic fever, polyglandular syndromes, bullous pemphigoid, pemphigus vulgaris, Type 1 diabetes, Type 2 diabetes, Henoch-Schonlein purpura, post-streptococcal nephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjögren's syndrome, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis, fibrosing alveolitis, organ transplant rejection and graft-versus-host disease.

In an embodiment of the first aspect, the anti-hapten antibody and the anti-target antigen antibody are chimeric, humanized or human antibodies.

In an embodiment of the first aspect, the anti-hapten antibody fragment and the anti-target antigen antibody fragment are selected from the group consisting of F(ab')₂, Fab, scFv or Fv antibody fragments.

More specifically, the problem underlying the present invention is solved in a second aspect by a kit for use in a method of diagnosis of a disease or condition comprising detecting a fluorescently labeled target antigen associated with the disease or condition by an intraoperative, intraperitoneal, laparoscopic, endoscopic or intravascular procedure, wherein the kit comprises:
a) a DNL complex comprising (i) an anti-hapten antibody or antigen-binding fragment thereof conjugated to an AD moiety from an A-kinase anchor protein (AKAP); and (ii) a DDD moiety from human protein kinase A RIα, RIβ, RIIα or RIIβ, conjugated to an antibody or antigen-binding fragment thereof that binds to the target antigen associated with the disease or condition, wherein two copies of the DDD moiety form a dimer that binds to the AD moiety to form the DNL complex; and
b) a targetable construct comprising (iii) at least one hapten; and (iv) a fluorescent probe, wherein the hapten binds to the DNL complex.

In an embodioment of the second aspect, the targetable construct is selected from the group consisting of IMP-448, IMP-449, IMP-460, IMP-461, IMP-462, IMP-467, IMP-468, IMP-470, IMP-485 and IMP-499. Methods for pretargeting using bispecific or multispecific antibodies are well known in the art (see, e.g., U.S. Patent No. 6,962,702.)

Exemplary targetable construct peptides described in the Examples below, of use for pretargeting delivery of fluorescent probes or other agents, include but are not limited to IMP-448, IMP-449, IMP-460, IMP-461, IMP-462, IMP-467, IMP-468, IMP-470, IMP-485 and IMP-499. However, the person of ordinary skill in the art will realize that other targetable constructs are known in the art may also be utilized (see, e.g., U.S. Patent Nos. 5,965,131; 6,458,933; 6,962,702; 7,074,405; 7,172,751; 7,230,084; 7,429,381; 7,521,416; 7,776,311; 7,833,528; 7,892,547; 7,914,787 and 7,951,921).

In exemplary embodiments discussed in the Examples below, the peptides may contain an HSG (histamine-succinyl-glycine) hapten and may be used with an HSG-binding 679 antibody. However, other haptens and anti-hapten antibodies are known in the art and may be utilized in the claimed methods and compositions. Exemplary haptens and anti-hapten antibodies include, but are not limited to HSG and the 679 antibody (e.g., U.S. Patent Nos. 7,429,381; 7,563,439; 7,666,415) and In-DTPA and the 734 antibody (e.g., U.S. Patent Nos. 7,534,431; 7,892,547).

The type of diseases or conditions that may be imaged, detected and/or diagnosed is limited only by the availability of a suitable molecule for targeting a cell or tissue associated with the disease or condition. Exemplary antigens associated with a disease or condition may include a tumor-associated antigen, an autoimmune disease-associated antigen or an antigen produced or displayed by a pathogenic organism, such as a virus, bacterium, fungus or other microorganism. Antibodies of use may bind to any disease-associated antigen known in the art. Where the disease state is cancer, for example, many antigens expressed by or otherwise associated with tumor cells are known in the art, including but not limited to, carbonic anhydrase IX, alpha-fetoprotein, α-actinin-4, A3, antigen specific for A33 antibody, ART-4, B7, Ba 733, BAGE, BrE3-antigen, CA125, CAMEL, CAP-1, CASP-8/m, , CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CXCR4, CXCR7, CXCL12, HIF-1α, colon-specific antigen-p (CSAp), CEA (CEACAM5), CEACAM6, c-met, DAM, EGFR, EGFRvIII, EGP-1, EGP-2, ELF2-M, Ep-CAM, Flt-1, Flt-3, folate receptor, G250 antigen, GAGE, gp100, GROB, HLA-DR, HM1.24, human chorionic gonadotropin (HCG) and its subunits, HER2/neu, HMGB-1, hypoxia inducible factor (HIF-1). HSP70-2M, HST-2, Ia, IGF-1R, IFN-γ, IFN-α. IFN-β, IL-2, IL-4R, IL-6R, IL-13R, IL-15R, IL-17R, IL-18R, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5. MUC13, MUC16, MUM-1/2, MUM-3, NCA66, NCA95, NCA90, pancreatic cancer mucin, placental growth factor, p53, PLAGL2, prostatic acid phosphatase, PSA, PRAME, PSMA, P1GF, ILGF, ILGF-1R, IL-6, IL-25, RS5, RANTES, T101, SAGE, S100, survivin, survivin-2B, TAC, TAG-72, tenascin, TRAIL receptors, TNF-a, Tn antigen, Thomson-Friedenreich antigens, tumor necrosis antigens, TROP-2, VEGFR, ED-B fibronectin, WT-1, 17-1A-antigen, complement factors C3, C3a, C3b, C5a, C5, an angiogenesis marker, bcl-2, bcl-6, Kras, cMET, an oncogene marker and an oncogene product (see, e.g., Sensi et al., Clin Cancer Res 2006, 12:5023-32; Parmiani et al., J Immunol 2007, 178:1975-79; Novellino et al. Cancer Immunol Immunother 2005, 54:187-207).

Exemplary antibodies that may be utilized include, but are not limited to, hRl (anti-IGF-1R, U.S. Patent Application Serial No. 12/722,645, filed 3/12/10), hPAM4 (anti-mucin, U.S. Patent No. 7,282,567), hA20 (anti-CD20, U.S. Patent No. 7,251,164), hA19 (anti-CD19, U.S. Patent No. 7,109,304), hIMMU31 (anti-AFP, U.S. Patent No. 7,300,655), hLL1 (anti-CD74, U.S. Patent No. 7,312,318), hLL2 (anti-CD22, U.S. Patent No. 7,074,403), hMu-9 (anti-CSAp, U.S. Patent No. 7,387,773), hL243 (anti-HLA-DR, U.S. Patent No. 7,612,180), hMN-14 (anti-CEACAM5, U.S. Patent No. 6,676,924), hMN-15 (anti-CEACAM6, U.S. Patent No. 7,541,440), hRS7 (anti-EGP-1, U.S. Patent No. 7,238,785), hMN-3 (anti-CEACAM6, U.S. Patent No. 7,541,440), Ab124 and Ab125 (anti-CXCR4, U.S. Patent No. 7,138,496).

Alternative antibodies of use include, but are not limited to, abciximab (anti-glycoprotein IIb/IIIa), alemtuzumab (and-CD52), bevacizumab (anti-VEGF), cetuximab (anti-EGFR), gemtuzumab (anti-CD33), ibritumomab tiuxetan (anti-CD20), panitumumab (anti-EGFR), rituximab (anti-CD20), tositumomab (anti-CD20), trastuzumab (anti-ErbB2), abagovomab (anti-CA-125), adecatumumab (anti-EpCAM), atlizumab (anti-IL-6 receptor), benralizumab (anti-CD125), CC49 (anti-TAG-72), AB-PG1-XG1-026 (anti-PSMA, U.S. Patent Application 11/983,372, deposited as ATCC PTA-4405 and PTA-4406), D2/B (anti-PSMA, WO 2009/130575), tocilizumab (anti-IL-6 receptor), basiliximab (anti-CD25), daclizumab (anti-CD25), efalizumab (anti-CD11a), GA101 (anti-CD20; Glycart Roche), muromonab-CD3 (anti-CD3 receptor), natalizumab (anti-α4 integrin), omalizumab (anti-IgE); anti-TNF-α antibodies such as CDP571 (Ofei et al., 2011, Diabetes 45:881-85), MTNFAI, M2TNFAI, M3TNFAI, M3TNFABI, M302B, M303 (Thermo Scientific, Rockford, IL), infliximab (Centocor, Malvern, PA), certolizumab pegol (UCB, Brussels, Belgium), anti-CD40L (UCB, Brussels, Belgium), adalimumab (Abbott, Abbott Park, IL), Benlysta (Human Genome Sciences); antibodies for Alzheimer's disease such as Alz 50 (Ksiezak-Reding et al., 1987, J Biol Chem 263:7943-47), gantenerumab, solanezumab and infliximab; anti-fibrin antibodies like 59D8, T2G1s, MH1; and-HIV antibodies such as P4/D10 (U.S. Patent Application Serial No. 11/745,692), Ab 75, Ab 76, Ab 77 (Paulik et al., 1999, Biochem Pharmacol 58:1781-90); and antibodies against pathogens such as CR6261 (anti-influenza), exbivirumab (anti-hepatitis B), felvizumab (anti-respiratory syncytial virus), foravirumab (anti-rabies virus), motavizumab (anti-respiratory syncytial virus), palivizumab (anti-respiratory syncytial virus), panobacumab (anti-Pseudomonas), rafivirumab (anti-rabies virus), regavirumab (and-cytomegalovirus), sevirumab (anti-cytomegalovirus), tivirumab (anti-hepatitis B), and urtoxazumab (anti-E. coli).

An antibody or antigen-binding fragment of use may be murine, chimeric, humanized or human. The use of chimeric antibodies is preferred to the parent murine antibodies for *in vivo* use because they possess human antibody constant region sequences and therefore do not elicit as strong a human anti-mouse antibody (HAMA) response as murine antibodies. The use of humanized antibodies is even more preferred, in order to further reduce the possibility of inducing a HAMA reaction. Techniques for humanization of murine antibodies by replacing murine framework and constant region sequences with corresponding human antibody framework and constant region sequences are well known in the art and have been applied to numerous murine anti-cancer antibodies. Antibody humanization may also involve the substitution of one or more human framework amino acid residues with the corresponding residues from the parent murine framework region sequences. As discussed below, techniques for production of human antibodies are also well known in the art.

The targeting molecule may comprise an antibody fragment, such as F(ab')₂, Fab, scFv, Fv, or a fusion protein utilizing part or all of the light and heavy chains of the F(ab')₂, Fab, scFv. The antibody may be multivalent, or multivalent and multispecific. The antibody may include human constant regions of IgG1, IgG2a, IgG3, or IgG4.

Although according to the invention as defined in the claims, discussed in the Examples below, antibodies or antibody fragments are utilized to target the fluorescent probe to a diseased-associated antigen, cell or tissue, the skilled artisan will realize that virtually any targeting molecule can be attached to a fluorescent probe for imaging purposes, so long as it contains derivatizable groups that may be modified without affecting the ligand-receptor binding interaction between the targeting molecule and the cellular or tissue target receptor. Many types of targeting molecules, such as oligonucleotides, hormones, growth factors, cytokines, chemokines, angiogenic factors, anti-angiogenic factors, immunomodulators, proteins, nucleic acids, antibodies, antibody fragments, drugs, interleukins, interferons, oligosaccharides, polysaccharides, lipids and others may be fluorescently-labeled and utilized for imaging purposes. For example, molecules which bind directly to receptors, such as somatostatin, octreotide, bombesin, folate or a folate analog, an RGD peptide or other known receptor ligands may be labeled and used for imaging. Receptor targeting agents may include, for example, TA138, a non-peptide antagonist for the integrin αᵥβ₃ receptor (Liu et al., 2003, Bioconj. Chem. 14:1052-56).

In certain embodiments, the fluorescent probe is a DYLIGHT® dye (Thermo Fisher Scientific, Rockford, IL). The DYLIGHT® dye series are highly polar (hydrophilic), compatible with aqueous buffers, photostable and exhibit high fluorescence intensity. They remain highly fluorescent over a wide pH range and are preferred for various applications. However, the skilled artisan will realize that a variety of fluorescent dyes are known and/or are commercially available and may be utilized. Other fluorescent agents include, but are not limited to, dansyl chloride, rhodamine isothiocyanate, Alexa 350, Alexa 430, AMCA, aminoacridine, BODIPY 630/650, BODIPY 650/665, BODIPY-FL, BODIPY-R6G, BODIPY-TMR, BODIPY-TRX, 5-carboxy-4',5'-dichloro-2',7'-dimethoxy fluorescein, 5-carboxy-2',4',5',7'-tetrachlorofluorescein, 5-carboxyfluorescein, 5-carboxyrhodamine, 6-carboxyrhodamine, 6-carboxytetramethyl amino, Cascade Blue, Cy2, Cy3, Cy5,6-FAM, dansyl chloride, fluorescein, HEX, 6-JOE, NBD (7-nitrobenz-2-oxa-1,3-diazole), Oregon Green 488, Oregon Green 500, Oregon Green 514, Pacific Blue, phthalic acid, terephthalic acid, isophthalic acid, cresyl fast violet, cresyl blue violet, brilliant cresyl blue, para-aminobenzoic acid, erythrosine, phthalocyanines, azomethines, cyanines, xanthines, succinylfluoresceins, rare earth metal cryptates, europium trisbipyridine diamine, a europium cryptate or chelate, diamine, dicyanins, La Jolla blue dye, allopycocyanin, allococyanin B, phycocyanin C, phycocyanin R, thiamine, phycoerythrocyanin, phycoerythrin R, REG, Rhodamine Green, rhodamine isothiocyanate, Rhodamine Red, ROX, TAMRA, TET, TRIT (tetramethyl rhodamine isothiol), Tetramethylrhodamine, and Texas Red. (See, e.g., U.S. Pat. Nos. 5,800,992; 6,319,668.) These and other luminescent labels may be obtained from commercial sources such as Molecular Probes (Eugene, Oreg.), and EMD Biosciences (San Diego, Calif.).

The diseases or conditions that may be imaged, detected and/or diagnosed include, but are not limited to, non-Hodgkin's lymphomas, B-cell acute and chronic lymphoid leukemias, Burkitt lymphoma, Hodgkin's lymphoma, hairy cell leukemia, acute and chronic myeloid leukemias, T-cell lymphomas and leukemias, multiple myeloma, glioma, Waldenstrom's macroglobulinemia, carcinomas, melanomas, sarcomas, gliomas, and skin cancers. The carcinomas may include carcinomas of the oral cavity, gastrointestinal tract, pulmonary tract, lung, breast, ovary, prostate, uterus, endometrium, cervix, urinary bladder, pancreas, bone, brain, connective tissue, liver, gall bladder, kidney, skin, central nervous system, and testes.

In addition, the invention as defined in the claims may be used for imaging, detection and/or diagnosis of an autoimmune disease or immune dysfunction, for example acute immune thrombocytopenia, chronic immune thrombocytopenia, dermatomyositis, Sydenham's chorea, myasthenia gravis, systemic lupus erythematosus, lupus nephritis, rheumatic fever, polyglandular syndromes, bullous pemphigoid, pemphigus vulgaris, diabetes mellitus (e.g., juvenile diabetes), Henoch-Schonlein purpura, post-streptococcal nephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjögren's syndrome, primary biliary cirrhosis, Hashimoto's thyroiditis, thyrotoxicosis, scleroderma, chronic active hepatitis, polymyositis/dermatomyositis, polychondritis, pemphigus vulgaris; Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis, fibrosing alveolitis, organ transplant rejection or graft-versus-host disease.

In alternative embodiments, the invention as defined in the claims may be used for imaging, detection and/or diagnosis of a metabolic disease, such as type-2 diabetes or amyloidosis, a cardiovascular disease, such as atherosclerosis, or a neurologic disease, such as Alzheimer's disease. Antibodies of use for detection, diagnosis or imaging of such conditions are known in the art, as discussed in more detail below.

### Brief Description of the Drawings

The following Figures are included to illustrate particular embodiments of the invention and are not meant to be limiting as to the scope of the claimed subject matter.
**FIG. 1****.** Structure of IMP-499 (SEQ ID NO:88).
**FIG. 2****.** Structure of The maleimide form of the DYLIGHT® dye 800.
**FIG. 3****.** Schematic structure of RDC018.
**FIG. 4****.** Static PET/CT imaging study of a BALB/c nude mouse with a subcutaneous LS174T tumor (0.1 g) on the right side (arrow), that received 6.0 nmol TF2 and 0.25 nmol A1¹⁸F-IMP-449 (5 MBq) intravenously with a 16 hour interval. The animal was imaged one hour after injection of A1¹⁸F-IMP-449. The panel shows the 3D volume rendering **(A)** posterior view, and cross sections at the tumor region, **(B)** coronal, **(C)** sagittal.

### DETAILED DESCRIPTION

The following definitions are provided to facilitate understanding of the disclosure herein. Terms that are not explicitly defined are used according to their plain and ordinary meaning.

As used herein, "a" or "an" may mean one or more than one of an item.

As used herein, the terms "and" and "or" may be used to mean either the conjunctive or disjunctive. That is, both terms should be understood as equivalent to "and/or" unless otherwise stated.

As used herein, "about" means within plus or minus ten percent of a number. For example, "about 100" would refer to any number between 90 and 110.

An "antibody" refers to a full-length (i.e., naturally occurring or formed by normal immunoglobulin gene fragment recombinatorial processes) immunoglobulin molecule (e.g., an IgG antibody) or an immunologically active (i.e., antigen-binding) portion of an immunoglobulin molecule, like an antibody fragment.

An "antibody fragment" is a portion of an antibody such as F(ab')₂, F(ab)₂, Fab', Fab, Fv, scFv, single domain antibodies (DABs or VHHs) and the like, including half-molecules of IgG4 (van der Neut Kolfschoten et al. (Science 2007; 317(14 Sept):1554-1557). Regardless of structure, an antibody fragment binds with the same antigen that is recognized by the intact antibody. The term "antibody fragment" also includes isolated fragments consisting of the variable regions, such as the "Fv" fragments consisting of the variable regions of the heavy and light chains and recombinant single chain polypeptide molecules in which light and heavy chain variable regions are connected by a peptide linker ("scFv proteins"). As used herein, the term "antibody fragment" does not include fragments such as Fc fragments that do not contain antigen-binding sites.

A "chimeric antibody" is a recombinant protein that contains the variable domains including the complementarity determining regions (CDRs) of an antibody derived from one species, preferably a rodent antibody, while the constant domains of the antibody molecule are derived from those of a human antibody. For veterinary applications, the constant domains of the chimeric antibody may be derived from that of other species, such as a cat or dog.

A "humanized antibody" is a recombinant protein in which the CDRs from an antibody from one species; e.g., a rodent antibody, are transferred from the heavy and light variable chains of the rodent antibody into human heavy and light variable domains. Additional FR amino acid substitutions from the parent, e.g. murine, antibody may be made. The constant domains of the antibody molecule are derived from those of a human antibody.

A "human antibody" is, for example, an antibody obtained from transgenic mice that have been genetically engineered to produce human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A fully human antibody also can be constructed by genetic or chromosomal transfection methods, as well as phage display technology, all of which are known in the art. (See, e.g., McCafferty et al., Nature 348:552-553 (1990) for the production of human antibodies and fragments thereof *in vitro,* from immunoglobulin variable domain gene repertoires from unimmunized donors). In this technique, antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see, e.g. Johnson and Chiswell, Current Opinion in Structural Biology 3:5564-571 (1993). Human antibodies may also be generated by *in vitro* activated B cells. (See, U.S. Pat. Nos. 5,567,610 and 5,229,275).

An "immunoconjugate" is a conjugate of an antibody, antibody fragment, antibody fusion protein, bispecific antibody or multispecific antibody with an atom, molecule, or a higher-ordered structure (e.g., with a carrier, a therapeutic agent, or a diagnostic agent). A "naked antibody" is an antibody that is not conjugated to any other agent.

As used herein, the term "antibody fusion protein" is a recombinantly produced antigen-binding molecule in which an antibody or antibody fragment is covalently linked to another protein or peptide, such as the same or different antibody or antibody fragment. The fusion protein may comprise a single antibody component, a multivalent or multispecific combination of different antibody components or multiple copies of the same antibody component. The fusion protein may additionally comprise an antibody or an antibody fragment and a therapeutic agent.

A "multispecific antibody" is an antibody that can bind simultaneously to at least two targets that are of different structure, e.g., two different antigens, two different epitopes on the same antigen, or a hapten and/or an antigen or epitope. A "multivalent antibody" is an antibody that can bind simultaneously to at least two targets that are of the same or different structure. Valency indicates how many binding arms or sites the antibody has to a single antigen or epitope; i.e., monovalent, bivalent, trivalent or multivalent. The multivalency of the antibody means that it can take advantage of multiple interactions in binding to an antigen, thus increasing the avidity of binding to the antigen. Specificity indicates how many antigens or epitopes an antibody is able to bind; i.e., monospecific, bispecific, trispecific, multispecific. Using these definitions, a natural antibody, e.g., an IgG, is bivalent because it has two binding arms but is monospecific because it binds to one epitope. Multispecific, multivalent antibodies are constructs that have more than one binding site of different specificity. For example, a diabody, where one binding site reacts with one antigen and the other with another antigen.

A "bispecific antibody" is an antibody that can bind simultaneously to two targets which are of different structure.

As used herein, a "peptide" refers to any sequence of naturally occurring or non-naturally occurring amino acids of between 2 and 100 amino acid residues in length, more preferably between 2 and 10, more preferably between 2 and 6 amino acids in length. An "amino acid" may be an L-amino acid, a D-amino acid, an amino acid analogue, an amino acid derivative or an amino acid mimetic.

As used herein, the term "pathogen" includes, but is not limited to fungi, viruses, parasites and bacteria, including but not limited to human immunodeficiency virus (HIV), herpes virus, cytomegalovirus, rabies virus, influenza virus, hepatitis B virus, Sendai virus, feline leukemia virus, Reovirus, polio virus, human serum parvo-like virus, simian virus 40, respiratory syncytial virus, mouse mammary tumor virus, Varicella-Zoster virus. Dengue virus, rubella virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus, blue tongue virus, *Streptococcus agalactiae, Legionella pneumophila, Streptococcus pyogenes, Escherichia coli, Neisseria gonorrhoeae, Neisseria meningitidis, Pneumococcus, Hemophilus influenzae B, Treponema pallidum,* Lyme disease spirochetes, *Pseudomonas aeruginosa, Mycobacterium leprae. Brucella abortus, Mycobacterium tuberculosis* and *Clostridium tetani.*

### Targetable Constructs

According to the invention as defined in the claims, the moiety labeled with a fluorescent probe may comprise a targetable construct comprising a peptide.

Targetable constructs of use can be of diverse structure and are selected not only for the availability of an antibody or fragment that binds with high affinity to the targetable construct, but also for rapid *in vivo* clearance when used within the pretargeting method and bispecific antibodies (bsAb) or multispecific antibodies. Hydrophobic agents are best at eliciting strong immune responses, whereas hydrophilic agents are preferred for rapid *in vivo* clearance. Thus, a balance between hydrophobic and hydrophilic character is established. Sub-units of the targetable construct may be chosen which have opposite solution properties, for example, peptides, which contain amino acids, some of which are hydrophobic and some of which are hydrophilic. Aside from peptides, carbohydrates may also be used.

Peptides having as few as two amino acid residues, preferably two to ten residues, may be used and may also be coupled to other moieties, such as chelating agents. More usually, the targetable construct peptide will have four or more residues. The targetable construct may also comprise unnatural amino acids, e.g., D-amino acids, in the backbone structure to increase the stability of the peptide *in vivo.* In alternative embodiments, other backbone structures such as those constructed from non-natural amino acids or peptoids may be used.

The peptides used as targetable constructs are conveniently synthesized on an automated peptide synthesizer using a solid-phase support and standard techniques of repetitive orthogonal deprotection and coupling. Free side chain groups in the peptide, that are to be used later for conjugation of fluorescent probes or other agents, are advantageously blocked with standard protecting groups such as a Boc group, while N-terminal residues may be acetylated to increase serum stability. Such protecting groups are well known to the skilled artisan. See Greene and Wuts Protective Groups in Organic Synthesis, 1999 (John Wiley and Sons, N.Y.). When the peptides are prepared for later use within the bispecific antibody system, they are advantageously cleaved from the resins to generate the corresponding C-terminal amides, in order to inhibit *in vivo* carboxypeptidase activity. Exemplary methods of peptide synthesis are disclosed in the Examples below.

Where pretargeting with bispecific antibodies is used, the antibody will contain a first binding site for an antigen produced by or associated with a target tissue and a second binding site for a hapten on the targetable construct. Exemplary haptens include, but are not limited to, HSG and In-DTPA. Antibodies raised to the HSG hapten are known (e.g. 679 antibody) and can be easily incorporated into the appropriate bispecific antibody (see, e.g., U.S. Patent Nos. 6,962,702; 7,138,103 and 7,300,644). However, other haptens and antibodies that bind to them are known in the art and may be used, such as In-DTPA and the 734 antibody (e.g., U.S. Patent No.7,534,431).

The skilled artisan will realize that although the majority of targetable constructs disclosed in the Examples below are peptides, other types of molecules may be used as targetable constructs. For example, polymeric molecules, such as polyethylene glycol (PEG) may be easily derivatized with fluorescent probes. Many examples of such carrier molecules are known in the art and may be utilized, including but not limited to polymers, nanoparticles, microspheres, liposomes and micelles. For use in pretargeted delivery, the only requirement is that the carrier molecule comprises one or more derivatizable groups for attachment of a fluorescent probe and one or more hapten moieties to bind to a bispecific or multispecific antibody or other targeting molecule.

### Antibodies

### Target Antigens

Targeting antibodies of use may be specific to or selective for a variety of cell surface or disease-associated antigens. Exemplary target antigens of use for imaging, detection and/or diagnosis of various diseases or conditions, such as a malignant disease, a cardiovascular disease, an infectious disease, an inflammatory disease, an autoimmune disease, a metabolic disease, or a neurological (e.g., neurodegenerative) disease may include carbonic anhydrase IX, CCCL19, CCCL21, CSAp, CD1, CD1a, CD2, CD3, CD4, CD5, CD8, CD11A, CD14, CD15, CD16, CD18, CD19, IGF-1R, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD74, CD79a, CD80, CD83, CD95, CD126, CD133, CD138, CD147, CD154, CXCR4, CXCR7, CXCL12, HIF-1α, AFP, CEACAM5, CEACAM6, c-met, B7, ED-B of fibronectin, Factor H, FHL-1. Flt-3, folate receptor, GROB, HMGB-1, hypoxia inducible factor (HIF), HM1.24, insulin-like growth factor-1 (ILGF-1). IFN-γ, IFN-α, IFN-β. IL-2, IL-4R, IL-6R, IL-13R, IL-15R, IL-17R, IL-18R, IL-6, IL-8, IL-12, IL-15, IL-17, IL-18, IL-25, IP-10, MAGE, mCRP, MCP-1, MIP-1A, MIP-1B, MIF, MUC1, MUC2, MUC3, MUC4, MUC5, NCA-95, NCA-90, la, pancreatic cancer mucin, placental growth factor, p53, PLAGL2, prostatic acid phosphatase, PSA, PRAME, PSMA, P1GF, HM1.24, EGP-1, EGP-2, HLA-DR, tenascin, Le(y), RANTES, T101, TAC, Tn antigen, Thomson-Fhedenreich antigens, tumor necrosis antigens, TNF-a, TRAIL receptor (R1 and R2), VEGFR, EGFR, complement factors C3, C3a, C3b, C5a, C5, and an oncogene product.

In certain embodiments, such as imaging, detection and/or diagnosis of tumors, antibodies of use may target tumor-associated antigens. These antigenic markers may be substances produced by a tumor or may be substances which accumulate at a tumor site, on tumor cell surfaces or within tumor cells. Among such tumor-associated markers are those disclosed by Herberman, "Immunodiagnosis of Cancer", in Fleisher ed., "The Clinical Biochemistry of Cancer", page 347 (American Association of Clinical Chemists, 1979) and in U.S. Pat. Nos. 4,150,149; 4,361,544; and 4,444.741. Reports on tumor associated antigens (TAAs) include Mizukami et al., (2005, Nature Med. 11:992-97); Hatfield et al., (2005, Curr. Cancer Drug Targets 5:229-48); Vallbohmer et al. (2005, J. Clin. Oncol. 23:3536-44); and Ren et al. (2005, Ann. Surg. 242:55-63).

Tumor-associated markers have been categorized by Herberman, supra, in a number of categories including oncofetal antigens, placental antigens, oncogenic or tumor virus associated antigens, tissue associated antigens, organ associated antigens, ectopic hormones and normal antigens or variants thereof. Occasionally, a sub-unit of a tumor-associated marker is advantageously used to raise antibodies having higher tumor-specificity, e.g., the beta-subunit of human chorionic gonadotropin (HCG) or the gamma region of carcinoembryonic antigen (CEA), which stimulate the production of antibodies having a greatly reduced cross-reactivity to non-tumor substances as disclosed in U.S. Pat. Nos. 4,361,644 and 4,444,744.

Another marker of interest is transmembrane activator and CAML-interactor (TACI). See Yu et al. Nat Immunol. 1:252-256 (2000). Briefly, TACI is a marker for B-cell malignancies (e.g., lymphoma). TACI and B-cell maturation antigen (BCMA) are bound by the tumor necrosis factor homolog - a proliferation-inducing ligand (APRIL). APRIL stimulates in vitro proliferation of primary B and T-cells and increases spleen weight due to accumulation of B-cells *in vivo.* APRIL also competes with TALL-I (also called BLyS or BAFF) for receptor binding. Soluble BCMA and TACI specifically prevent binding of APRIL and block APRIL-stimulated proliferation of primary B-cells. BCMA-Fc also inhibits production of antibodies against keyhole limpet hemocyanin and Pneumovax in mice, indicating that APRIL and/or TALL-I signaling via BCMA and/or TACI are required for generation of humoral immunity. Thus, APRIL-TALL-I and BCMA-TACI form a two ligand-two receptor pathway involved in stimulation of B and T-cell function.

Where the disease involves a lymphoma, leukemia or autoimmune disorder, targeted antigens may be selected from the group consisting of CD4, CD5, CD8, CD14, CD15, CD19, CD20, CD21, CD22, CD23, CD25, CD33, CD37, CD38, CD40, CD40L, CD46, CD52, CD54, CD67, CD74, CD79a, CD80, CD126, CD138, CD154, B7, MUC1, Ia, Ii, HM1.24, HLA-DR, tenascin, VEGF, P1GF, ED-B fibronectin, an oncogene (e.g., c-met or PLAGL2), an oncogene product, CD66a-d, necrosis antigens, IL-2, T101, TAG, IL-6, MIF, TRAIL-R1 (DR4) and TRAIL-R2 (DR5).

### Methods for Raising Antibodies

MAbs can be isolated and purified from hybridoma cultures by a variety of well-established techniques. Such isolation techniques include affinity chromatography with Protein-A or Protein-G Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG)," in METHODS IN MOLECULAR BIOLOGY, VOL. 10, pages 79-104 (The Humana Press, Inc. 1992). After the initial raising of antibodies to the immunogen, the antibodies can be sequenced and subsequently prepared by recombinant techniques. Humanization and chimerization of murine antibodies and antibody fragments are well known to those skilled in the art, as discussed below.

### Chimeric Antibodies

A chimeric antibody is a recombinant protein in which the variable regions of a human antibody have been replaced by the variable regions of, for example, a mouse antibody, including the complementarity-determining regions (CDRs) of the mouse antibody. Chimeric antibodies exhibit decreased immunogenicity and increased stability when administered to a subject. General techniques for cloning murine immunoglobulin variable domains are disclosed, for example, in Orlandi et al., Proc. Nat'l Acad. Sci. USA 6: 3833 (1989). Techniques for constructing chimeric antibodies are well known to those of skill in the art. As an example, Leung et al., Hybridoma 13:469 (1994), produced an LL2 chimera by combining DNA sequences encoding the V_{κ} and V_{H} domains of murine LL2, an anti-CD22 monoclonal antibody, with respective human κ and IgG₁ constant region domains.

### Humanized Antibodies

Techniques for producing humanized MAbs are well known in the art (see, e.g., Jones et al., Nature 321: 522 (1986), Riechmann et al., Nature 332: 323 (1988), Verhoeyen et al., Science 239: 1534 (1988), Carter et al., Proc. Nat'l Acad. Sci. USA 89: 4285 (1992), Sandhu, Crit. Rev. Biotech. 12: 437 (1992), and Singer et al., J. Immun. 150: 2844 (1993)). A chimeric or murine monoclonal antibody may be humanized by transferring the mouse CDRs from the heavy and light variable chains of the mouse immunoglobulin into the corresponding variable domains of a human antibody. The mouse framework regions (FR) in the chimeric monoclonal antibody are also replaced with human FR sequences. As simply transferring mouse CDRs into human FRs often results in a reduction or even loss of antibody affinity, additional modification might be required in order to restore the original affinity of the murine antibody. This can be accomplished by the replacement of one or more human residues in the FR regions with their murine counterparts to obtain an antibody that possesses good binding affinity to its epitope. See, for example, Tempest et al., Biotechnology 9:266 (1991) and Verhoeyen et al., Science 239: 1534 (1988). Preferred residues for substitution include FR residues that are located within 1, 2, or 3 Angstroms of a CDR residue side chain, that are located adjacent to a CDR sequence, or that are predicted to interact with a CDR residue.

### Human Antibodies

Methods for producing fully human antibodies using either combinatorial approaches or transgenic animals transformed with human immunoglobulin loci are known in the art (*e.g.,* Mancini et al., 2004, New Microbiol. 27:315-28; Conrad and Scheller, 2005, Comb. Chem. High Throughput Screen. 8:117-26; Brekke and Loset, 2003, Curr. Opin. Pharmacol. 3:544-50). A fully human antibody also can be constructed by genetic or chromosomal transfection methods, as well as phage display technology, all of which are known in the art. See for example, McCafferty et al., Nature 348:552-553 (1990). Such fully human antibodies are expected to exhibit even fewer side effects than chimeric or humanized antibodies and to function *in vivo* as essentially endogenous human antibodies.

In one alternative, the phage display technique may be used to generate human antibodies (*e.g.,* Dantas-Barbosa et al., 2005, Genet. Mol. Res. 4:126-40). Human antibodies may be generated from normal humans or from humans that exhibit a particular disease state, such as cancer (Dantas-Barbosa et al., 2005). The advantage to constructing human antibodies from a diseased individual is that the circulating antibody repertoire may be biased towards antibodies against disease-associated antigens.

In one non-limiting example of this methodology, Dantas-Barbosa et al. (2005) constructed a phage display library of human Fab antibody fragments from osteosarcoma patients. Generally, total RNA was obtained from circulating blood lymphocytes (*Id*.). Recombinant Fab were cloned from the µ, γ and κ chain antibody repertoires and inserted into a phage display library (*Id*.). RNAs were converted to cDNAs and used to make Fab cDNA libraries using specific primers against the heavy and light chain immunoglobulin sequences (Marks et al., 1991, J. Mol. Biol. 222:581-97). Library construction was performed according to Andris-Widhopf et al. (2000, In: Phage Display Laboratory Manual, Barbas et al. (eds), 1st edition, Cold Spring Harbor Laboratory Press, Cold Spring Harbor, NY pp. 9.1 to 9.22). The final Fab fragments were digested with restriction endonucleases and inserted into the bacteriophage genome to make the phage display library. Such libraries may be screened by standard phage display methods, as known in the art. Phage display can be performed in a variety of formats, for their review, see e.g. Johnson and Chiswell, Current Opinion in Structural Biology 3:5564-571 (1993).

Human antibodies may also be generated by *in vitro* activated B-cells. See U.S. Patent Nos. 5,567,610 and 5,229,275. The skilled artisan will realize that these techniques are exemplary and any known method for making and screening human antibodies or antibody fragments may be utilized.

In another alternative, transgenic animals that have been genetically engineered to produce human antibodies may be used to generate antibodies against essentially any immunogenic target, using standard immunization protocols. Methods for obtaining human antibodies from transgenic mice are disclosed by Green et al., Nature Genet. 7:13 (1994), Lonberg et al., Nature 368:856 (1994), and Taylor et al., Int. Immun. 6:579 (1994). A non-limiting example of such a system is the XenoMouse® (*e.g.,* Green et al., 1999, J. Immunol. Methods 231:11-23) from Abgenix (Fremont, CA). In the XenoMouse® and similar animals, the mouse antibody genes have been inactivated and replaced by functional human antibody genes, while the remainder of the mouse immune system remains intact.

The XenoMouse® was transformed with germline-conflgured YACs (yeast artificial chromosomes) that contained portions of the human IgH and Igkappa loci, including the majority of the variable region sequences, along with accessory genes and regulatory sequences. The human variable region repertoire may be used to generate antibody producing B-cells, which may be processed into hybridomas by known techniques. A XenoMouse® immunized with a target antigen will produce human antibodies by the normal immune response, which may be harvested and/or produced by standard techniques discussed above. A variety of strains of XenoMouse® are available, each of which is capable of producing a different class of antibody. Transgenically produced human antibodies have been shown to have therapeutic potential, while retaining the pharmacokinetic properties of normal human antibodies (Green et al., 1999). The skilled artisan will realize that the claimed compositions and methods are not limited to use of the XenoMouse® system but may utilize any transgenic animal that has been genetically engineered to produce human antibodies.

### Known Antibodies

The skilled artisan will realize that the targeting molecules of use for imaging, detection and/or diagnosis may incorporate any antibody or fragment known in the art that has binding specificity for a target antigen associated with a disease state or condition. Such known antibodies include, but are not limited to, hR1 (anti-IGF-1R, U.S. Patent Application Serial No. 12/772,645, filed 3/12/10) hPAM4 (anti-pancreatic cancer mucin, U.S. Patent No. 7,282,567), hA20 (anti-CD20, U.S. Patent No. 7,251,164), hA19 (and-CD19, U.S. Patent No. 7,109,304), hIMMU31 (and-AFP, U.S. Patent No. 7300,655), hLL1 (anti-CD74, U.S. Patent No. 7,312,318), hLL2 (anti-CD22, U.S. Patent No. 7,074,403), hMu-9 (anti-CSAp, U.S. Patent No. 7,387,773), hL243 (anti-HLA-DR, U.S. Patent No. 7,612,180), hMN-14 (anti-CEACAM5, U.S. Patent No. 6,676,924), hMN-15 (anti-CEACAM6, U.S. Patent No. 7,662,378, U.S. Patent Application Serial No. 12/846,062, filed 7/29/10), hRS7 (anti-EGP-1, U.S. Patent No. 7,238,785), hMN-3 (anti-CEACAM6, U.S. Patent No. 7,541,440), Ab124 and Ab125 (anti-CXCR4, U.S. Patent No. 7,138,496).

Anti-TNF-α antibodies are known in the art and may be of use to image, detect and/or diagnose immune diseases, such as autoimmune disease, immune dysfunction (e.g., graft-versus-host disease, organ transplant rejection) or diabetes. Known antibodies against TNF-α include the human antibody CDP571 (Ofei et al., 2011, Diabetes 45:881-85); murine antibodies MTNFAI, M2TNFAI, M3TNFAI, M3TNFABI, M302B and M303 (Thermo Scientific, Rockford, IL); infliximab (Centocor, Malvern, PA); certolizumab pegol (UCB, Brussels, Belgium); and adalimumab (Abbott, Abbott Park, IL). These and many other known anti-TNF-a antibodies may be used in the claimed methods and compositions. Other antibodies of use for immune dysregulatory or autoimmune disease include, but are not limited to, anti-B-cell antibodies such as veltuzumab, epratuzumab, milatuzumab or hL243; tocilizumab (anti-IL-6 receptor); basiliximab (anti-CD25); daclizumab (anti-CD25); efalizumab (anti-CD11a); muromonab-CD3 (anti-CD3 receptor); anti-CD40L (UCB, Brussels, Belgium); natalizumab (anti-α4 integrin) and omalizumab (anti-IgE).

The pharmaceutical composition of the present disclosure may be used to image, detect and/or diagnose a metabolic disease, such amyloidosis, or a neurodegenerative disease, such as Alzheimer's disease. Bapineuzumab is in clinical trials for Alzheimer's disease. Other antibodies proposed for Alzheimer's disease include Alz 50 (Ksiezak-Reding et al., 1987, J Biol Chem 263:7943-47), gantenerumab, and solanezumab. Infliximab, an anti-TNF-α antibody, has been reported to reduce amyloid plaques and improve cognition.

In a preferred embodiment, diseases that may be detected and/or diagnosed using the claimed compositions and methods include cardiovascular diseases, such as fibrin clots, atherosclerosis, myocardial ischemia and infarction. Antibodies to fibrin (e.g., scFv(59D8); T2G1s; MH1) are known and in clinical trials as imaging agents for disclosing said clots and pulmonary emboli, while anti-granulocyte antibodies, such as MN-3, MN-15, anti-NCA95, and anti-CD15 antibodies, can target myocardial infarcts and myocardial ischemia. (See, e.g., U.S. Patent Nos. 5,487,892; 5,632,968; 6,294,173; 7,541,440) Anti-macropahage, anti-low-density lipoprotein (LDL), anti-MIF (e.g., U.S. Patent Nos. 6,645,493; 7,517,523), and anti-CD74 (e.g., hLL1) antibodies can be used to target atherosclerotic plaques. Abciximab (anti-glycoprotein IIb/IIIa) has been approved for adjuvant use for restenosis in percutaneous coronary interventions and unstable angina (Waldmann et al., 2000, Hematol 1:394-408). Commercially available monoclonal antibodies to leukocyte antigens are represented by: OKT anti-T-cell monoclonal antibodies (available from Ortho Pharmaceutical Company) which bind to normal T-lymphocytes; the monoclonal antibodies produced by the hybridomas having the ATCC accession numbers HB44, HB55, HB12, HB78 and HB2; G7E11, W8E7, NKP15 and GO22 (Becton Dickinson); NEN9.4 (New England Nuclear); and FMC11 (Sera Labs). A description of antibodies against fibrin and platelet antigens is contained in Knight, Semin. Nucl. Med., 20:52-67 (1990).

Other antibodies that may be used include antibodies against infectious disease agents, such as bacteria, viruses, mycoplasms or other pathogens. Many antibodies against such infectious agents are known in the art and any such known antibody may be used in the claimed methods and compositions. For example, antibodies against the gp120 glycoprotein antigen of human immunodeficiency virus I (HIV-1) are known. See, e.g., Rossi et al., Proc. Natl. Acad. Sci. USA. 86:8055-8058, 1990. Known anti-HIV antibodies include the anti-envelope antibody described by Johansson et al. (AIDS. 2006 Oct 3;20(15):1911-5), as well as the anti-HIV antibodies described and sold by Polymun (Vienna, Austria), also described in U.S. Patent 5,831,034, U.S. patent 5,911,989, and Vcelar et al., AIDS 2007; 21(16):2161-2170 and Joos et al., Antimicrob. Agents Chemother. 2006; 50(5):1773-9. Antibodies against hepatitis virus are also known and may be utilized (e.g., Dagan and Eren, Curr Opin Mol Ther, 2003, 5:148-55; Keck et al., 2008, Curr Top Microbiol Immunol 317:1-38; El-Awady et al., 2006,12:2530-35).

Antibodies against malaria parasites can be directed against the sporozoite, merozoite, schizont and gametocyte stages. Monoclonal antibodies have been generated against sporozoites (cirumsporozoite antigen), and have been shown to neutralize sporozoites in vitro and in rodents (N. Yoshida et al., Science 207:71-73, 1980). Several groups have developed antibodies to T. gondii, the protozoan parasite involved in toxoplasmosis (Kasper et al., J. Immunol. 129:1694-1699, 1982; Id., 30:2407-2412, 1983). Antibodies have been developed against schistosomular surface antigens and have been found to act against schistosomulae *in vivo* or *in vitro* (Simpson et al., Parasitology, 83:163-177, 1981; Smith et al., Parasitology, 84:83-91, 1982: Gryzch et al., J. Immunol., 129:2739-2743, 1982; Zodda et al., J. Immunol. 129:2326-2328, 1982; Dissous et al., J. immunol., 129:2232-2234, 1982)

Trypanosoma cruzi is the causative agent of Chagas' disease, and is transmitted by blood-sucking reduviid insects. An antibody has been generated that specifically inhibits the differentiation of one form of the parasite to another (epimastigote to trypomastigote stage) *in vitro,* and which reacts with a cell-surface glycoprotein; however, this antigen is absent from the mammalian (bloodstream) forms of the parasite (Sher et al., Nature, 300:639-640, 1982).

Anti-fungal antibodies are known in the art, such as anti-Sclerotinia antibody (U.S. Patent 7,910,702); antiglucuronoxylomannan antibody (Zhong and Priofski, 1998, Clin Diag Lab Immunol 5:58-64); anti-Candida antibodies (Matthews and Burnie, 2001, 2:472-76); and anti-glycosphingolipid antibodies (Toledo et al., 2010, BMC Microbiol 10:47).

Suitable antibodies have been developed against most of the microorganism (bacteria, viruses, protozoa, fungi, other parasites) responsible for the majority of infections in humans, and many have been used previously for *in vitro* diagnostic purposes. These antibodies, and newer antibodies that can be generated by conventional methods, are appropriate for use in the present invention.

Where bispecific antibodies are used, the second MAb may be selected from any anti-hapten antibody known in the art, including but not limited to h679 (U.S. Patent No. 7,429,381) and 734 (U.S. Patent Nos. 7,429,381; 7,563,439; 7,666,415; and 7,534,431).

Various other antibodies of use are known in the art (e.g., U.S. Patent Nos. 5,686,072; 5,874,540; 6,107,090; 6,183,744; 6,306,393; 6,653,104; 6,730.300; 6,899,864; 6,926,893; 6,962,702; 7,074,403; 7,230,084; 7,238,785; 7,238,786; 7,256,004; 7,282,567; 7,300,655; 7,312,318; 7,585,491; 7,612,180; 7,642,239 and U.S. Patent Application Publ. No. 20060193865.) Such known antibodies are of use for detection and/or imaging of a variety of disease states or conditions (e.g., hMN-14 or TF2 (CEA-expressing carcinomas), hA20 or TF-4 (lymphoma), hPAM4 or TF-10 (pancreatic cancer), RS7 (lung, breast, ovarian, prostatic cancers), hMN-15 or hMN3 (inflammation), anti-gp120 and/or anti-gp41 (HIV), anti-platelet and anti-thrombin (clot imaging), anti-myosin (cardiac necrosis), anti-CXCR4 (cancer and inflammatory disease)).

Antibodies of use may be commercially obtained from a wide variety of known sources. For example, a variety of antibody secreting hybridoma lines are available from the American Type Culture Collection (ATCC, Manassas, VA). A large number of antibodies against various disease targets, including but not limited to tumor-associated antigens, have been deposited at the ATCC and/or have published variable region sequences and are available for use in the claimed methods and compositions. See, e.g., U.S. Patent Nos. 7,312,318; 7,282,567; 7,151,164; 7,074,403; 7,060,802; 7,056,509; 7,049,060; 7,045,132; 7,041,803; 7,041,802; 7,041,293; 7,038,018; 7,037,498; 7,012,133; 7,001,598; 6,998,468; 6,994,976; 6,994,852; 6,989,241; 6,974,863; 6,965,018; 6,964,854; 6,962,981; 6,962,813; 6,956,107; 6,951,924; 6,949,244; 6,946,129; 6,943,020; 6,939,547; 6,921,645; 6,921,645; 6,921,533; 6,919,433; 6,919,078; 6,916,475; 6,905,681; 6,899,879; 6,893,625; 6,887,468; 6,887,466; 6,884,594; 6,881,405; 6,878,812; 6,875,580; 6,872,568; 6,867,006; 6,864,062; 6,861,511; 6,861,227; 6,861,226; 6,838,282; 6,835,549; 6,835,370; 6,824,780; 6,824,778; 6,812,206; 6,793,924; 6,783,758; 6,770,450; 6,767,711; 6,764,688; 6,764,681; 6,764,679; 6,743,898; 6,733,981; 6,730,307; 6,720,155; 6,716,966; 6,709,653; 6,693,176; 6,692,908; 6,689,607; 6,689,362; 6,689,355; 6,682,737; 6,682,736; 6,682,734; 6,673,344; 6,653,104; 6,652,852; 6,635,482; 6,630,144; 6,610,833; 6,610,294; 6,605,441; 6,605,279; 6,596,852; 6,592,868; 6,576,745; 6,572;856; 6,566,076; 6,562,618; 6,545,130; 6,544,749; 6,534,058; 6,528,625; 6,528,269; 6,521,227; 6,518,404; 6,511,665; 6,491,915; 6,488,930; 6,482,598; 6,482,408; 6,479,247; 6,468,531; 6,468,529; 6,465,173; 6,461,823; 6,458,356; 6,455,044; 6,455,040; 6,451,310; 6,444,206; 6,441,143; 6,432,404; 6,432,402; 6,419,928; 6,413,726; 6,406,694; 6,403,770; 6,403,091; 6,395,276; 6,395,274; 6,387,350; 6,383,759; 6,383,484; 6,376,654; 6,372,215; 6,359,126; 6,355,481; 6,355,444; 6,355,245; 6,355,244; 6,346,246; 6,344,198; 6,340,571; 6,340,459; 6,331,175; 6,306,393; 6,254,868; 6,187,287; 6,183,744; 6,129,914; 6,120,767; 6,096,289; 6,077,499; 5,922,302; 5,874,540; 5,814,440; 5,798,229; 5,789,554; 5,776,456; 5,736,119; 5,716,595; 5,677,136; 5,587,459; 5,443,953; 5,525,338. These are exemplary only and a wide variety of other antibodies and their hybridomas are known in the art. The skilled artisan will realize that antibody sequences or antibody-secreting hybridomas against almost any disease-associated antigen may be obtained by a simple search of the ATCC, NCBI and/or USPTO databases for antibodies against a selected disease-associated target of interest. The antigen binding domains of the cloned antibodies may be amplified, excised, ligated into an expression vector, transfected into an adapted host cell and used for protein production, using standard techniques well known in the art (see, e.g., U.S. Patent Nos. 7,531,327; 7,537,930; 7,608,425 and 7,785,880).

### Antibody Fragments

Antibody fragments which recognize specific epitopes can be generated by known techniques. The antibody fragments are antigen binding portions of an antibody, such as F(ab')₂, Fab', F(ab)₂, Fab, Fv, sFv and the like. F(ab')₂ fragments can be produced by pepsin digestion of the antibody molecule and Fab' fragments can be generated by reducing disulfide bridges of the F(ab')₂ fragments. Alternatively, Fab' expression libraries can be constructed (Huse et al., 1989, Science, 246:1274-1281) to allow rapid and easy identification of monoclonal Fab' fragments with the desired specificity. An antibody fragment can be prepared by proteolytic hydrolysis of the full length antibody or by expression in *E. coli* or another host of the DNA coding for the fragment. These methods are described, for example, by Goldenberg, U.S. Patent Nos. 4,036,945 and 4,331,647 and references contained therein. Also, see Nisonoff et al., Arch Biochem. Biophys. 89: 230 (1960); Porter, Biochem. J. 73: 119 (1959), Edelman et al., in METHODS IN ENZYMOLOGY VOL. 1, page 422 (Academic Press 1967), and Coligan at pages 2.8.1-2.8.10 and 2.10.-2.10.4.

A single chain Fv molecule (scFv) comprises a V_{L} domain and a V_{H} domain. The V_{L} and V_{H} domains associate to form a target binding site. These two domains are further covalently linked by a peptide linker (L). Methods for making scFv molecules and designing suitable peptide linkers are described in US Patent No. 4,704,692, US Patent No. 4,946,778, R. Raag and M. Whitlow, "Single Chain Fvs." FASEB Vol 9:73-80 (1995) and R.E. Bird and B.W. Walker, "Single Chain Antibody Variable Regions," TIBTECH, Vol 9: 132-137 (1991).

An scFv library with a large repertoire can be constructed by isolating V-genes from non-immunized human donors using PCR primers corresponding to all known V_{H}, Vₖₐₚₚₐ and V₈₀ gene families. See, e.g., Vaughn et al., Nat. Biotechnol., 14: 309-314 (1996). Following amplification, the Vₖₐₚₚₐ and V_{lambda} pools are combined to form one pool. These fragments are ligated into a phagemid vector. The scFv linker is then ligated into the phagemid upstream of the V_{L} fragment. The V_{H} and linker-V_{L} fragments are amplified and assembled on the J_{H} region. The resulting V_{H}-linker-V_{L} fragments are ligated into a phagemid vector. The phagemid library can be panned for binding to the selected antigen.

Other antibody fragments, for example single domain antibody fragments, are known in the art and may be used in the claimed constructs. Single domain antibodies (VHH) may be obtained, for example, from camels, alpacas or Ilamas by standard immunization techniques. (See, e.g., Muyldermans et al., TIBS 26:230-235, 2001; Yau et al., J Immunol Methods 281:161-75, 2003; Maass et al., J Immunol Methods 324:13-25, 2007). The VHH may have potent antigen-binding capacity and can interact with novel epitopes that are inaccessible to conventional VH-VL pairs. (Muyldermans et al., 2001) Alpaca serum IgG contains about 50% camelid heavy chain only IgG antibodies (Cabs) (Maass et al., 2007). Alpacas may be immunized with known antigens and VHHs can be isolated that bind to and neutralize the target antigen (Maass et al., 2007). PCR primers that amplify virtually all alpaca VHH coding sequences have been identified and may be used to construct alpaca VHH phage display libraries, which can be used for antibody fragment isolation by standard biopanning techniques well known in the art (Maass et al., 2007). These and other known antigen-binding antibody fragments may be utilized in the claimed methods and compositions.

### General techniques for antibody cloning and production

Various techniques, such as production of chimeric or humanized antibodies, may involve procedures of antibody cloning and construction. The antigen-binding Vκ (variable light chain) and V_{H} (variable heavy chain) sequences for an antibody of interest may be obtained by a variety of molecular cloning procedures, such as RT-PCR, 5'-RACE, and cDNA library screening. The V genes of a MAb from a cell that expresses a murine MAb can be cloned by PCR amplification and sequenced. To confirm their authenticity, the cloned V_{L} and V_{H} genes can be expressed in cell culture as a chimeric Ab as described by Orlandi et al., (Proc. Natl. Acad Sci., USA, 86: 3833 (1989)). Based on the V gene sequences, a humanized MAb can then be designed and constructed as described by Leung et al. (Mol. Immunol., 32: 1413 (1995)).

cDNA can be prepared from any known hybridoma line or transfected cell line producing a murine MAb by general molecular cloning techniques (Sambrook et al., Molecular Cloning, A laboratory manual, 2nd Ed (1989)). The Vκ sequence for the MAb may be amplified using the primers VK1BACK and VK1FOR (Orlandi *et al.,* 1989) or the extended primer set described by Leung et al. (BioTechniques, 15: 286 (1993)). The V_{H} sequences can be amplified using the primer pair VH1BACK/VH1FOR (Orlandi *et al.,* 1989) or the primers annealing to the constant region of murine IgG described by Leung et al. (Hybridoma, 13:469 (1994)). Humanized V genes can be constructed by a combination of long oligonucleotide template syntheses and PCR amplification as described by Leung et al. (Mol. Immunol., 32: 1413 (1995)).

PCR products for Vκ can be subcloned into a staging vector, such as a pBR327-based staging vector, VKpBR, that contains an Ig promoter, a signal peptide sequence and convenient restriction sites. PCR products for V_{H} can be subcloned into a similar staging vector, such as the pBluescript-based VHpBS. Expression cassettes containing the Vκ and V_{H} sequences together with the promoter and signal peptide sequences can be excised from VKpBR and VHpBS and ligated into appropriate expression vectors, such as pKh and pG1g, respectively (Leung et al., Hybridoma, 13:469 (1994)). The expression vectors can be co-transfected into an appropriate cell and supernatant fluids monitored for production of a chimeric, humanized or human MAb. Alternatively, the Vκ and V_{H} expression cassettes can be excised and subcloned into a single expression vector, such as pdHL2, as described by Gillies et al. (J. Immunol. Methods 125:191 (1989) and also shown in Losman et al., Cancer, 80:2660 (1997)).

In an alternative embodiment, expression vectors may be transfected into host cells that have been pre-adapted for transfection, growth and expression in serum-free medium. Exemplary cell lines that may be used include the Sp/EEE, Sp/ESF and Sp/ESF-X cell lines (see, e.g., U.S. Patent Nos. 7,531,327; 7,537,930 and 7,608,425). These exemplary cell lines are based on the Sp2/0 myeloma cell line, transfected with a mutant Bcl-EEE gene, exposed to methotrexate to amplify transfected gene sequences and pre-adapted to serum-free cell line for protein expression.

### Bispecific and Multispecific Antibodies

Certain embodiments concern pretargeting methods with bispecific antibodies and hapten-bearing targetable constructs. Numerous methods to produce bispecific or multispecific antibodies are known, as disclosed, for example, in U.S. Patent No. 7,405,320. Bispecific antibodies can be produced by the quadroma method, which involves the fusion of two different hybridomas, each producing a monoclonal antibody recognizing a different antigenic site (Milstein and Cuello, Nature, 1983; 305:537-540).

Another method for producing bispecific antibodies uses heterobifunctional crosslinkers to chemically tether two different monoclonal antibodies (Staerz, et al. Nature. 1985; 314:628-631; Perez, et al. Nature. 1985; 316:354-356). Bispecific antibodies can also be produced by reduction of each of two parental monoclonal antibodies to the respective half molecules, which are then mixed and allowed to reoxidize to obtain the hybrid structure (Staerz and Bevan. Proc Natl Acad Sci U S A. 1986; 83:1453-1457). Other methods include improving the efficiency of generating hybrid hybridomas by gene transfer of distinct selectable markers via retrovirus-derived shuttle vectors into respective parental hybridomas, which are fused subsequently (DeMonte, et al. Proc Natl Acad Sci U S A. 1990, 87:2941-2945); or transfection of a hybridoma cell line with expression plasmids containing the heavy and light chain genes of a different antibody.

Cognate V_{H} and V_{L} domains can be joined with a peptide linker of appropriate composition and length (usually consisting of more than 12 amino acid residues) to form a single-chain Fv (scFv), as discussed above. Reduction of the peptide linker length to less than 12 amino acid residues prevents pairing of V_{H} and V_{L} domains on the same chain and forces pairing of V_{H} and V_{L} domains with complementary domains on other chains, resulting in the formation of functional multimers. Polypeptide chains of V_{H} and V_{L} domains that are joined with linkers between 3 and 12 amino acid residues form predominantly dimers (termed diabodies). With linkers between 0 and 2 amino acid residues, trimers (termed triabody) and tetramers (termed tetrabody) are favored, but the exact patterns of oligomerization appear to depend on the composition as well as the orientation of V-domains (V_{H}-linker-V_{L} or V_{L}-linker-V_{H}), in addition to the linker length.

These techniques for producing multispecific or bispecific antibodies exhibit various difficulties in terms of low yield, necessity for purification, low stability or the labor-intensiveness of the technique. More recently, a technique for making bispecific or multispecific DOCK-AND-LOCK™ (DNL™) complexes, discussed in more detail below, has been utilized to produce combinations of virtually any desired antibodies, antibody fragments and other effector molecules. The DNL™ technique allows the assembly of monospecific, bispecific or multispecific antibodies, either as naked antibody moieties or in combination with a wide range of other effector molecules such as immunomodulators, enzymes, chemotherapeutic agents, chemokines, cytokines, diagnostic agents, therapeutic agents, radionuclides, imaging agents, anti-angiogenic agents, growth factors, oligonucleotides, hormones, peptides, toxins, pro-apoptotic agents, or a combination thereof. Any of the techniques known in the art for making bispecific or multispecific antibodies may be utilized in the practice of the presently claimed methods.

### DOCK-AND-LOCK™ (DNL™)

In preferred embodiments, a bivalent or multivalent antibody is formed as a DOCK-AND-LOCK™ (DNL™) complex (see, e.g., U.S. Patent Nos. 7,521,056; 7,527,787; 7,534,866; 7,550,143 and 7,666,400.) Generally, the technique takes advantage of the specific and high-affinity binding interactions that occur between a dimerization and docking domain (DDD) sequence of the regulatory (R) subunits of cAMP-dependent protein kinase (PKA) and an anchor domain (AD) sequence derived from any of a variety of AKAP proteins (Baillie et al., FEBS Letters. 2005; 579: 3264. Wong and Scott, Nat. Rev. Mol. Cell Biol. 2004; 5: 959). The DDD and AD peptides may be attached to any protein, peptide or other molecule. Because the DDD sequences spontaneously dimerize and bind to the AD sequence, the technique allows the formation of complexes between any selected molecules that may be attached to DDD or AD sequences.

Although the standard DNL™ complex comprises a trimer with two DDD-linked molecules attached to one AD-linked molecule, variations in complex structure allow the formation of dimers, trimers, tetramers, pentamers, hexamers and other multimers. In some embodiments, the DNL™ complex may comprise two or more antibodies, antibody fragments or fusion proteins which bind to the same antigenic determinant or to two or more different antigens. The DNL™ complex may also comprise one or more other effectors, such as proteins, peptides, immunomodulators, cytokines, interleukins, interferons, binding proteins, peptide ligands, carrier proteins, toxins, ribonucleases such as onconase, inhibitory oligonucleotides such as siRNA, antigens or xenoantigens, polymers such as PEG, enzymes, therapeutic agents, hormones, cytotoxic agents, anti-angiogenic agents, pro-apoptotic agents or any other molecule or aggregate.

PKA, which plays a central role in one of the best studied signal transduction pathways triggered by the binding of the second messenger cAMP to the R subunits, was first isolated from rabbit skeletal muscle in 1968 (Walsh et al., J. Biol. Chem. 1968;243:3763). The structure of the holoenzyme consists of two catalytic subunits held in an inactive form by the R subunits (Taylor, J. Biol. Chem. 1989;264:8443). Isozymes of PKA are found with two types of R subunits (RI and RII), and each type has α and β isoforms (Scott, Pharmacol. Ther. 1991;50:123). Thus, the four isoforms of PKA regulatory subunits are RIα, RIβ, RIIα and PIIβ. The R subunits have been isolated only as stable dimers and the dimerization domain has been shown to consist of the first 44 amino-terminal residues of RIIα (Newlon et al., Nat. Struct. Biol. 1999; 6:222). As discussed below, similar portions of the amino acid sequences of other regulatory subunits are involved in dimerization and docking, each located near the N-terminal end of the regulatory subunit. Binding of cAMP to the R subunits leads to the release of active catalytic subunits for a broad spectrum of serine/threonine kinase activities, which are oriented toward selected substrates through the compartmentalization of PKA via its docking with AKAPs (Scott et al., J. Biol. Chem. 1990;265;21561)

Since the first AKAP, microtubule-associated protein-2, was characterized in 1984 (Lohmann et al., Proc. Natl. Acad. Sci USA. 1984; 81:6723), more than 50 AKAPs that localize to various sub-cellular sites, including plasma membrane, actin cytoskeleton, nucleus, mitochondria, and endoplasmic reticulum, have been identified with diverse structures in species ranging from yeast to humans (Wong and Scott, Nat. Rev. Mol. Cell Biol. 2004;5:959). The AD of AKAPs for PKA is an amphipathic helix of 14-18 residues (Carr et al., J. Biol. Chem. 1991;266:14188). The amino acid sequences of the AD are quite varied among individual AKAPs, with the binding affinities reported for RII dimers ranging from 2 to 90 nM (Alto et al., Proc. Natl. Acad. Sci. USA. 2003;100:4445). AKAPs will only bind to dimeric R subunits. For human RIIα, the AD binds to a hydrophobic surface formed by the 23 amino-terminal residues (Colledge and Scott, Trends Cell Biol. 1999; 6:216). Thus, the dimerization domain and AKAP binding domain of human RIIα are both located within the same N-terminal 44 amino acid sequence (Newlon et al., Nat. Struct. Biol. 1999;6:222; Newlon et al., EMBO J. 2001;20:1651), which is termed the DDD herein.

We have developed a platform technology to utilize the DDD of human PKA regulatory subunits and the AD of AKAP as an excellent pair of linker modules for docking any two entities, referred to hereafter as **A** and **B,** into a noncovalent complex, which could be further locked into a DNL™ complex through the introduction of cysteine residues into both the DDD and AD at strategic positions to facilitate the formation of disulfide bonds. The general methodology of the approach is as follows. Entity **A** is constructed by linking a DDD sequence to a precursor of **A,** resulting in a first component hereafter referred to as a. Because the DDD sequence would effect the spontaneous formation of a dimer, **A** would thus be composed of **a₂**. Entity **B** is constructed by linking an AD sequence to a precursor of **B,** resulting in a second component hereafter referred to as **b.** The dimeric motif of DDD contained in **a₂** will create a docking site for binding to the AD sequence contained in **b,** thus facilitating a ready association of **a₂** and **b** to form a binary, trimeric complex composed of **a₂b.** This binding event is made irreversible with a subsequent reaction to covalently secure the two entities via disulfide bridges, which occurs very efficiently based on the principle of effective local concentration because the initial binding interactions should bring the reactive thiol groups placed onto both the DDD and AD into proximity (Chmura et al., Proc. Natl. Acad. Sci. USA. 2001;98:8480) to ligate site-specifically. Using various combinations of linkers, adaptor modules and precursors, a wide variety of DNL™ constructs of different stoichiometry may be produced and used (see, e.g., U.S. Nos. 7,550,143; 7,521,056; 7,534,866; 7,527,787 and 7,666,400.)

By attaching the DDD and AD away from the functional groups of the two precursors, such site-specific ligations are also expected to preserve the original activities of the two precursors. This approach is modular in nature and potentially can be applied to link, site-specifically and covalently, a wide range of substances, including peptides, proteins, antibodies, antibody fragments, and other effector moieties with a wide range of activities. Utilizing the fusion protein method of constructing AD and DDD conjugated effectors described in the Examples below, virtually any protein or peptide may be incorporated into a DNL™ construct. However, the technique is not limiting and other methods of conjugation may be utilized.

A variety of methods are known for making fusion proteins, including nucleic acid synthesis, hybridization and/or amplification to produce a synthetic double-stranded nucleic acid encoding a fusion protein of interest. Such double-stranded nucleic acids may be inserted into expression vectors for fusion protein production by standard molecular biology techniques (see, e.g. Sambrook et al., Molecular Cloning, A laboratory manual, 2nd Ed, 1989). In such preferred embodiments, the AD and/or DDD moiety may be attached to either the N-terminal or C-terminal end of an effector protein or peptide. However, the skilled artisan will realize that the site of attachment of an AD or DDD moiety to an effector moiety may vary, depending on the chemical nature of the effector moiety and the part(s) of the effector moiety involved in its physiological activity. Site-specific attachment of a variety of effector moieties may be performed using techniques known in the art, such as the use of bivalent cross-linking reagents and/or other chemical conjugation techniques.

### Structure-Function Relationships in AD and DDD Moieties

For different types of DNL™ constructs, different AD or DDD sequences may be utilized. Exemplary DDD and AD sequences are provided below.
*DDD1* SHIQIPPGLTELLQGYTVEVLRQQPPDLVEFAVEYFTRLREARA (SEQ ID NO:1)
*DDD2* CGHIQIPPGLTELLQGYTVEVLRQQPPDLVEFAVEYFTRLREARA (SEQ ID NO:2)
*AD1* QIEYLAKQIVDNAIQQA (SEQ ID NO:3)
*AD2* CGQIEYLAKQIVDNAIQQAGC (SEQ ID NO:4)

The skilled artisan will realize that DDD1 and DDD2 are based on the DDD sequence of the human RIIα isoform of protein kinase A. However, in alternative embodiments, the DDD and AD moieties may be based on the DDD sequence of the human RIα form of protein kinase A and a corresponding AKAP sequence, as exemplified in DDD3, DDD3C and AD3 below.
*DDD3* SLRECELYVQKHNIQALLKDSIVQLCTARPERPMAFLREYFERLEKEEAK (SEQ ID NO:5)
*DDD3C*
*AD3* CGFEELAWKIAKMIWSDVFQQGC (SEQ ID NO:7)

In other alternative embodiments, other sequence variants of AD and/or DDD moieties may be utilized in construction of the DNL™ complexes. For example, there are only four variants of human PKA DDD sequences, corresponding to the DDD moieties of PKA RIα, RIIα, RIβ and RIIβ. The RIIα DDD sequence is the basis of DDD1 and DDD2 disclosed above. The four human PKA DDD sequences are shown below. The DDD sequence represents residues 1-44 of RIIα, 1-44 of RIIβ, 12-61 of RIa and 13-66 of RIβ. (Note that the sequence of DDD1 is modified slightly from the human PKA RIIα DDD moiety.)
*PKA RIα* SLRECELYVQKHNIQALLKDVSIVQLCTARPERPMAFLREYFEKLEKEEAK (SEQ ID NO:8)
*PKA RIβ* SLKGCELYVQLHGIQQVLKDCIVHLCISKPERPMKFLREHFEKLEKEENRQILA (SEQ ID NO:9)
*PKA RII*α SHIQIPPGLTELLQGYTVEVGQQPPDLVDFAVEYFTRLREARRQ (SEQ ID NO: 10)
*PKA RIIβ* SIEIPAGLTELLQGFTVEVLRHQPADLLEFALQHFTRLQQENER (SEQ ID NO:11)

The structure-function relationships of the AD and DDD domains have been the subject of investigation. (See, e.g., Bums-Hamuro et al., 2005, Protein Sci 14:2982-92; Carr et al., 2001, J Biol Chem 276:17332-38; Alto et al., 2003, Proc Natl Acad Sci USA 100:4445-50; Hundsrucker et al., 2006, Biochem J 396:297-306; Stokka et al., 2006, Biochem J 400:493-99; Gold et al., 2006, Mol Cell 24:383-95; Kinderman et al., 2006, Mol Cell 24:397-408.)

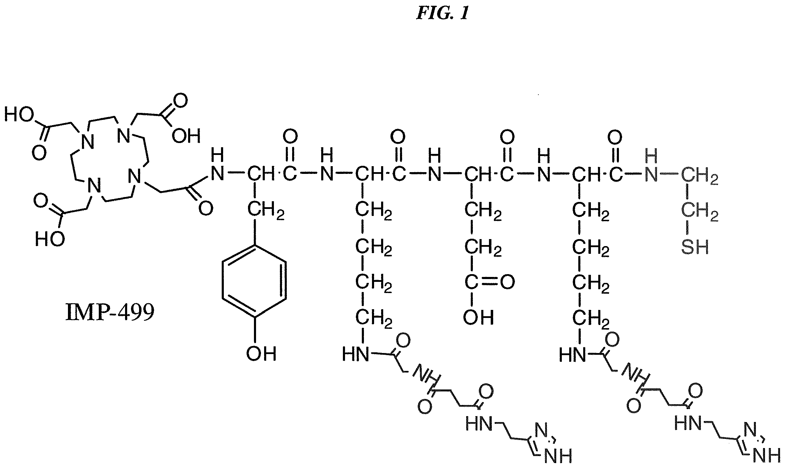

For example, Kinderman et al. (2006, Mol Cell 24:397-408) examined the crystal structure of the AD-DDD binding interaction and concluded that the human DDD sequence contained a number of conserved amino acid residues that were important in either dimer formation or AKAP binding, underlined in SEQ ID NO:1 below. (See Figure 1 of Kinderman et al., 2006.) The skilled artisan will realize that in designing sequence variants of the DDD sequence, one would desirably avoid changing any of the underlined residues, while conservative amino acid substitutions might be made for residues that are less critical for dimerization and AKAP binding. SHIQIPPGLTELLQGYTVEVLRQQPPDLVEFAVEYFTRLREARA (SEQ ID NO:1)

As discussed in more detail below, conservative amino acid substitutions have been characterized for each of the twenty common L-amino acids. Thus, based on the data of Kinderman (2006) and conservative amino acid substitutions, potential alternative DDD sequences based on SEQ ID NO:1 are shown in **Table 1.** In devising **Table 1,** only highly conservative amino acid substitutions were considered. For example, charged residues were only substituted for residues of the same charge, residues with small side chains were substituted with residues of similar size, hydroxyl side chains were only substituted with other hydroxyls, etc. Because of the unique effect of proline on amino acid secondary structure, no other residues were substituted for proline. A limited number of such potential alternative DDD moiety sequences are shown in SEQ ID NO:12 to SEQ ID NO:31 below. The skilled artisan will realize that an almost unlimited number of alternative species within the genus of DDD moieties can be constructed by standard techniques, for example using a commercial peptide synthesizer or well known site-directed mutagenesis techniques. The effect of the amino acid substitutions on AD moiety binding may also be readily determined by standard binding assays, for example as disclosed in Alto et al. (2003, Proc Natl Acad Sci USA 100:4445-50).

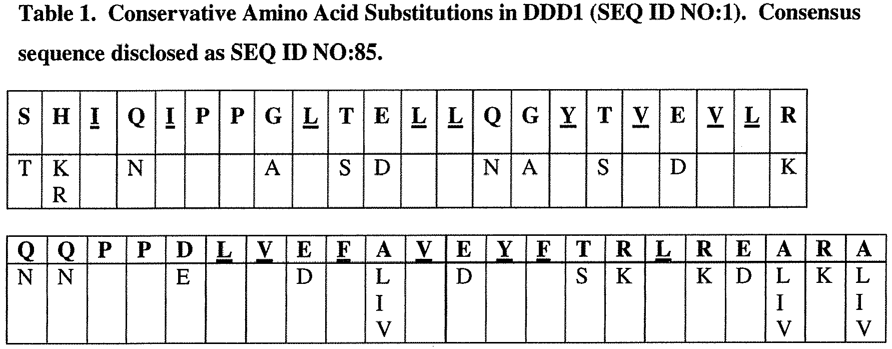

THIQIPPGLTELLQGYTVEVLRQQPPDLVEFAVEYFTRLREARA (SEQ ID NO:12)
SKIQIPPGLTELLQGYTVEVLRQQPPDLVEFAVEYFTRLREARA (SEQ ID NO:13)
SRIQIPPGLTELLQGYTVEVLRQQPPDLVEFAVEYFTRLREARA (SEQ ID NO:14)
SHINIPPGLTELLQGYTVEVLRQQPPDLVEFAVEYFTRLREARA (SEQ ID NO:15)
SHIQIPPALTELLQGYTVEVLRQQPPDLVEFAVEYFTRLREARA (SEQ ID NO:16)
SHIQIPPGLSELLQGYTVEVLRQQPPDLVEFAVEYFTRLREARA (SEQ ID NO:17)
SHIQIPPGLTDLLQGYTVEVLRQQPPDLVEFAVEYFTRLREARA (SEQ ID NO:18)
SHIQIPPGLTELLNGYTVEVLRQQPPDLVEFAVEYFTRLREARA (SEQ ID NO:19)
SHIQIPPGLTELLQAYTVEVLRQQPPDLVEFAVEYFTRLREARA (SEQ ID NO:20)
SHIQIPPGLTELLQGYSVEVLRQQPPDLVEFAVEYFTRLREARA (SEQ ID NO:21)
SHIQIPPGLTELLQGYTVDVLRQQPPDLVEFAVEYFTRLREARA (SEQ ID NO:22)
SHIQIPPGLTELLQGYTVEVLKQQPPDLVEFAVEYFTRLREARA (SEQ ID NO:23)
SHIQIPPGLTELLQGYTVEVLRNQPPDLVEFAVEYFTRLREARA (SEQ ID NO:24)
SHIQIPPGLTELLQGYTVEVLRQNPPDLVEFAVEYFTRLREARA (SEQ ID NO:25)
SHIQIPPGLTELLQGYTVEVLRQQPPELVEFAVEYFTRLREARA (SEQ ID NO:26)
SHIQIPPGLTELLQGYTVEVLRQQPPDLVDFAVEYFTRLREARA (SEQ ID NO:27)
SHIQIPPGLTELLQGYTVEVLRQQPPDLVEFLVEYFTRLREARA (SEQ ID NO:28)
SHIQIPPGLTELLQGYTVEVLRQQPPDLVEFIVEYFTRLREARA (SEQ ID NO:29)
SHIQIPPGLTELLQGYTVEVLRQQPPDLVEFVVEYFTRLREARA (SEQ ID NO:30)
SHIQIPPGLTELLQGYTVEVLRQQPPDLVEFAVDYFTRLREARA (SEQ ID NO:31)

Alto et al. (2003, Proc Natl Acad Sci USA 100:4445-50) performed a bioinformatic analysis of the AD sequence of various AKAP proteins to design an RII selective AD sequence called AKAP-IS (SEQ ID NO:3), with a binding constant for DDD of 0.4 nM. The AKAP-IS sequence was designed as a peptide antagonist of AKAP binding to PKA. Residues in the AKAP-IS sequence where substitutions tended to decrease binding to DDD are underlined in SEQ ID NO:3 below. The skilled artisan will realize that in designing sequence variants of the AD sequence, one would desirably avoid changing any of the underlined residues, while conservative amino acid substitutions might be made for residues that are less critical for DDD binding. **Table 2** shows potential conservative amino acid substitutions in the sequence of AKAP-IS (AD1, SEQ ID NO:3), similar to that shown for DDD1 (SEQ ID NO:1) in **Table 1** above.

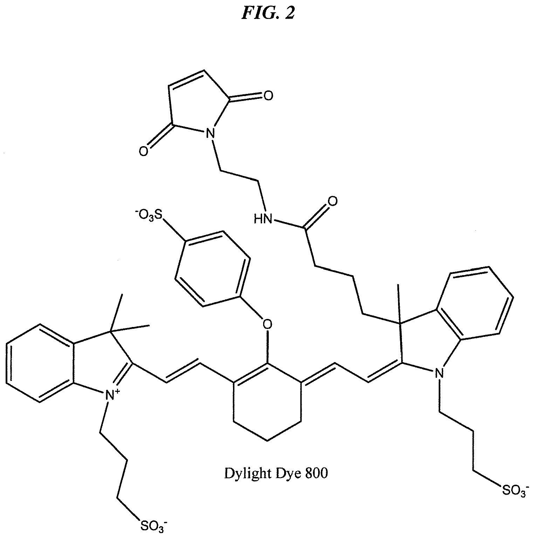

A limited number of such potential alternative AD moiety sequences are shown in SEQ ID NO:32 to SEQ ID NO:49 below. Again, a very large number of species within the genus of possible AD moiety sequences could be made, tested and used by the skilled artisan, based on the data of Alto et al. (2003). It is noted that Figure 2 of Alto (2003) shows an even large number of potential amino acid substitutions that may be made, while retaining binding activity to DDD moieties, based on actual binding experiments.

### AKAP-IS

QIEYLAKQIVDNAIQQA (SEQ ID NO:3)

**Table 2. Conservative Amino Acid Substitutions in AD1 (SEQ ID NO:3). Consensus sequence disclosed as SEQ ID NO:86.**

| **Q** | **I** | **E** | **Y** | **L** | **A** | **K** | **Q** | **I** | **V** | **D** | **N** | **A** | **I** | **Q** | **Q** | **A** |
|---|---|---|---|---|---|---|---|---|---|---|---|---|---|---|---|---|
| N | L V | D | F T S | I V | | R | N | | | E | Q | | | N | N | L I V |

NIEYLAKQIVDNAIQQA (SEQ ID NO:32)
QLEYLAKQIVDNAIQQA (SEQ ID NO:33)
QVEYLAKQIVDNAIQQA (SEQ ID NO:34)
QIDYLAKQIVDNAIQQA (SEQ ID NO:35)
QIEFLAKQIVDNAIQQA (SEQ ID NO:36)
QIETLAKQIVDNAIQQA (SEQ ID NO:37)
QIESLAKQIVDNAIQQA (SEQ ID NO:38)
QIEYIAKQIVDNAIQQA (SEQ ID NO:39)
QIEYVAKQIVDNAIQQA (SEQ ID NO:40)
QIEYLARQIVDNAIQQA (SEQ ID NO:41)
QIEYLAKNIVDNAIQQA (SEQ ID NO:42)
QIEYLAKQIVENAIQQA (SEQ ID NO:43)
QIEYLAKQIVDQAIQQA (SEQ ID NO:44)
QIEYLAKQIVDNAINQA (SEQ ID NO:45)
QIEYLAKQIVDNAIQNA (SEQ ID NO:46)
QIEYLAKQIVDNAIQQL (SEQ ID NO:47)
QIEYLAKQIVDNAIQQI (SEQ ID NO:48)
QIEYLAKQIVDNAIQQV (SEQ ID NO:49)

Gold et al. (2006, Mol Cell 24:383-95) utilized crystallography and peptide screening to develop a SuperAKAP-IS sequence (SEQ ID NO:50), exhibiting a five order of magnitude higher selectivity for the RII isoform of PKA compared with the RI isoform. Underlined residues indicate the positions of amino acid substitutions, relative to the AKAP-IS sequence, which increased binding to the DDD moiety of RIIα. In this sequence, the N-terminal Q residue is numbered as residue number 4 and the C-terminal A residue is residue number 20. Residues where substitutions could be made to affect the affinity for RIIα were residues 8, 11, 15, 16, 18, 19 and 20 (Gold et al., 2006). It is contemplated that in certain alternative embodiments, the SuperAKAP-IS sequence may be substituted for the AKAP-IS AD moiety sequence to prepare DNL™ constructs. Other alternative sequences that might be substituted for the AKAP-IS AD sequence are shown in SEQ ID NO:51-53. Substitutions relative to the AKAP-IS sequence are underlined. It is anticipated that, as with the AD2 sequence shown in SEQ ID NO:4, the AD moiety may also include the additional N-terminal residues cysteine and glycine and C-terminal residues glycine and cysteine.
*SuperAKAP-IS*
QIEYVAKQIVDYAIHQA (SEQ ID NO:50)
*Alternative AKAP sequences*
QIEYKAKQIVDHAIHQA (SEQ ID NO:51)
QIEYHAKQIVDHAIHQA (SEQ ID NO:52)
QIEYVAKQIVDHAIHQA (SEQ ID NO:53)

Figure 2 of Gold et al. disclosed additional DDD-binding sequences from a variety of AKAP proteins, shown below.

### RII-Specific AKAPs

*AKAP-KL* PLEYQAGLLVQNAIQQAI (SEQ ID NO:54)
*AKAP79* LLIETASSLVKNAIQLSI (SEQ ID NO:55)
*AKAP-Lbc* LIEEAASRIVDAVIEQVK (SEQ ID NO:56)

### RI-Specific AKAPs

*AKAPce* ALYQFADRFSELVISEAL (SEQ ID NO:57)
*RIAD* LEQVANQLADQIIKEAT (SEQ ID NO:58)
*PV38* FEELAWKIAKMIWSDVF (SEQ ID NO:59)

### Dual-Specificity AKAPs

*AKAP7* ELVRLSKRLVENAVLKAV (SEQ ID NO:60)
*MAP2D* TAEEVSARIVQVVTAEAV (SEQ ID NO:61)
*DAKAP1* QIKQAAFQLISQVILEAT (SEQ ID NO:62)
*DAKAP2* LAWKIAKMIVSDVMQQ (SEQ ID NO:63)

Stokka et al. (2006, Biochem J 400:493-99) also developed peptide competitors of AKAP binding to PKA, shown in SEQ ID NO:64-66. The peptide antagonists were designated as Ht31 (SEQ ID NO:64), RIAD (SEQ ID NO:65) and PV-38 (SEQ ID NO:66). The Ht-31 peptide exhibited a greater affinity for the RII isoform of PKA, while the RIAD and PV-38 showed higher affinity for RI.
*Ht31* DLIEEAASRIVDAVIEQVKAAGAY (SEQ ID NO:64)
*RIAD* LEQYANQLADQIIKEATE (SEQ ID NO:65)
*PV-38* FEELAWKIAKMIWSDVFQQC (SEQ ID NO:66)

Hundsrucker et al. (2006, Biochem J 396:297-306) developed still other peptide competitors for AKAP binding to PKA, with a binding constant as low as 0.4 nM to the DDD of the RII form of PKA. The sequences of various AKAP antagonistic peptides are provided in Table 1 of Hundsrucker et al., reproduced in **Table 3** below. AKAPIS represents a synthetic RII subunit-binding peptide. All other peptides are derived from the RII-binding domains of the indicated AKAPs.

**Table 3. AKAP Peptide sequences**

| | **Peptide Sequence** |
|---|---|
| AKAPIS | QIEYLAKQIVDNAIQQA (SEQ ID NO:3) |
| AKAPIS-P | QIEYLAKQIPDNAIQQA (SEQ ID NO:67) |
| Ht31 | KGADLIEEAASRIVDAVIEQVKAAG (SEQ ID NO:68) |
| Ht31-P | KGADLIEEAASRIPDAPIEQVKAAG (SEQ ID NO:69) |
| AKAP7*δ*-wt-pep | PEDAELVRLSKRLVENAVLKAVQQY (SEQ ID NO:70) |
| AKAP7*δ*-L304T-pep | PEDAELVRTSKRLVENAVLKAVQQY (SEQ ID NO:71) |
| AKAP7*δ*-L308D-pep | PEDAELVRLSKRDVENAVLKAVQQY (SEQ ID NO:72) |
| AKAP7*δ*-P-pep | PEDAELVRLSKRLPENAVLKAVQQY (SEQ ID NO:73) |
| AKAP7*δ*-PP-pep | PEDAELVRLSKRLPENAPLKAVQQY (SEQ ID NO:74) |
| AKAP7*δ*-L314E-pep | PEDAELVRLSKRLVENAVEKAVQQY (SEQ ID NO:75) |
| AKAP1-pep | EEGLDRNEEIKRAAFQIISQVISEA (SEQ ID NO:76) |
| AKAP2-pep | LVDDPLEYQAGLLVQNAIQQAIAEQ (SEQ ID NO:77) |
| AKAP5-pep | QYETLLIETASSLVKNAIQLSIEQL (SEQ ID NO:78) |
| AKAP9-pep | LEKQYQEQLEEEVAKVIVSMSIAFA (SEQ ID NO:79) |
| AKAP10-pep | NTDEAQEELAWKIAKMIVSDIMQQA (SEQ ID NO:80) |
| AKAP11-pep | VNLDKKAVLAEKIVAEAIEKAEREL (SEQ ID NO:81) |
| AKAP12-pep | NGILELETKSSKLVQNIITAVDQF (SEQ ID NO:82) |
| AKAP14-pep | TQDKNYEDELTQVALALVEDVINYA (SEQ ID NO:83) |
| Rab32-pep | ETSAKDNINIEEAARFLVEKILVNH (SEQ ID NO:84) |

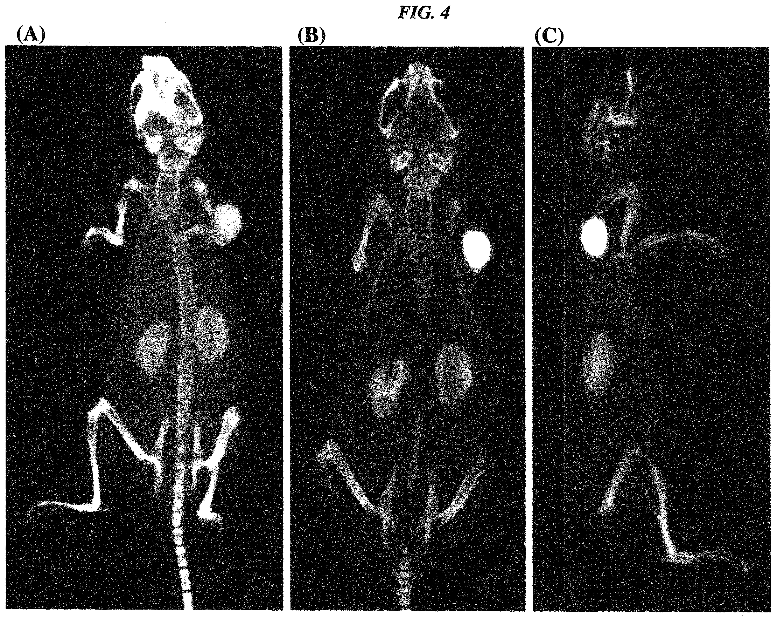

Residues that were highly conserved among the AD domains of different AKAP proteins are indicated below by underlining with reference to the AKAP IS sequence (SEQ ID NO:3). The residues are the same as observed by Alto et al. (2003), with the addition of the C-terminal alanine residue. (See FIG. 4 of Hundsrucker et al. (2006). The sequences of peptide antagonists with particularly high affinities for the RII DDD sequence were those of AKAP-IS, AKAP7δ-wt-pep, AKAP7δ-L304T-pep and AKAP7δ-L308D-pep.
*AKAP-IS*
QIEYLAKQIVDNAIQQA (SEQ ID NO:3)

Carr et al. (2001, J Biol Chem 276:17332-38) examined the degree of sequence homology between different AKAP-binding DDD sequences from human and non-human proteins and identified residues in the DDD sequences that appeared to be the most highly conserved among different DDD moieties. These are indicated below by underlining with reference to the human PKA RIIα DDD sequence of SEQ ID NO:1. Residues that were particularly conserved are further indicated by italics. The residues overlap with, but are not identical to those suggested by Kinderman et al. (2006) to be important for binding to AKAP proteins. The skilled artisan will realize that in designing sequence variants of DDD, it would be most preferred to avoid changing the most conserved residues (italicized), and it would be preferred to also avoid changing the conserved residues (underlined), while conservative amino acid substitutions may be considered for residues that are neither underlined nor italicized..

### SHIQIPPGLTELLQGYTVEVLRQQPPDLVEFAVEYFTRLREARA (SEQ ID NO:1)

A modified set of conservative amino acid substitutions for the DDD1 (SEQ ID NO:1) sequence, based on the data of Carr et al. (2001) is shown in **Table 4.** Even with this reduced set of substituted sequences, there are over 65,000 possible alternative DDD moiety sequences that may be produced, tested and used by the skilled artisan without undue experimentation. The skilled artisan could readily derive such alternative DDD amino acid sequences as disclosed above for **Table 1** and **Table 2.**

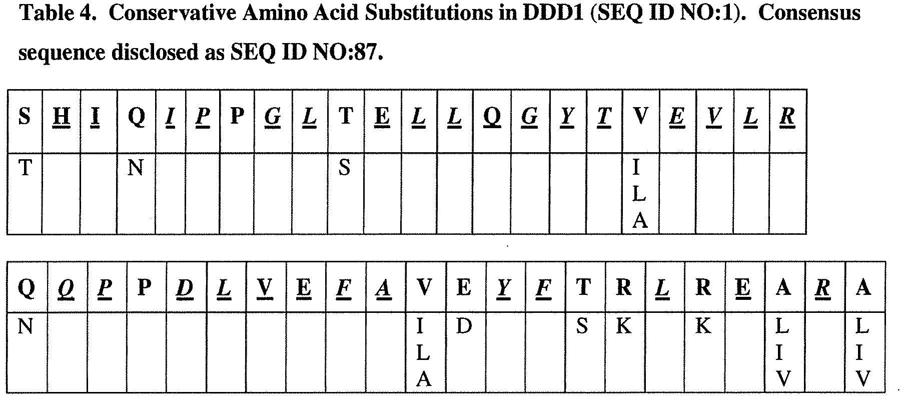

The skilled artisan will realize that these and other amino acid substitutions in the DDD or AD amino acid sequences may be utilized to produce alternative species within the genus of AD or DDD moieties, using techniques that are standard in the field and only routine experimentation.

### Pretargeting

Bispecific or multispecific antibodies may be utilized in pretargeting techniques. Pretargeting is a multistep process originally developed to resolve the slow blood clearance of directly targeting antibodies, which contributes to undesirable toxicity to normal tissues such as bone marrow. With pretargeting, a fluorescent probe, radionuclide or other diagnostic or therapeutic agent is attached to a targetable construct that is cleared within minutes from the blood. A pretargeting bispecific or multispecific antibody, which has binding sites for the targetable construct as well as a target antigen, is administered first, free antibody is allowed to clear from circulation and then the targetable construct is administered.

Pretargeting methods are disclosed, for example, in Goodwin et al., U.S. Pat. No. 4,863,713; Goodwin et al., J. Nucl. Med. 29:226, 1988; Hnatowich et al., J. Nucl. Med. 28:1294, 1987; Oehr et al., J. Nucl. Med. 29:728, 1988; Klibanov et al., J. Nucl. Med. 29:1951, 1988; Sinitsyn et al., J. Nucl. Med. 30:66, 1989; Kalofonos et al., J. Nucl. Med. 31:1791, 1990; Schechter et al., Int. J. Cancer 48:167, 1991; Paganelli et al., Cancer Res. 51:5960,1991; Paganelli et al., Nucl. Med. Commun. 12:211, 1991; U.S. Pat. No. 5,256,395; Stickney et al., Cancer Res. 51:6650, 1991; Yuan et al., Cancer Res. 51:3119, 1991; U.S. Pat. Nos. 6,077,499; 7,011,812; 7,300,644; 7,074,405; 6,962,702; 7,387,772; 7,052,872; 7,138,103; 6,090,381; 6,472,511; 6,962,702; and 6,962,702.

A pretargeting method of imaging, detecting and/or diagnosing a disease or disorder in a subject may be provided by: (1) administering to the subject a bispecific antibody or antibody fragment; (2) optionally administering to the subject a clearing composition, and allowing the composition to clear the antibody from circulation; and (3) administering to the subject the targetable construct, containing one or more conjugated fluorescent probes.

### Immunoconjugates

Any of the antibodies, antibody fragments or antibody fusion proteins described herein may be conjugated to a fluorescent probe or other diagnostic or therapeutic agent to form an immunoconjugate. Methods for covalent conjugation of fluorescent probes and other functional groups are known in the art and any such known method may be utilized.

For example, a fluorescent probes can be attached at the hinge region of a reduced antibody component via disulfide bond formation or sulfhydryl-maleimide interaction. Alternatively, such agents can be attached using a heterobifunctional cross-linker, such as *N-*succinyl 3-(2-pyridyldithio)propionate (SPDP). Yu et al., Int. J. Cancer 56: 244 (1994). General techniques for such conjugation are well-known in the art. See, for example, Wong, CHEMISTRY OF PROTEIN CONJUGATION AND CROSS-LINKING (CRC Press 1991); Upeslacis et al., "Modification of Antibodies by Chemical Methods," in MONOCLONAL ANTIBODIES: PRINCIPLES AND APPLICATIONS, Birch et al. (eds.), pages 187-230 (Wiley-Liss, Inc. 1995); Price, "Production and Characterization of Synthetic Peptide-Derived Antibodies," in MONOCLONAL ANTIBODIES: PRODUCTION, ENGINEERING AND CLINICAL APPLICATION, Ritter et al. (eds.), pages 60-84 (Cambridge University Press 1995).

Alternatively, the fluorescent probes can be conjugated via a carbohydrate moiety in the Fc region of the antibody. See, for example, Shih et al., Int. J. Cancer 41: 832 (1988); Shih et al., Int. J. Cancer 46: 1101 (1990); and Shih et al., U.S. Patent No. 5,057,313. The general method involves reacting an antibody component having an oxidized carbohydrate portion with a fluorescent probes that has at least one free amine function. This reaction results in an initial Schiff base (imine) linkage, which can be stabilized by reduction to a secondary amine to form the final conjugate.

The Fc region may be absent if the antibody used as the antibody component of the immunoconjugate is an antibody fragment. However, it is possible to introduce a carbohydrate moiety into the light chain variable region of a full length antibody or antibody fragment. See, for example, Leung et al., J. Immunol. 154: 5919 (1995); U.S. Patent Nos. 5,443,953 and 6,254,868. The engineered carbohydrate moiety is used to attach the functional group to the antibody fragment.

### Click Chemistry

An alternative method for attaching fluorescent probes or other functional groups to a targeting molecule involves use of click chemistry reactions. The click chemistry approach was originally conceived as a method to rapidly generate complex substances by joining small subunits together in a modular fashion. (See, e.g., Kolb et al., 2004, Angew Chem Int Ed 40:3004-31; Evans, 2007, Aust J Chem 60:384-95.) Various forms of click chemistry reaction are known in the art, such as the Huisgen 1,3-dipolar cycloaddition copper catalyzed reaction (Tornoe et al., 2002, J Organic Chem 67:3057-64), which is often referred to as the "click reaction." Other alternatives include cycloaddition reactions such as the Diels-Alder, nucleophilic substitution reactions (especially to small strained rings like epoxy and aziridine compounds), carbonyl chemistry formation of urea compounds and reactions involving carbon-carbon double bonds, such as alkynes in thiol-yne reactions.

The azide alkyne Huisgen cycloaddition reaction uses a copper catalyst in the presence of a reducing agent to catalyze the reaction of a terminal alkyne group attached to a first molecule. In the presence of a second molecule comprising an azide moiety, the azide reacts with the activated alkyne to form a 1,4-disubstituted 1,2,3-triazole. The copper catalyzed reaction occurs at room temperature and is sufficiently specific that purification of the reaction product is often not required. (Rostovstev et al., 2002, Angew Chem Int Ed 41:2596; Tornoe et al., 2002, J Org Chem 67:3057.) The azide and alkyne functional groups are largely inert towards biomolecules in aqueous medium, allowing the reaction to occur in complex solutions. The triazole formed is chemically stable and is not subject to enzymatic cleavage, making the click chemistry product highly stable in biological systems. Although the copper catalyst is toxic to living cells, the copper-based click chemistry reaction may be used *in vitro* for immunoconjugate formation.

A copper-free click reaction has been proposed for covalent modification of biomolecules. (See, e.g., Agard et al., 2004, J Am Chem Soc 126:15046-47.) The copper-free reaction uses ring strain in place of the copper catalyst to promote a [3 + 2] azide-alkyne cycloaddition reaction *(Id.)* For example, cyclooctyne is a 8-carbon ring structure comprising an internal alkyne bond. The closed ring structure induces a substantial bond angle deformation of the acetylene, which is highly reactive with azide groups to form a triazole. Thus, cyclooctyne derivatives may be used for copper-free click reactions (*Id.*)

Another type of copper-free click reaction was reported by Ning et al. (2010, Angew Chem Int Ed 49:3065-68), involving strain-promoted alkyne-nitrone cycloaddition. To address the slow rate of the original cyclooctyne reaction, electron-withdrawing groups are attached adjacent to the triple bond (*Id.*) Examples of such substituted cyclooctynes include difluorinated cyclooctynes, 4-dibenzocyclooctynol and azacyclooctyne (*Id.*) An alternative copper-free reaction involved strain-promoted alkyne-nitrone cycloaddition to give N-alkylated isoxazolines (*Id.*) The reaction was reported to have exceptionally fast reaction kinetics and was used in a one-pot three-step protocol for site-specific modification of peptides and proteins (*Id.*) Nitrones were prepared by the condensation of appropriate aldehydes with N-methylhydroxylamine and the cycloaddition reaction took place in a mixture of acetonitrile and water (*Id*.) These and other known click chemistry reactions may be used to attach chelating moieties to antibodies or other targeting molecules *in vitro.*

### Methods of Administration

In various embodiments, bispecific antibodies and targetable constructs may be used for imaging normal or diseased tissue and organs (see, e.g. U.S. Pat Nos. 6,126,916; 6,077,499; 6,010,680; 5,776,095; 5,776,094; 5,776,093; 5,772,981; 5,753,206; 5,746,996; 5,697,902; 5,328,679; 5,128,119; 5,101,827; and 4,735,210).

The administration of a bispecific antibody (bsAb) and a fluorescent-labeled targetable construct may be conducted by administering the bsAb antibody at some time prior to administration of the targetable construct The doses and timing of the reagents can be readily devised by a skilled artisan, and are dependent on the specific nature of the reagents employed. If a bsAb-F(ab')₂ derivative is given first, then a waiting time of 24-72 hr (alternatively 48-96 hours) before administration of the targetable construct would be appropriate. If an IgG-Fab' bsAb conjugate is the primary targeting vector, then a longer waiting period before administration of the targetable construct would be indicated, in the range of 3-10 days. After sufficient time has passed for the bsAb to target to the diseased tissue, the fluorescent-labeled targetable construct is administered. Subsequent to administration of the targetable construct, imaging can be performed.

Certain embodiments concern the use of multivalent target binding proteins which have at least three different target binding sites as described in patent application Ser. No. 60/220,782. Multivalent target binding proteins have been made by cross-linking several Fab-like fragments via chemical linkers. See U.S. Pat. Nos. 5,262,524; 5,091,542 and Landsdorp et al. Euro. J. Immunol. 16: 679-83 (1986). Multivalent target binding proteins also have been made by covalently linking several single chain Fv molecules (scFv) to form a single polypeptide. See U.S. Pat No. 5,892,020. A multivalent target binding protein which is basically an aggregate of scFv molecules has been disclosed in U.S. Pat. Nos. 6,025,165 and 5,837,242. A trivalent target binding protein comprising three scFv molecules has been described in Krott et al. Protein Engineering 10(4): 423-433 (1997).

Alternatively, the technique for making DNL™ complexes, described in more detail above, has been demonstrated for the simple and reproducible construction of a variety of multivalent complexes, including complexes comprising two or more different antibodies or antibody fragments. (See, e.g., U.S. Patent Nos. 7,550,143; 7,521,056; 7,534,866; 7,527,787 and 7,666,400). Such constructs are also of use for the practice of the claimed methods and compositions described herein.

A clearing agent may be used which is given between doses of the bispecific antibody (bsAb) and the targetable construct. A clearing agent of novel mechanistic action may be used, namely a glycosylated anti-idiotypic Fab' fragment targeted against the disease targeting arm(s) of the bsAb. In one example, anti-CEA (MN-14 Ab) x anti-peptide bsAb is given and allowed to accrete in disease targets to its maximum extent. To clear residual bsAb from circulation, an anti-idiotypic Ab to MN-14, termed WI2, is given, preferably as a glycosylated Fab' fragment. The clearing agent binds to the bsAb in a monovalent manner, while its appended glycosyl residues direct the entire complex to the liver, where rapid metabolism takes place. Then the fluorescent-labeled targetable construct is given to the subject. The WI2 Ab to the MN-14 arm of the bsAb has a high affinity and the clearance mechanism differs from other disclosed mechanisms (see Goodwin et al., ibid), as it does not involve cross-linking, because the WI2-Fab' is a monovalent moiety. However, alternative methods and compositions for clearing agents are known and any such known clearing agents may be used.

### Formulation and Administration

The fluorescent-labeled molecules may be formulated to obtain compositions that include one or more pharmaceutically suitable excipients, one or more additional ingredients, or some combination of these. These can be accomplished by known methods to prepare pharmaceutically useful dosages, whereby the active ingredients (i.e., the fluorescent-labeled molecules) are combined in a mixture with one or more pharmaceutically suitable excipients. Sterile phosphate-buffered saline is one example of a pharmaceutically suitable excipient. Other suitable excipients are well known to those in the art. See, e.g., Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

The preferred route for administration of the compositions described herein is parenteral injection. Injection may be intravenous, intraarterial, intralymphatic, intrathecal, or intracavitary (i.e., parenterally). In parenteral administration, the compositions will be formulated in a unit dosage injectable form such as a solution, suspension or emulsion, in association with a pharmaceutically acceptable excipient. Such excipients are inherently nontoxic and nontherapeutic. Examples of such excipients are saline, Ringer's solution, dextrose solution and Hank's solution. Nonaqueous excipients such as fixed oils and ethyl oleate may also be used. A preferred excipient is 5% dextrose in saline. The excipient may contain minor amounts of additives such as substances that enhance isotonicity and chemical stability, including buffers and preservatives. Other methods of administration, including oral administration, are also contemplated.

Formulated compositions comprising fluorescent-labeled molecules can be used for intravenous administration via, for example, bolus injection or continuous infusion. Compositions for injection can be presented in unit dosage form, e.g., in ampoules or in multi-dose containers, with an added preservative. Compositions can also take such forms as suspensions, solutions or emulsions in oily or aqueous vehicles, and can contain formulatory agents such as suspending, stabilizing and/or dispersing agents. Alternatively, the compositions can be in powder form for constitution with a suitable vehicle, e.g., sterile pyrogen-free water, before use.

The compositions may be administered in solution. The pH of the solution should be in the range of pH 5 to 9.5, preferably pH 6.5 to 7.5. The formulation thereof should be in a solution having a suitable pharmaceutically acceptable buffer such as phosphate, TRIS (hydroxymethyl) aminomethane-HCl or citrate and the like. Buffer concentrations should be in the range of 1 to 100 mM. The formulated solution may also contain a salt, such as sodium chloride or potassium chloride in a concentration of 50 to 150 mM. An effective amount of a stabilizing agent such as glycerol, albumin, a globulin, a detergent, a gelatin, a protamine or a salt of protamine may also be included. The compositions may be administered to a mammal subcutaneously, intravenously, intramuscularly or by other parenteral routes. Moreover, the administration may be by continuous infusion or by single or multiple boluses.

Where bispecific antibodies are administered, for example in a pretargeting technique, the dosage of an administered antibody for humans will vary depending upon such factors as the patient's age, weight, height, sex, general medical condition and previous medical history. Typically, for imaging purposes it is desirable to provide the recipient with a dosage of bispecific antibody that is in the range of from about 1 mg to 200 mg as a single intravenous infusion, although a lower or higher dosage also may be administered as circumstances dictate. Typically, it is desirable to provide the recipient with a dosage that is in the range of from about 10 mg per square meter of body surface area or 17 to 18 mg of the antibody for the typical adult, although a lower or higher dosage also may be administered as circumstances dictate. Examples of dosages of bispecific antibodies that may be administered to a human subject for imaging purposes are 1 to 200 mg, more preferably 1 to 70 mg, most preferably 1 to 20 mg, although higher or lower doses may be used.

In general, the dosage of fluorescent label to administer will vary depending upon such factors as the patient's age, weight, height, sex, general medical condition and previous medical history. Preferably, a saturating dose of the fluorescent-labeled molecules is administered to a patient.

### Administration of Peptides

Various embodiments of the claimed methods and/or compositions may concern one or more fluorescent-labeled peptides to be administered to a subject. Administration may occur by any route known in the art, including but not limited to oral, nasal, buccal, inhalational, rectal, vaginal, topical, orthotopic, intradermal, subcutaneous, intramuscular, intraperitoneal, intraarterial, intrathecal or intravenous injection. Where, for example, fluorescent-labeled peptides are administered in a pretargeting protocol, the peptides would preferably be administered i.v.

Unmodified peptides administered orally to a subject can be degraded in the digestive tract and depending on sequence and structure may exhibit poor absorption across the intestinal lining. However, methods for chemically modifying peptides to render them less susceptible to degradation by endogenous proteases or more absorbable through the alimentary tract are well known (see, for example, Blondelle et al., 1995, Biophys. J. 69:604-11; Ecker and Crooke, 1995, Biotechnology 13:351-69; Goodman and Ro, 1995, BURGER'S MEDICINAL CHEMISTRY AND DRUG DISCOVERY, VOL. I, ed. Wollf, John Wiley & Sons; Goodman and Shao, 1996, Pure & Appl. Chem. 68:1303-08). Methods for preparing libraries of peptide analogs, such as peptides containing D-amino acids; peptidomimetics consisting of organic molecules that mimic the structure of a peptide; or peptoids such as vinylogous peptoids, have also been described and may be used to construct peptide based fluorescent-labeled molecules suitable for oral administration to a subject.

In certain embodiments, the standard peptide bond linkage may be replaced by one or more alternative linking groups, such as CH₂-NH, CH₂-S, CH₂-CH₂, CH=CH, CO-CH₂, CHOH-CH₂ and the like. Methods for preparing peptide mimetics are well known (for example, Hruby, 1982, Life Sci 31:189-99; Holladay et al., 1983, Tetrahedron Lett. 24:4401-04; Jennings-White et al., 1982, Tetrahedron Lett. 23:2533; Almquiest et al., 1980, J. Med. Chem. 23:1392-98; Hudson et al., 1979, Int. J. Pept. Res. 14:177-185; Spatola et al., 1986, Life Sci 38:1243-49; U.S. Patent Nos. 5,169,862; 5,539,085; 5,576,423, 5,051,448, 5,559,103.) Peptide mimetics may exhibit enhanced stability and/or absorption *in vivo* compared to their peptide analogs.

Alternatively, peptides may be administered by oral delivery using N-terminal and/or C-terminal capping to prevent exopeptidase activity. For example, the C-terminus may be capped using amide peptides and the N-terminus may be capped by acetylation of the peptide. Peptides may also be cyclized to block exopeptidases, for example by formation of cyclic amides, disulfides, ethers, sulfides and the like.

Peptide stabilization may also occur by substitution of D-amino acids for naturally occurring L-amino acids, particularly at locations where endopeptidases are known to act. Endopeptidase binding and cleavage sequences are known in the art and methods for making and using peptides incorporating D-amino acids have been described (*e.g*., U.S. Patent Application Publication No. 20050025709, McBride et al., filed June 14, 2004). In certain embodiments, peptides and/or proteins may be orally administered by co-formulation with proteinase- and/or peptidase-inhibitors.

Other methods for oral delivery of peptides are disclosed in Mehta ("Oral delivery and recombinant production of peptide hormones," June 2004, BioPharm International). The peptides are administered in an enteric-coated solid dosage form with excipients that modulate intestinal proteolytic activity and enhance peptide transport across the intestinal wall. Relative bioavailability of intact peptides using this technique ranged from 1% to 10% of the administered dosage. Insulin has been successfully administered in dogs using enteric-coated microcapsules with sodium cholate and a protease inhibitor (Ziv et al., 1994, J. Bone Miner. Res. 18 (Suppl. 2):792-94. Oral administration of peptides has been performed using acylcarnitine as a permeation enhancer and an enteric coating (Eudragit L30D-55, Rohm Pharma Polymers, see Mehta, 2004). Excipients of use for orally administered peptides may generally include one or more inhibitors of intestinal proteases/peptidases along with detergents or other agents to improve solubility or absorption of the peptide, which may be packaged within an enteric-coated capsule or tablet (Mehta, 2004). Organic acids may be included in the capsule to acidify the intestine and inhibit intestinal protease activity once the capsule dissolves in the intestine (Mehta, 2004). Another alternative for oral delivery of peptides would include conjugation to polyethylene glycol (PEG)-based amphiphilic oligomers, increasing absorption and resistance to enzymatic degradation (Soltero and Ekwuribe, 2001, Pharm. Technol. 6:110).

### Imaging Using Labeled Molecules

Methods of imaging using labeled molecules are known in the art, and any such known methods may be used with the fluorescent-labeled molecules disclosed herein. See, e.g., U.S Patent Nos. 5,928,627; 6,096,289; 6,387,350; 7,201,890; 7,597,878; 7,947,256. In preferred embodiments, methods of fluorescent imaging, detection and/or diagnosis may be performed *in vivo,* for example by intraoperative, intraperitoneal, laparoscopic, endoscopic or intravascular techniques. Alternatively, *in vitro* fluorescent imaging, detection and/or diagnose may be performed using any method known in the art.

Endoscopic devices and techniques have been used for *in vivo* imaging of tissues and organs, including peritoneum (Gahlen et al., 1999, J Photochem Photobiol B 52:131-135), ovarian cancer (Major et al., 1997, Gynecol Oncol 66:122-132), colon (Mycek et al., 1998, Gastrointest Endosc 48:390-394; Stepp et al., 1998, Endoscopy 30:379-386), bile ducts (Izuishi et al., 1999, Hepatogastroenterology 46:804-807), stomach (Abe et al., 2000, Endoscopy 32:281-286), bladder (Kriegmair et al., 1999, Urol Int 63:27-31), and brain (Ward, 1998, J Laser Appl 10:224-228). Catheter based devices, such as fiber optics devices, are particularly suitable for intravascular imaging. (See, e.g., Tearney et al., 1997, Science 276:2037-2039.) Other imaging technologies include phased array detection (Boas et al., 1994, Proc Natl Acad Sci USA 91:4887-4891; Chance, 1998, Ann NY Acad Sci 38:29-45), diffuse optical tomography (Cheng et al., 1998, Optics Express 3:118-123; Siegel et al., 1999, Optics Express 4:287-298), intravital microscopy (Dellian et al., 2000, Br J Cancer 82:1513-1518; Monsky et al., 1999, Cancer Res 59:4129-4135; Fukumura et al., 1998, Cell 94:715-725), and confocal imaging (Korlach et al., 1999, Proc Natl Acad Sci. USA 96:8461-8466; Rajadhyaksha et al., 1995, J Invest Dermatol 104:946-952).

In certain embodiments, fluorescent-labeled molecules may be of use in imaging normal or diseased tissue and organs, for example using the methods described in U.S. Pat. Nos. 5,928,627; 6,096,289; 6,387,350; 7,201,890; 7,597,878; 7,947,256. Such imaging can be conducted by direct fluorescent labeling of the appropriate targeting molecules, or by a pretargeted imaging method, as described in Goldenberg et al. (2007, Update Cancer Ther. 2:19-31); Sharkey et al. (2008, Radiology 246:497-507); Goldenberg et al. (2008, J. Nucl. Med. 49:158-63); Sharkey et al. (2007, Clin. Cancer Res. 13:5777s-5585s); McBride et al. (2006, J. Nucl. Med. 47:1678-88); Goldenberg et al. (2006, J. Clin. Oncol.24:823-85), see also U.S. Patent Publication Nos. 20050002945, 20040018557,20030148409 and 20050014207.

In preferred embodiments, the invention as defined in the claims is of use for imaging of cancer. Examples of cancers include, but are not limited to, carcinoma, lymphoma, blastoma, sarcoma, and leukemia or lymphoid malignancies. More particular examples of such cancers are noted below and include: squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial cancer or uterine carcinoma, salivary gland carcinoma, kidney or renal cancer, prostate cancer, vulval cancer, thyroid cancer, hepatic carcinoma, anal carcinoma, penile carcinoma, as well as head and neck cancer. The term "cancer" includes primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor).

Other examples of cancers or malignancies include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Disease, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Disease, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma During Pregnancy, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Purpura, Parathyroid Cancer, Penile Cancer, Pheochromocytoma, Pituitary Tumor, Plasma Cell Neoplasm/Multiple Myeloma, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilms' Tumor, and any other hyperproliferative disease, besides neoplasia, located in an organ system listed above.

The invention as defined in the claims may be used to detect or diagnose malignant or premalignant conditions. Such uses are indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins and Angell, Basic Pathology, 2d Ed., W. B. Saunders Co., Philadelphia, pp. 68-79 (1976)).

Dysplasia is frequently a forerunner of cancer, and is found mainly in the epithelia. It is the most disorderly form of non-neoplastic cell growth, involving a loss in individual cell uniformity and in the architectural orientation of cells. Dysplasia characteristically occurs where there exists chronic irritation or inflammation. Dysplastic disorders which can be detected include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, opthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which can be detected include, but are not limited to, benign dysproliferative disorders (e.g., benign tumors, fibrocystic conditions, tissue hypertrophy, intestinal polyps, colon polyps, and esophageal dysplasia), leukoplakia, keratoses, Bowen's disease, Farmer's Skin, solar cheilitis, and solar keratosis.

Additional hyperproliferative diseases, disorders, and/or conditions include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendroglioma, menangioma, melanoma, neuroblastoma, and retinoblastoma.

In a preferred embodiment, diseases that may be diagnosed include cardiovascular diseases, such as fibrin clots, atherosclerosis, myocardial ischemia and infarction. Antibodies to fibrin (e.g., scFv(59D8); T2G1s; MH1) are known and in clinical trials as imaging agents for disclosing said clots and pulmonary emboli, while anti-granulocyte antibodies, such as MN-3, MN-15, anti-NCA95, and anti-CD15 antibodies, can target myocardial infarcts and myocardial ischemia. (See, e.g., U.S. Patent Nos. 5,487,892; 5,632,968; 6,294,173; 7,541,440) Anti-macrophage, anti-low-density lipoprotein (LDL) and anti-CD74 (e.g., hLL1) antibodies can be used to target atherosclerotic plaques. Abciximab (anti-glycoprotein IIb/IIIa) has been approved for adjuvant use for prevention of restenosis in percutaneous coronary interventions and unstable angina (Waldmann et al., 2000, Hematol 1:394-408). Anti-CD3 antibodies have been reported to reduce development and progression of atherosclerosis (Steffens et al., 2006, Circulation 114:1977-84). Blocking MIF antibody has been reported to induce regression of established atherosclerotic lesions (Sanchez-Madrid and Sessa, 2010, Cardiovasc Res 86:171-73). Antibodies against oxidized LDL also induced a regression of established atherosclerosis in a mouse model (Ginsberg, 2007, J Am Coll Cardiol 52:2319-21). Anti-ICAM-1 antibody was shown to reduce ischemic cell damage after cerebral artery occlusion in rats (Zhang et al., 1994, Neurology 44:1747-51). Commercially available monoclonal antibodies to leukocyte antigens are represented by: OKT anti-T-cell monoclonal antibodies (available from Ortho Pharmaceutical Company) which bind to normal T-lymphocytes; the monoclonal antibodies produced by the hybridomas having the ATCC accession numbers HB44, HB55, HB12, HB78 and HB2; G7E11, W8E7, NKP15 and GO22 (Becton Dickinson); NEN9.4 (New England Nuclear); and FMC11 (Sera Labs). A description of antibodies against fibrin and platelet antigens is contained in Knight, Semin. Nucl. Med., 20:52-67 (1990).

A pharmaceutical composition of the present disclosure may be used to image, detect and/or diagnosis a metabolic disease, such amyloidosis, or a neurodegenerative disease, such as Alzheimer's disease, amyotrophic lateral sclerosis (ALS), Parkinson's disease, Huntington's disease, olivopontocerebellar atrophy, multiple system atrophy, progressive supranuclear palsy, diffuse lewy body disease, corticodentatonigral degeneration, progressive familial myoclonic epilepsy, strionigral degeneration, torsion dystonia, familial tremor, Gilles de la Tourette syndrome or Hallervorden-Spatz disease. Bapineuzumab is in clinical trials for Alzheimer's disease. Other antibodies proposed for Alzheimer's disease include Alz 50 (Ksiezak-Reding et al., 1987, J Biol Chem 263:7943-47), gantenerumab, and solanezumab. Infliximab, an anti-TNF-α antibody, has been reported to reduce amyloid plaques and improve cognition. Antibodies against mutant SOD1, produced by hybridoma cell lines deposited with the International Depositary Authority of Canada (accession Nos. ADI-290806-01, ADI-290806-02, ADI-290806-03) have been proposed for ALS, Parkinson's disease and Alzheimer's disease (see U.S. Patent Appl. Publ. No. 20090068194). Anti-CD3 antibodies have been proposed for type 1 diabetes (Cemea et al., 2010, Diabetes Metab Rev 26:602-05). In addition, the present invention may be used for detection or diagnosis of an immune-dysregulatory disorder, such as graft-versus-host disease or organ transplant rejection.

The exemplary conditions listed above that may be detected, diagnosed and/or imaged are not limiting. The skilled artisan will be aware that antibodies, antibody fragments or targeting peptides are known for a wide variety of conditions, such as autoimmune disease, cardiovascular disease, neurodegenerative disease, metabolic disease, cancer, infectious disease and hyperproliferative disease. Any such condition for which an fluorescent-labeled molecule, such as a protein or peptide, may be prepared and utilized by the methods described herein, may be imaged, diagnosed and/or detected as described herein.

### Kits

The invention as defined in the claims also encompasses the use of a kit.

If the composition containing components for administration is not formulated for delivery via the alimentary canal, such as by oral delivery, a device capable of delivering the kit components through some other route may be included. One type of device, for applications such as parenteral delivery, is a syringe that is used to inject the composition into the body of a subject. Inhalation devices may also be used for certain applications.

The kit components may be packaged together or separated into two or more containers. In some embodiments, the containers may be vials that contain sterile, lyophilized formulations of a composition that are suitable for reconstitution. A kit may also contain one or more buffers suitable for reconstitution and/or dilution of other reagents. Other containers that may be used include, but are not limited to, a pouch, tray, box, tube, or the like. Kit components may be packaged and maintained sterilely within the containers. Another component that can be included is instructions to a person using a kit for its use.

### EXAMPLES

### Example 1. Preparation of RDC017 and RDC018 for In Vitro and In Vivo Fluorescent Labeling

Exemplary fluorescent dyes were conjugated to a bis-HSG targetable construct for *in vitro* and *in vivo* studies. RDC017 and RDC018 were prepared by conjugating IMP-499 (FIG. 1, SEQ ID NO:88) to DYLIGHT® dye 488 and DYLIGHT® dye 800, respectively. The two dye-bis-HSG conjugates were purified using reverse-phase HPLC (RP-HPLC) and characterized by LC-MS. Evaluation of the ability of the dye-hapten to bind to the h679 anti-HSG antibody was accomplished by incubation with TF10 and TF12 bispecific antibodies, designed for use in pretargeting, and looking at the formation of the hapten-antibody complex by size-exclusion HPLC (SE-HPLC). The dye conjugation to IMP-499 did not compromise the hapten binding to h679, as expected for the conjugation taking place at the terminal end of the peptide scaffold for the bis-HSG. *In vitro* studies on the binding and internalization of TF12 and TF2 were performed in tumor cell lines.
*IMP-499*
DOTA-D-Tyr-D-Lys(HSG)-D-Glu-D-Lys(HSG)-CH₂CH₂-SH (SEQ ID NO:88)

Targetable Construct IMP-499 (**FIG. 1**) was prepared by standard automated peptide synthesis, as discussed in the Examples below. The structural features of IMP-499 include all D-amino acid residues; two HSG-haptens, each linked to the ε-amino group of the D-lysine residue; a DOTA amide at the *N*-terminus for radiolabeling; and a free thiol at the *C*-terminus for dye conjugation. RP-HPLC analysis of IMP-499 showed a singled well-defined peak on a C4 column (not shown). Analysis by mass spectrometry showed observed [M+H] 1512.7266 (calculated 1512.7270) and [M+Na] 1534.7083 (calculated 1534.7091) forms of the peptide.

Fluorescent Dyes The maleimide form of the DYLIGHT® dye 488 was obtained from Thermo Scientific (Rockford, IL, Catalog # PI-46602). The DYLIGHT® dye 488 exhibits high photostability under very acidic conditions. Hence cell internalization of the dye or its conjugate will not destroy its fluorescence properties, unlike fluorescein. Spectrally, DYLIGHT® 488 is similar to Cy2 (GE Amersham), Alexa Fluor 488 (Invitrogen) and fluorescein and so is compatible with *in vitro* studies using flow cytometry and fluorescence microscopy without any change in equipment required. The structure of DYLIGHT® dye 488 was not disclosed by the manufacturer. The estimated molecular weight of the maleimide form of DYLIGHT® dye 488 was -800 g/mole.

The maleimide form of the DYLIGHT® dye 800 was obtained from Thermo Scientific (Catalog # PI-46621). DYLIGHT® dye 800 is hydrophilic, water soluble, similar in fluorescence property to indocyanine green (ICG) and IR800, and would have the depth penetration properties of near-IR dyes. The dye is ionic with multiple sulfonate groups and multiply charged and is compatible with aqueous buffers. The structure of the dye is shown in **FIG. 2****.** The molecular weight of the disodium salt form of the dye was -1075 g/mole.

Bispecific Antibodies DNL™ complexes of bispecific antibodies designed for pretargeting applications were prepared as discussed in the Examples below. The TF10 DNL™ complex (Fab-h679-(Fab-hPAM4)₂, 3 mg/mL in PBS) comprises the hPAM4 anti-pancreatic cancer mucin antibody, attached to an h679 anti-HSG antibody. The TF12 (Fab-h679-(Fab-hRS7)₂, 1.88 mg/mL in PBS) DNL™ complex comprises the anti-EGP-1 (anti-TROP2) antibody attached to an h679 anti-HSG antibody.

Characterization The purity of IMP-499, RDC017 and RDC018 were assessed by RP-HPLC using a Waters ALLIANCE® system equipped with a Model 486 tunable UV detector and a Jupiter C4 column (5 µm, 4.6x250mm) eluted with a linear gradient of 100% 0.3% NH₄OAc (Buffer A) to 70% acetonitrile in 35 minutes at a flow rate of 1.0 mL/min. A Waters ALLIANCE® Workstation system equipped with a Waters 2475 Multi λ Fluorescence detector, and a Jupiter C4 column (5 µm, 4.6x250mm) was also used for analyzing RDC017 and RDC018.

Mass spectral data was obtained on an Agilent time-of-flight LC/MS system equipped with a 1200 series HPLC system and a Model 6210 with an electrospray ionization (ESI/TOF/MS). The sample was ran through a KINETEX® XB-C18 column (2.6 µm, 2.1x50 mm) eluted with 100% A (5% MeCN in water, 0.01% formic acid) to 100 % B (10% water in MeCN, 0.01% formic acid) in 8 minutes at a flow rate of 0.5 mL/min.

Size exclusion-HPLC was obtained on a Varian ProStar Workstation using a BIOSEP™ SEC s3000 column and on a Waters ALLIANCE® Workstation using a BioRad BIOSIL® SEC column eluted with 1x PBS at 1.0 mL/min.

Preparation of RDC017 IMP-499 (3 mg) dissolved in 100 µL 0.10 M sodium phosphate buffer at pH ∼7 was added to a solution of DYLIGHT® dye 488 (1 mg, 1.25 µmole) in 10 µL of DMF. After incubation overnight at room temperature under argon in the dark, the reaction mixture was injected in ∼10 µL aliquots onto a Jupiter C4 reverse-phase column (5 µm, 4.6x250 mm) and eluted with a linear gradient (100% 0.3% NH₄OAc to 70% acetonitrile in 35 minutes) at a flow rate of 1.0 mL/min. The chromatographic process was monitored at λ 493 nm and the product-containing fractions as identified by LC-MS were collected and lyophilized to obtain an orange solid.

Preparation of RDC018 IMP-499 (3 mg) dissolved in 0.10 M sodium phosphate buffer (100 µL) at pH ∼7 was added to a solution of DYLIGHT® dye 800 (1 mg, 1.25 µmole) in 10 µL of DMF. After incubation overnight at room temperature under argon in the dark, the reaction mixture was injected in -20 µL aliquots onto a Waters NOVAPAK® C18 reverse-phase column (10 µm, 7.8x250 mm) and eluted with a linear gradient (100% 0.3% NH₄OAc to 70% acetonitrile in 35 minutes) at a flow rate of 3.0 mL/min. The chromatographic process was monitored at λ 254 nm and the product-containing fractions as identified by LC-MS were collected and lyophilized to obtain a blue solid.

Binding Analysis RDC017 or RDC018 (∼ 1-5 nM) were incubated with bispecific antibody for 30 minutes at room temperature followed by analysis on SE-HPLC.

### Results

RP-HPLC and LC-MS The initial purification of RDC017 by RP-HPLC yielded two major peaks (not shown), which were collected and subsequently shown to be the desired product as described below. When each fraction and a mixture of both were reanalyzed by RP-HPLC using the same conditions as the purification procedure, only a single sharp peak of nearly the same retention time was detected (not shown) for each of them (17.456 min, fraction 1; 17.540 min, fraction 2; and 17.437 min, mixture). Reverse-phase HPLC with fluorescence detection also gave a single peak for the mixture of the two fractions (not shown). The LC-MS profiles of the two product-containing fractions were also the same. From the MS analysis, we estimate the MW of the isolated product RDC017 ∼ 2294 g/mol.

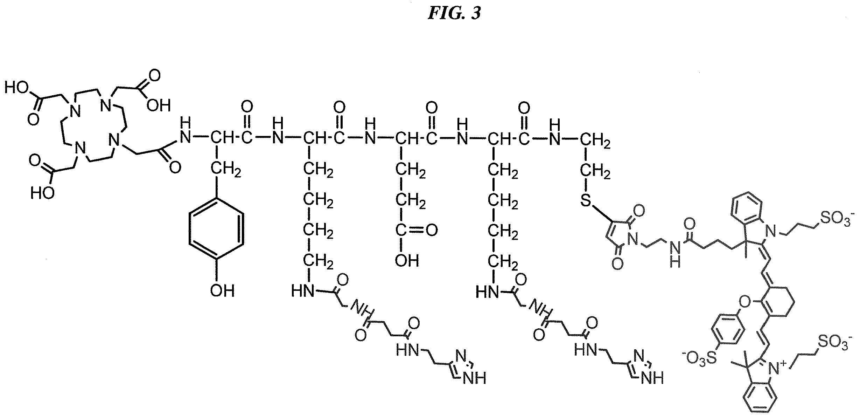

For RDC018, a single major peak was detected in the reaction mixture upon RP-HPLC (not shown). Subsequently, RDC018 was purified from the reaction mixture on a semi-prep C18 column and its purity further confirmed by analysis on an analytical C4 column (not shown) and with fluorescence detection (not shown). LC-MS confirms the product with a molecular weight of ∼2542 g/mol. A schematic structure of RDC018 is shown in **FIG. 3****.**

Binding Analysis The formation of a hapten-antibody complex was evidenced by the observation of a new peak at 7.2 min on SE-HPLC, which was detectable with either λ 280 and 493 nm, when RDC017 was incubated with TF10 (not shown). Similar results were obtained for RDC017 and TF12, with the new peak at 8.1 min (not shown), as well as for RDC018 and TF10, with the new peak at 7.3 to 7.4 min (not shown), and for RDC018 and TF12, with a new peak at 8.0 to 8.1 min (not shown). These new peaks were also detected by fluorescence (not shown). The appearance of new, fluorescent-labeled peaks at earlier retention time shows the ability of RDC017 and RDC018 targetable construct peptides to bind to anti-HSG antibody DNL™ complexes was not affected by the presence of the fluorescent probe molecules. These results show that fluorescently labeled targetable constructs are suitable for *in vitro* and *in vivo* imaging, detection and/or diagnosis.

### Discussion

Maleimide conjugation to a thiol to form a stable thioether bond has been well established in biochemical systems. Both DYLIGHT® dyes were obtained in the maleimide form and ready for thiol conjugation. The reaction was done at pH ∼7 and under inert conditions to prevent the oxidation of IMP-499 to its disulfide form. Separation from the starting materials was achieved using reverse-phase HPLC under a linear gradient to obtain the pure product. The product was confirmed using LC-MS and purity confirmed through analytical RP-HPLC with and without fluorescence detection.

In both syntheses, the dye was conjugated to the C-terminal end of the peptide scaffold for the targeting bis-HSG arms and we therefore would not have expected the ability of HSG to bind to h679 antibody to be compromised. This was demonstrated using the DNL™ bispecific constructs, TF10 and TF12, which contain the h679 Fab fragment. The formation of hapten- antibody complex, as confirmed by the formation of a new peak at an earlier retention time, was observed using size-exclusion HPLC monitored using the absorbance peak for the dye (RDC017 λ 493 nm and RDC018 λ 777nm) in addition to λ 280 nm. SE-HPLC with fluorescence detection also demonstrated hapten-antibody complex formation and the fluorescence activity of the dye-hapten conjugate. These results confirm that the hapten's ability to bind to h679 was not compromised by the conjugation of the dyes, DYLIGHT® dye 488 or DYLIGHT® dye 800, at the C-terminal end of the peptide scaffold.

We have synthesized two hydrophilic dye conjugates of IMP-499, namely RDC017 and RDC018, and have shown retention of its fluorescence activity and its immunoreactivity to h679. With the conjugation, we do not expect the molar absorptivity of the dye to be altered from its original value and have used it to quantify the amount of material in solution. For RDC017, the assumed molar extinction coefficient of the DYLIGHT® dye 488 at λ 493 nm is 70,000 M⁻¹cm⁻¹ while for RDC018, the assumed molar extinction coefficient at λ 777 nm is 270,000 M⁻¹cm⁻¹.

### Example 2. Preparation of DNL™ Constructs for Fluorescent Imaging by Pretargeting

The technique for making DNL™ constructs can produce dimers, trimers, tetramers, hexamers, etc. comprising virtually any antibodies or fragments thereof or other effector moieties. For certain preferred embodiments, IgG antibodies, Fab fragments or other proteins or peptides may be produced as fusion proteins containing either a DDD (dimerization and docking domain) or AD (anchoring domain) sequence. Bispecific antibodies may be formed by combining a Fab-DDD fusion protein of a first antibody with a Fab-AD fusion protein of a second antibody. Alternatively, constructs may be made that combine IgG-AD fusion proteins with Fab-DDD fusion proteins. For purposes of fluorescent imaging, detection and/or diagnosis, an antibody or fragment containing a binding site for an antigen associated with a target tissue to be imaged, such as a tumor, may be combined with a second antibody or fragment that binds a hapten on a targetable construct, such as RDC017 or RDC018. The bispecific antibody DNL™ construct is administered to a subject, circulating antibody is allowed to clear from the blood and localize to target tissue, and the fluorescent-labeled targetable construct is added and binds to the localized antibody for imaging.

Independent transgenic cell lines may be developed for each Fab or IgG fusion protein. Once produced, the modules can be purified if desired or maintained in the cell culture supernatant fluid. Following production, any DDD₂-fusion protein module can be combined with any corresponding AD-fusion protein module to generate a bispecific DNL™ construct. For different types of constructs, different AD or DDD sequences may be utilized. The following DDD sequences are based on the DDD moiety of PKA RIIα, while the AD sequences are based on the AD moiety of the optimized synthetic AKAP-*IS* sequence (Alto et al., Proc. Natl. Acad. Sci. USA. 2003; 100:4445).
DDD1: SHIQIPPGLTELLQGYTVEVLRQQPPDLVEFAVEYFTRLREARA (SEQ ID NO:1)
DDD2: CGHIQIPPGLTELLQGYTVEVLRQQPPDLVEFAVEYFTRLREARA (SEQ ID NO:2)
AD1: QIEYLAKQIVDNAIQQA (SEQ ID NO:3)
AD2: CGQIEYLAKQIVDNAIQQAGC (SEQ ID NO:4)

The plasmid vector pdHL2 has been used to produce a number of antibodies and antibody-based constructs. See Gillies et al., J Immunol Methods (1989), 125:191-202; Losman et al., Cancer (Phila) (1997), 80:2660-6. The di-cistronic mammalian expression vector directs the synthesis of the heavy and light chains of IgG. The vector sequences are mostly identical for many different IgG-pdHL2 constructs, with the only differences existing in the variable domain (VH and VL) sequences. Using molecular biology tools known to those skilled in the art, these IgG expression vectors can be converted into Fab-DDD or Fab-AD expression vectors. To generate Fab-DDD expression vectors, the coding sequences for the hinge, CH2 and CH3 domains of the heavy chain are replaced with a sequence encoding the first 4 residues of the hinge, a 14 residue Gly-Ser linker and the first 44 residues of human RIIα (referred to as DDD1). To generate Fab-AD expression vectors, the sequences for the hinge, CH2 and CH3 domains of IgG are replaced with a sequence encoding the first 4 residues of the hinge, a 15 residue Gly-Ser linker and a 17 residue synthetic AD called AKAP-IS (referred to as AD1), which was generated using bioinformatics and peptide array technology and shown to bind RIIα dimers with a very high affinity (0.4 nM). See Alto, et al. Proc. Natl. Acad. Sci., U.S.A (2003), 100:4445-50.

Two shuttle vectors were designed to facilitate the conversion of IgG-pdHL2 vectors to either Fab-DDD1 or Fab-AD1 expression vectors, as described below.

### Preparation of CH1

The CH1 domain was amplified by PCR using the pdHL2 plasmid vector as a template. The left PCR primer consisted of the upstream (5') end of the CH1 domain and a SacII restriction endonuclease site, which is 5' of the CH1 coding sequence. The right primer consisted of the sequence coding for the first 4 residues of the hinge followed by four glycines and a serine, with the final two codons (GS) comprising a Bam HI restriction site. The 410 bp PCR amplimer was cloned into the pGemT PCR cloning vector (Promega, Inc.) and clones were screened for inserts in the T7 (5') orientation.

A duplex oligonucleotide was synthesized to code for the amino acid sequence of DDD1 preceded by 11 residues of a linker peptide, with the first two codons comprising a BamHI restriction site. A stop codon and an EagI restriction site are appended to the 3'end. The encoded polypeptide sequence is shown below, with the DDD1 sequence underlined. GSGGGGSGGGGSHIQIPPGLTELLOGYTVEVLRQQPPDLVEFAVEYFTRLREARA (SEQ ID NO:89)

Two oligonucleotides, designated RIIA1-44 top and RIIA1-44 bottom, that overlap by 30 base pairs on their 3' ends, were synthesized (Sigma Genosys) and combined to comprise the central 154 base pairs of the 174 bp DDD1 sequence. The oligonucleotides were annealed and subjected to a primer extension reaction with Taq polymerase. Following primer extension, the duplex was amplified by PCR. The amplimer was cloned into pGemT and screened for inserts in the T7 (5') orientation.

A duplex oligonucleotide was synthesized to code for the amino acid sequence of AD1 preceded by 11 residues of the linker peptide with the first two codons comprising a BamHI restriction site. A stop codon and an EagI restriction site are appended to the 3'end. The encoded polypeptide sequence is shown below, with the sequence of AD1 underlined.
GSGGGGSGGGGSQIEYLAKQIVDNAIQQA (SEQ ID NO:90)

Two complimentary overlapping oligonucleotides encoding the above peptide sequence, designated AKAP-IS Top and AKAP-IS Bottom, were synthesized and annealed. The duplex was amplified by PCR. The amplimer was cloned into the pGemT vector and screened for inserts in the T7 (5') orientation.

### Ligating DDD1 with CH1

A 190 bp fragment encoding the DDD1 sequence was excised from pGemT with BamHI and NotI restriction enzymes and then ligated into the same sites in CH1-pGemT to generate the shuttle vector CH1-DDD1-pGemT.

### Ligating AD1 with CH1

A 110 bp fragment containing the AD1 sequence was excised from pGemT with BamHI and NotI and then ligated into the same sites in CH1-pGemT to generate the shuttle vector CH1-AD1-pGemT.

### Cloning CH1-DDD1 or CH1-AD1 into pdHL2-based vectors

With this modular design either CH1-DDD1 or CH1-AD1 can be incorporated into any IgG construct in the pdHL2 vector. The entire heavy chain constant domain is replaced with one of the above constructs by removing the SacII/EagI restriction fragment (CH1-CH3) from pdHL2 and replacing it with the SacII/EagI fragment of CH1-DDD1 or CH1-AD1, which is excised from the respective pGemT shuttle vector.

### Construction of h679-Fd-ADl-pdHL2

h679-Fd-AD1-pdHL2 is an expression vector for production of h679 Fab with AD1 coupled to the carboxyl terminal end of the CH1 domain of the Fd via a flexible Gly/Ser peptide spacer composed of 14 amino acid residues. A pdHL2-based vector containing the variable domains of h679 was converted to h679-Fd-AD1-pdHL2 by replacement of the SacII/EagI fragment with the CH1-AD1 fragment, which was excised from the CH1-AD1-SV3 shuttle vector with SacII and EagI.

### Construction of C-DDD1-Fd-hMN-14-pdHL2

C-DDDl-Fd-hMN-14-pdHL2 is an expression vector for production of a stable dimer that comprises two copies of a fusion protein C-DDD1-Fab-hMN-14, in which DDD1 is linked to hMN-14 Fab at the carboxyl terminus of CH1 via a flexible peptide spacer. The plasmid vector hMN14(I)-pdHL2, which has been used to produce hMN-14 IgG, was converted to C-DDD1-Fd-hMN-14-pdHL2 by digestion with SacII and EagI restriction endonucleases to remove the CH1-CH3 domains and insertion of the CH1-DDD1 fragment, which was excised from the CH1-DDD1-SV3 shuttle vector with SacII and EagI.

The same technique has been utilized to produce plasmids for Fab expression of a wide variety of known antibodies, such as hLL1, hLL2, hPAM4, hRl, hRS7, hMN-14, hMN-15, hA19, hA20 and many others. Generally, the antibody variable region coding sequences were present in a pdHL2 expression vector and the expression vector was converted for production of an AD- or DDD-fusion protein as described above. The AD- and DDD-fusion proteins comprising a Fab fragment of any of such antibodies may be combined, in an approximate ratio of two DDD-fusion proteins per one AD-fusion protein, to generate a trimeric DNL™ construct comprising two Fab fragments of a first antibody and one Fab fragment of a second antibody.

### C-DDD2-Fd-hMN-14-pdHL2

C-DDD2-Fd-hMN-14-pdHL2 is an expression vector for production of C-DDD2-Fab-hMN-14, which possesses a dimerization and docking domain sequence of DDD2 appended to the carboxyl terminus of the Fd of hMN-14 via a 14 amino acid residue Gly/Ser peptide linker. The fusion protein secreted is composed of two identical copies of hMN-14 Fab held together by non-covalent interaction of the DDD2 domains.

Two overlapping, complimentary oligonucleotides, which comprise the coding sequence for part of the linker peptide and residues 1-13 of DDD2, were made synthetically. The oligonucleotides were annealed and phosphorylated with T4 PNK, resulting in overhangs on the 5' and 3' ends that are compatible for ligation with DNA digested with the restriction endonucleases BamHI and PstI, respectively.

The duplex DNA was ligated with the shuttle vector CH1-DDD1-pGemT, which was prepared by digestion with BamHI and PstI, to generate the shuttle vector CH1-DDD2-pGemT. A 507 bp fragment was excised from CH1-DDD2-pGemT with SacII and EagI and ligated with the IgG expression vector hMN14(I)-pdHL2, which was prepared by digestion with SacII and EagI. The final expression construct was designated C-DDD2-Fd-hMN-14-pdHL2. Similar techniques have been utilized to generated DDD2-fusion proteins of the Fab fragments of a number of different humanized antibodies.

### H679-Fd-AD2-pdHL2

h679-Fab-AD2, was designed to pair as **B** to C-DDD2-Fab-hMN-14 as **A.** h679-Fd-AD2-pdHL2 is an expression vector for the production of h679-Fab-AD2, which possesses an anchor domain sequence of AD2 appended to the carboxyl terminal end of the CH1 domain via a 14 amino acid residue Gly/Ser peptide linker. AD2 has one cysteine residue preceding and another one following the anchor domain sequence of AD1.

The expression vector was engineered as follows. Two overlapping, complimentary oligonucleotides (AD2 Top and AD2 Bottom), which comprise the coding sequence for AD2 and part of the linker sequence, were made synthetically. The oligonucleotides were annealed and phosphorylated with T4 PNK, resulting in overhangs on the 5' and 3' ends that are compatible for ligation with DNA digested with the restriction endonucleases BamHI and SpeI, respectively.

The duplex DNA was ligated into the shuttle vector CH1-AD1-pGemT, which was prepared by digestion with BamHI and SpeI, to generate the shuttle vector CH1-AD2-pGemT. A 429 base pair fragment containing CH1 and AD2 coding sequences was excised from the shuttle vector with SacII and EagI restriction enzymes and ligated into h679-pdHL2 vector that prepared by digestion with those same enzymes. The final expression vector is h679-Fd-AD2-pdHL2.

### Example 3. Generation of TF2 DNL™ Construct

A trimeric DNL™ construct designated TF2 was obtained by reacting C-DDD2-Fab-hMN-14 with h679-Fab-AD2. A pilot batch of TF2 was generated with >90% yield as follows. Protein L-purified C-DDD2-Fab-hMN-14 (200 mg) was mixed with h679-Fab-AD2 (60 mg) at a 1.4:1 molar ratio. The total protein concentration was 1.5 mg/ml in PBS containing 1 mM EDTA. Subsequent steps involved TCEP reduction, HIC chromatography, DMSO oxidation, and affinity chromatography on an HSG-conjugated column. Before the addition of TCEP, SE-HPLC did not show any evidence of **a₂b** formation. Addition of 5 mM TCEP rapidly resulted in the formation of **a₂b** complex consistent with a 157 kDa protein expected for the binary structure. TF2 was purified to near homogeneity by affinity chromatography on an HSG column (not shown). SE-HPLC analysis of the affinity purified fraction demonstrated the removal of a₄, a₂ and free kappa chains from the product (not shown).

Non-reducing SDS-PAGE analysis demonstrated that the majority of TF2 exists as a large, covalent structure with a relative mobility near that of IgG (not shown). Additional bands suggest that disulfide formation is incomplete under these experimental conditions (not shown). Reducing SDS-PAGE shows that any additional bands apparent in the non-reducing gel are product-related (not shown), as only bands representing the constituent polypeptides of TF2 are evident. MALDI-TOF mass spectrometry (not shown) revealed a single peak of 156,434 Da, which is within 99.5% of the calculated mass (157,319 Da) of TF2.

The functionality of TF2 was determined by BIACORE™ assay. TF2, C-DDD1-hMN-14+h679-AD1 (used as a control sample of noncovalent **a₂b** complex), or C-DDD2-hMN-14+h679-AD2 (used as a control sample of unreduced a₂ and b components) were diluted to 1 µg/ml (total protein) and passed over a sensorchip immobilized with HSG. The response for TF2 was approximately two-fold that of the two control samples, indicating that only the h679-Fab-AD component in the control samples would bind to and remain on the sensorchip. Subsequent injections of WI2 IgG, an anti-idiotype antibody for hMN-14, demonstrated that only TF2 had a DDD-Fab-hMN-14 component that was tightly associated with h679-Fab-AD as indicated by an additional signal response. The additional increase of response units resulting from the binding of WI2 to TF2 immobilized on the sensorchip corresponded to two fully functional binding sites, each contributed by one subunit of C-DDD2-Fab-hMN-14. This was confirmed by the ability of TF2 to bind two Fab fragments of WI2 (not shown).

### Example 4. Production of TF10 and TF12 DNL™ Constructs

A similar protocol to that used to generate the TF2 construct was used to generate a trimeric TF10 DNL™ construct, comprising two copies of a C-DDD2-Fab-hPAM4 and one copy of C-AD2-Fab-679. The TF10 bispecific ([hPAM4]₂ x h679) antibody was produced using the method disclosed for production of the (anti CEA)₂ x anti HSG bsAb TF2, as described above. The TF10 construct bears two humanized PAM4 Fabs and one humanized 679 Fab.

The two fusion proteins (hPAM4-DDD2 and h679-AD2) were expressed independently in stably transfected myeloma cells. The tissue culture supernatant fluids were combined, resulting in a two-fold molar excess of hPAM4-DDD2. The reaction mixture was incubated at room temperature for 24 hours under mild reducing conditions using 1 mM reduced glutathione. Following reduction, the DNL™ reaction was completed by mild oxidation using 2 mM oxidized glutathione. TF10 was isolated by affinity chromatography using an HSG-conjugated affigel resin, which binds with high specificity to the h679 Fab.

The same technique was utilized to produce the TF12 DNL™ construct, comprising two copies of anti-EGP-1 (anti-TROP2) hRS7 Fab-DDD2 and one copy of anti-HSG 679 Fab-AD2. The TF12 construct retained binding activity for EGP-1 (TROP2) and HSG.

### Example 5. Production of IgG-Based DNL™ Subunits

C-H-AD2-IgG modules have an AD2 peptide fused to the carboxyl terminus (C) of the heavy (H) chain of IgG via a peptide linker. The DNA coding sequences for the linker peptide (GSGGGGSGG, SEQ ID NO:91) followed by the AD2 peptide (CGQIEYLAKQIVDNAIQQAGC, SEQ ID NO:4) are coupled to the 3' end of the CH3 (heavy chain constant domain 3) coding sequence by standard recombinant DNA methodologies, resulting in a contiguous open reading frame. When the heavy chain-AD2 polypeptide is co-expressed with a light chain polypeptide, an IgG molecule is formed possessing two C-terminal AD2 peptides, which can therefore bind two Fab-DDD2 dimers or to any other DDD2-conjugated effector moiety. Attachment of the AD2 moieties at the C-terminal end of the IgG minimizes any steric interference with the antigen-binding sites located at the N-terminal end. The C-H-AD2-IgG module can be combined with any Fab-DDD2 module to generate a wide variety of hexavalent structures composed of an IgG antibody and four Fab fragments. If the C-H-AD2-IgG module and the Fab-DDD2 module are derived from the same parental monoclonal antibody (MAb) the resulting DNL™ construct is monospecific with 6 binding arms to the same antigen. If the modules are instead derived from two different MAbs then the resulting DNL™ constructs are bispecific, with two binding arms for the specificity of the C-H-AD2-IgG module and 4 binding arms for the specificity of the Fab-DDD2 module. In alternative embodiments, combination of IgG-AD2 or Fab-AD2 modules with any DDD2-linked effector moiety can be used to produce an IgG or Fab₂ antigen-binding DNL™ construct attached to four copies of the effector moiety.

### Example 6. Production of AD- and DDD-linked Fab and IgG Fusion Proteins From Multiple Antibodies

Using the techniques described in the preceding Examples, the IgG and Fab fusion proteins shown in **Table 5** were constructed and incorporated into DNL constructs. The fusion proteins retained the antigen-binding characteristics of the parent antibodies and the DNL constructs exhibited the antigen-binding activities of the incorporated antibodies or antibody fragments.

**Table 5. Fusion proteins comprising IgG or Fab**

| **Fusion Protein** | **Binding Specificity** |
|---|---|
| **C-AD1-Fab-h679** | **HSG** |
| **C-AD2-Fab-h679** | **HSG** |
| **C-(AD)₂-Fab-h679** | **HSG** |
| **C-AD2-Fab-h734** | **Indium-DTPA** |
| **C-AD2-Fab-hA20** | **CD20** |
| **C-AD2-Fab-hA20L** | **CD20** |
| **C-AD2-Fab-hL243** | **HLA-DR** |
| **C-AD2-Fab-hLL2** | **CD22** |
| **N-AD2-Fab-hLL2** | **CD22** |
| **C-AD2-IgG-hMN-14** | **CEACAM5** |
| **C-AD2-IgG-hRl** | **IGF-1R** |
| **C-AD2-IgG-hRS7** | **EGP-1** |
| **C-AD2-IgG-hPAM4** | **MUC** |
| **C-AD2-IgG-hLL1** | **CD74** |
| | |
| **C-DDD1-Fab-hMN-14** | **CEACAM5** |
| **C-DDD2-Fab-hMN-14** | **CEACAM5** |
| **C-DDD2-Fab-h679** | **HSG** |
| **C-DDD2-Fab-hA19** | **CD19** |
| **C-DDD2-Fab-hA20** | **CD20** |
| **C-DDD2-Fab-hAFP** | **AFP** |
| **C-DDD2-Fab-hL243** | **HLA-DR** |
| **C-DDD2-Fab-hLL1** | **CD74** |
| **C-DDD2-Fab-hLL2** | **CD22** |
| **C-DDD2-Fab-hMN-3** | **CEACAM6** |
| **C-DDD2-Fab-hMN-15** | **CEACAM6** |
| **C-DDD2-Fab-hPAM4** | **MUC** |
| **C-DDD2-Fab-hR1** | **IGF-1R** |
| **C-DDD2-Fab-hRS7** | **EGP-1** |
| **N-DDD2-Fab-hMN-14** | **CEACAM5** |

### Example 7. Production and Use of Other Targetable Constructs for Fluorescent Imaging, Detection and/or Diagnosis

In various embodiments, targetable construct peptides may be utilized which contain a chelating moiety, such as NOTA, NODA, NETA, DTPA, DOTA, etc. The chelator may be utilized to attach a therapeutic or diagnostic agent, such as a radionuclide. Alternatively, some chelating moieties, such as In-DTPA, provide haptens of use in binding to bispecific or multispecific antibodies. In certain non-limiting embodiments discussed below, a chelating group may be used to attach ¹⁸F or ¹⁹F complexed with a metal, such as aluminum, to provide an alternative modality for imaging, detection and/or diagnosis. It is anticipated that fluorescent-labeled molecules may be of more use for intraoperative procedures, while ¹⁸F-labeled molecules may be of greater use for pre- or post-operative imaging, detection and/or diagnosis of diseased tissues. The targetable constructs may be modified to contain sulfhydryl groups for attaching maleimide-modified fluorescent probes, as discussed in Example 1 above. Alternatively, bis-functional cross-linking agents, or fluorescent dyes conjugated to other reactive species, may be used to attach the fluorescent probe to a different group on the targetable construct. For example, DYLIGHT® 488 and DYLIGHT® 800 are available as amine-reactive dyes derivatized with NHS ester for labeling primary amines (Product Nos. 46402 and 46421, Thermo Electric, Rockford, IL). Each of the peptides disclosed below contains a primary amine that could be conjugated to an amine-reactive DYLIGHT® dye. The skilled artisan will realize that the fluorescent probes of use are not limiting and other DYLIGHT® dyes, or alternative fluorescent probe molecules known in the art, may be used in the claimed methods and compositions.
*IMP-449*
NOTA-ITC benzyl-D-Ala-D-Lys(HSG)-D-Tyr-D-Lys(HSG)-NH₂ (SEQ ID NO:92)

The peptide, IMP-448 (D-Ala-D-Lys(HSG)-D-Tyr-D-Lys(HSG)-NH₂, SEQ ID NO:93) was made on Sieber Amide resin by adding the following amino acids to the resin in the order shown: Aloc-D-Lys(Fmoc)-OH, Trt-HSG-OH, the Aloc was cleaved, Fmoc-D-Tyr(But)-OH, Aloc-D-Lys(Fmoc)-OH, Trt-HSG-OH, the Aloc was cleaved, Fmoc-D-Ala-OH with final Fmoc cleavage to make the desired peptide. The peptide was then cleaved from the resin and purified by HPLC to produce IMP-448, which was then coupled to ITC-benzyl NOTA.

IMP-448 (0.0757g, 7.5 x 10⁻⁵ mol) was mixed with 0.0509 g (9.09 x 10⁻⁵ mol) ITC benzyl NOTA and dissolved in 1 mL water. Potassium carbonate anhydrous (0.2171 g) was then slowly added to the stirred peptide/NOTA solution. The reaction solution was pH 10.6 after the addition of all the carbonate. The reaction was allowed to stir at room temperature overnight. The reaction was carefully quenched with 1 M HCl after 14 hr and purified by HPLC to obtain 48 mg of IMP-449.
*IMP-460*
NODA-Ga-D-Ala-D-Lys(HSG)-D-Tyr-D-Lys(HSG)-NH₂ (SEQ ID NO:94)

IMP-460 NODA-Ga-D-Ala-D-Lys(HSG)-D-Tyr-D-Lys(HSG)-NH₂ (SEQ ID NO:94) was chemically synthesized. The NODA-Ga ligand was purchased from CHEMATECH® and attached on the peptide synthesizer like the other amino acids. The peptide was synthesized on Sieber amide resin with the amino acids and other agents added in the following order Aloc-D-Lys(Fmoc)-OH, Trt-HSG-OH, Aloc removal, Fmoc-D-Tyr(But)-OH, Aloc-D-Lys(Fmoc)-OH, Trt-HSG-OH, Aloc removal, Fmoc-D-Ala-OH, and NODA-GA(tBu)₃. The peptide was then cleaved and purified by HPLC to afford the product. HRMS C61H92N18O18.

### Synthesis of Di-t-butyl-NOTA

NO2A*t*Bu (0.501 g 1.4 x 10⁻³ mol) was dissolved in 5 mL anhydrous acetonitrile. Benzyl-2-bromoacetate (0.222 mL, 1.4 x 10⁻³ mol) was added to the solution followed by 0.387 g of anhydrous K₂CO₃. The reaction was allowed to stir at room temperature overnight. The reaction mixture was filtered and concentrated to obtain 0.605 g (86 % yield) of the benzyl ester conjugate. The crude product was then dissolved in 50 mL of isopropanol, mixed with 0.2 g of 10 % Pd/C (under Ar) and placed under 50 psi H₂ for 3 days. The product was then filtered and concentrated under vacuum to obtain 0.462 g of the desired product ESMS MH⁻ 415.
*IMP-461*
NOTA-D-Ala-D-Lys(HSG)-D-Tyr-D-Lys(HSG)-NH2 (SEQ ID NO:95)

The peptide was synthesized on Sieber amide resin with the amino acids and other agents added in the following order Aloc-D-Lys(Fmoc)-OH, Trt-HSG-OH, Aloc removal, Fmoc-D-Tyr(But)-OH, Aloc-D-Lys(Fmoc)-OH, Trt-HSG-OH, Aloc removal, Fmoc-D-Ala-OH, and Bis-t-butylNOTA-OH. The peptide was then cleaved and purified by HPLC to afford the product IMP-461 ESMS MH⁺1294 (NOTA-D-Ala-D-Lys(HSG)-D-Tyr-D-Lys(HSG)-NH₂; SEQ ID NO:95).
*IMP-462*
NOTA-D-Asp-D-Lys(HSG)-D-Tyr-D-Lys(HSG)-NH₂ (SEQ ID NO:96)

The peptide was synthesized on Sieber amide resin with the amino acids and other agents added in the following order Aloc-D-Lys(Fmoc)-OH, Trt-HSG-OH, Aloc removal, Fmoc-D-Tyr(But)-OH, Aloc-D-Lys(Fmoc)-OH, Trt-HSG-OH, Aloc removal, Fmoc-D-Asp(But)-OH, and Bis-t-butylNOTA-OH. The peptide was then cleaved and purified by HPLC to afford the product IMP-462 ESMS MH⁺1338 (NOTA-D-Asp-D-Lys(HSG)-D-Tyr-D-Lys(HSG)-NH₂; SEQ ID NO:96).
*IMP-467*
C-NETA-succinyl-D-Lys(HSG)-D-Tyr-D-Lys(HSG)-NH₂ (SEQ ID NO:97)

Tetra *tert*-butyl *C*-NETA-succinyl was produced. The *tert-Butyl* {4-[2-(Bis-(*tert-*butyoxycarbonyl)methyl-3-(4-nitrophenyl)propyl]-7-*tert*butyoxycarbonyl[1,4,7]triazanonan-1-yl} was prepared as described in Chong et al. (J. Med. Chem. 2008, 51:118-125).

The peptide, IMP-467 *C*-NETA-succinyl-D-Lys(HSG)-D-Tyr-D-Lys(HSG)-NH₂ (SEQ ID NO:97) was made on Sieber Amide resin by adding the following amino acids to the resin in the order shown: Aloc-D-Lys(Fmoc)-OH, Trt-HSG-OH, the Aloc was cleaved Fmoc-D-Tyr(But)-OH, Aloc-D-Lys(Fmoc)-OH, Trt-HSG-OH, the Aloc was cleaved, *tert-*Butyl{4-[Bis-(*tert*-butoxycarbonylmethyl)amino)-3-(4-succinylamidophenyl)propyl]-7-*tert-*butoxycarbonylmethyl[1,4,7]triazanonan-1-yl}acetate. The peptide was then cleaved from the resin and purified by RP-HPLC to yield 6.3 mg of IMP-467. The crude peptide was purified by high performance liquid chromatography (HPLC) using a C18 column.
*IMP-468*
NOTA-NH-(CH₂)₇CO-Gln-Trp-Val-Trp-Ala-Val-Gly-His-Leu-Met-NH₂ (SEQ ID NO:98)

The targeting molecules used to direct a fluorescent probe to a disease-associated antigen, cell or tissue are not limited to antibodies or antibody fragments, but rather can include any molecule that binds specifically or selectively to a cellular target that is associated with or diagnostic of a disease state or other condition that may be imaged by fluorescent labeling. Bombesin is a 14 amino acid peptide that is homologous to neuromedin B and gastrin releasing peptide, as well as a tumor marker for cancers such as lung and gastric cancer and neuroblastoma. IMP-468 (NOTA-NH-(CH₂)₇CO-Gln-Trp-Val-Trp-Ala-Val-Gly-His-Leu-Met-NH₂; SEQ ID NO:98) was synthesized as a bombesin analogue to labeled and target the gastrin-releasing peptide receptor.

The peptide was synthesized by Fmoc based solid phase peptide synthesis on Sieber amide resin, using a variation of a synthetic scheme reported in the literature (Prasanphanich et al., 2007, PNAS USA 104:12463-467). The synthesis was different in that a bis-t-butyl NOTA ligand was add to the peptide during peptide synthesis on the resin.
*IMP-470*
*L*-NETA-succinyl-D-Lys(HSG)-D-Tyr-D-Lys(HSG)-NH₂ (SEQ ID NO:99)

The peptide IMP-470 was made on Sieber Amide resin by adding the following amino acids to the resin in the order shown: Aloc-D-Lys(Fmoc)-OH, Trt-HSG-OH, the Aloc was cleaved, Fmoc-D-Tyr(But)-OH, Aloc-D-Lys(Fmoc)-OH, Trt-HSG-OH. The free amine obtained after the removal of Aloc was reacted with succinic anhydride, to generate a carboxylic acid group at the N-terminus, which is activated using DIC in DMF and subsequently coupled with *tert*-butyl protected L-NETA. The peptide was then cleaved from the resin and purified by RP-HPLC to yield 16.4 mg of IMP-470. A product with molecular mass 1037.15 corresponding to the peptide without L-NETA and with retention time 9.001 min was also obtained.
*IMP-485*
NODA-MPAA-D-LYS(HSG)-D-TYR-D-LYS(HSG)-NH₂ (SEQ ID NO:100)

IMP-485 (see U.S. Patent No. 8,202,509) was made on Sieber Amide resin by adding the following amino acids to the resin in the order shown: Aloc-D-Lys(Fmoc)-OH, Trt-HSG-OH, the Aloc was cleaved, Fmoc-D-Tyr(But)-OH, Aloc-D-Lys(Fmoc)-OH, Trt-HSG-OH, the Aloc was cleaved, (*tert*-Butyl)₂NODA-MPAA (methyl phenyl acetic acid). The peptide was then cleaved from the resin and purified by RP-HPLC to yield 44.8 mg of IMP-485.

### Example 8. ¹⁸F-Labeling of Targetable Construct Peptides

In certain embodiments, it may be preferred to label a targetable construct peptide with an alternative label, such as ¹⁸F. Peptides comprising a chelating moiety and a sulfhydryl, primary amine or other reactive groups may be dual-labeled with both ¹⁸F and a fluorescent probe molecule. In more preferred embodiments, ¹⁸F labeling may be performed by forming a complex between ¹⁸F and a Group III metal, such as aluminum. The Al-¹⁸F complex may bind to a NOTA, NODA, or other chelating moiety to form a stable ¹⁸F-labeled molecule.

### ¹⁸F Labeling of IMP-449

IMP-449 (0.002 g, 1.37 x 10⁻⁶ mol), produced as discussed above, was dissolved in 686 µL (2 mM peptide solution) 0.1 M NaOAc pH 4.02. Three microliters of a 2 mM solution of Al in a pH 4 acetate buffer was mixed with 15 µL, 1.3 mCi of ¹⁸F. The solution was then mixed with 20 µL of the 2 mM IMP-449 solution and heated at 105 °C for 15 min. Reverse Phase HPLC analysis showed 35% (*t*_{R} ∼ 10 min) of the activity was attached to the peptide and 65% of the activity was eluted at the void volume of the column (3.1 min, not shown) indicating that the majority of activity was not associated with the peptide. The crude labeled mixture (5 µL) was mixed with pooled human serum and incubated at 37°C. An aliquot was removed after 15 min and analyzed by HPLC. The HPLC showed 9.8 % of the activity was still attached to the peptide (down from 35 %). Another aliquot was removed after 1 hr and analyzed by HPLC. The HPLC showed 7.6 % of the activity was still attached to the peptide (down from 35 %), which was essentially the same as the 15 min trace (data not shown).

### High Dose ¹⁸F Labeling

Further studies with purified IMP-449 demonstrated that the ¹⁸F-labeled peptide was highly stable (91%, not shown) in human serum at 37°C for at least one hour and was partially stable (76%, not shown) in human serum at 37°C for at least four hours. Additional studies were performed in which the IMP-449 was prepared in the presence of ascorbic acid as a stabilizing agent. In those studies (not shown), the metal-¹⁸F-peptide complex showed no detectable decomposition in serum after 4 hr at 37°C. The mouse urine 30 min after injection of ¹⁸F-labeled peptide was found to contain ¹⁸F bound to the peptide (not shown). These results demonstrate that the ¹⁸F-labeled peptides disclosed herein exhibit sufficient stability under approximated *in vivo* conditions to be used for ¹⁸F imaging studies.

Since IMP-449 peptide contains a thiourea linkage, which is sensitive to radiolysis, several products are observed by RP-HPLC. However, when ascorbic acid is added to the reaction mixture, the side products generated are markedly reduced.

### Example 9. In Vivo Imaging With Pretargeting Antibody and Targetable Construct

Taconic nude mice bearing the four slow-growing sc CaPan1 xenografts were used for *in vivo* studies. Three of the mice were injected with TF10 (162 µg) followed with [Al¹⁸F] IMP-449 18 h later. TF10 is a humanized bispecific antibody of use for tumor imaging studies, with divalent binding to the PAM-4 defined tumor antigen and monovalent binding to HSG (see, e.g., Gold et al., 2007, J. Clin. Oncol. 25(18S):4564). One mouse was injected with peptide alone. All of the mice were necropsied at 1 h post peptide injection. Tissues were counted immediately. Comparison of mean distributions showed substantially higher levels of ¹⁸F-labeled peptide localized in the tumor than in any normal tissues in the presence of tumor-targeting bispecific antibody.

Tissue uptake was similar in animals given the [Al¹⁸F] IMP-449 alone or in a pretargeting setting (**Table 6**). Uptake in the human pancreatic cancer xenograft, CaPan1, at 1 h was increased 5-fold in the pretargeted animals as compared to the peptide alone (4.6 ± 0.9% ID/g vs. 0.89% ID/g). Exceptional tumor/nontumor ratios were achieved at this time (e.g., tumor/blood and liver ratios were 23.4 ± 2.0 and 23.5 ± 2.8, respectively).

**Table 6. Tissue uptake at 1 h post peptide injection, mean and the individual animals:**

| | **TF10 (162µg) -→18 h →** [Al¹⁸F] IMP-449 **(10 :1)** | | | | | | [Al¹⁸F] IMP-449 **alone** |
|---|---|---|---|---|---|---|---|
| Tissue | n | Mean | SD | Animal 1 | Animal 2 | Animal 3 | Animal 1 |
| Tumor *(mass)* | 3 | 4.591 | 0.854 | **4.330 *(0.675 g)*** | **5.546 *(0.306g)*** | **3.898 *(0.353g)*** | 0.893 *(0.721g)* |
| Liver | 3 | 0.197 | 0.041 | 0.163 | 0.242 | 0.186 | 0.253 |
| Spleen | 3 | 0.202 | 0.022 | 0.180 | 0.224 | 0.200 | 0.226 |
| Kidney | 3 | 5.624 | 0.531 | 5.513 | 6.202 | 5.158 | 5.744 |
| Lung | 3 | 0.421 | 0.197 | 0.352 | 0.643 | 0.268 | 0.474 |
| Blood | 3 | 0.196 | 0.028 | 0.204 | 0.219 | 0.165 | 0.360 |
| Stomach | 3 | 0.123 | 0.046 | 0.080 | 0.172 | 0.118 | 0.329 |
| Small Int. | 3 | 0.248 | 0.042 | 0.218 | 0.295 | 0.230 | 0.392 |
| Large Int. | 3 | 0.141 | 0.094 | 0.065 | 0.247 | 0.112 | 0.113 |
| Pancreas | 3 | 0.185 | 0.078 | 0.259 | 0.194 | 0.103 | 0.174 |
| Spine | 3 | 0.394 | 0.427 | 0.140 | 0.888 | 0.155 | 0.239 |
| Femur | 3 | 3.899 | 4.098 | 2.577 | 8.494 | 0.625 | 0.237 |
| Brain | 3 | 0.064 | 0.041 | 0.020 | 0.072 | 0.100 | 0.075 |
| Muscle | 3 | 0.696 | 0.761 | 0.077 | 1.545 | 0.465 | 0.162 |

### Imaging Methods

Experiments were performed in male nude BALB/c mice (6-8 weeks old), weighing 20-25 grams. Mice received a subcutaneous injection with 0.2 mL of a suspension of 1 x 10⁶ LS174T cells, a CEA-expressing human colon carcinoma cell line (American Type Culture Collection, Rockville, MD, USA). Studies were initiated when the tumors reached a size of about 0.1-0.3 g (10-14 days after tumor inoculation).

Mice with s.c. CEA-expressing LS174T tumors received TF2 (6.0 nmol; 0.94 mg) and 5 MBq ¹⁸F-labeled IMP-449 (0.25 nmol) intravenously, with an interval of 16 hours between the injection of the bispecific antibody and the radiolabeled peptide. One or two hours after the injection of the radiolabeled peptide, PET/CT images were acquired and the biodistribution of the radiolabeled peptide was determined. Uptake in the LS174T tumor was compared with that in an s.c. CEA-negative SK-RC 52 tumor.

PET images were acquired with an Inveon animal PET/CT scanner (Siemens Preclinical Solutions, Knoxville, TN). PET emission scans were acquired for 15 minutes, preceded by CT scans for anatomical reference (spatial resolution 113 µm, 80 kV, 500 µA, exposure time 300 msec).

### Results

The results of imaging with an ¹⁸F-labeled IMP-449 pretargeted with TF2 bispecific antibody DNL complex are shown in **FIG. 4****.** A subcutaneous LS174T tumor (0.1 g) is clearly visible on the right side of an animal that received 6.0 nmol TF2 and 0.25 nmol Al¹⁸F-IMP-449 (5 MBq) intravenously with a 16 hour interval. The animal was imaged one hour after injection of Al¹⁸F-IMP-449. The panel shows the 3D volume rendering of posterior (A), coronal (B) and sagittal views. The only normal tissue visibly labeled in this experiment was the kidney. The tumor-to-background ratio of the Al¹⁸F-IMP-449 signal was 66.

### Conclusions

These results show that pretargeting with labeled targetable constructs and bispecific antibodies can be used for imaging, detection and/or diagnosis of diseased tissues, including but not limited to cancer.

### Example 10. Flow Cytometry Detection of Fluorescent-Labeled Binding to CEACAM6 Antigen in Pancreatic Cancer Cell Lines

RDC017 was synthesized as described in Example 1 above. The product was isolated using RP-HPLC and confirmed by LC-MS. Retention of hapten binding was evaluated as described above using SE-HPLC. Concentration in solution was determined by measuring the absorbance at 493 nm.

Immunophenotyping of pancreatic cell lines Capan-1, BxPC-3, AsPC-1 and MIA PaCa-2 for CEACAM6 expression was evaluated by flow cytometry using hMN15 IgG (anti-CEACAM6, parental IgG) and the corresponding bsAb TF14 (2 x hMN15 Fab x h679 Fab) probed using RDC017. CEACAM6 is highly expressed in Capan-1, BxPC-3 and AsPC-1, but not in MIA PaCa-2, which is in agreement with immunohistochemical staining of tissue microarrays (30) and by cell binding studies indirectly stained with HRP-conjugated secondary antibody. Cell binding of fluorescent RDC017 through TF14 was demonstrated (not shown).

### Example 11. Endoscopic Tumor Detection

The anti-CEA TF2 bispecific antibody (C-DDD2-Fab-hMN-14 x h679-Fab-AD2) is administered i.v. to a patient with a suspected colonic polyp (having a history of recurrent colon polyps), having a recent positive test for hemoglobin in his stool and an elevated blood CEA titer of 12.5 ng/ml. After 16 hours, the IMP-499 targetable construct labeled with DYLIGHT® dye 488 (RDC017) is administered i.v. to the patient. The fiberoptic colonoscope used to detect the fluorescence of the agent targeted to a malignant polyp is similar to the fiberoptic bronchoscope described by Profio et al., Adv. Exp. Med. Biol. 193:43 (1985). The fluorescence intensity is converted to an audio signal, whose pitch is related to the signal's intensity by analyzing the fluorescence in a photomultiplier tube. In this case, the malignant polyp at a distance of 15 cm from the anal verge has a ratio of fluorescence to background of 6:1. The polyp of 0.5 cm in diameter is removed via the colonoscope, fixed in formalin, and processed for histopathology. An adenocarcinoma in the stalk of the polyp is found to be present.

### Example 12. Intraoperative Tumor Therapy

A woman with ovarian cancer having extensive abdominal spread is injected i.v. prior to surgery with the anti-EGP-1 bispecific antibody TF12 (Fab-hRS7-DDD2 x Fab-h679-AD2). Twenty-four hours later, the RDC018 fluorescent targetable construct is injected i.v. The next day, the patient undergoes a resection of all visible and palpable tumors in her abdominal cavity, followed by intraoperative irradiation of the exposed cavity with monochromatic X-rays of 40 keV to destroy micrometastatic cancer spread. The completeness of resection is confirmed by intraoperative spectroscopy using an ODYSSEY® Infrared Imaging System set on the 800 channel. At 6 and 9 months later, no evidence of disease is present, and the patient's blood CA-125 titer is within the normal range, as contrasted to its marked elevation prior to treatment.

## Claims

1. A DNL complex comprising (i) an anti-hapten antibody or antigen-binding fragment thereof conjugated to an AD moiety from an A-kinase anchor protein (AKAP); and (ii) a DDD moiety from human protein kinase A RIα, RIβ, RIIα or RIIβ, conjugated to an antibody or antigen-binding fragment thereof that binds to a target antigen associated with a disease or condition, wherein two copies of the DDD moiety form a dimer that binds to the AD moiety to form the DNL complex, for use in a method for diagnosising the disease or condition comprising detecting the target antigen associated with the disease or condition, wherein the method comprises
a) exposing the target antigen to the DNL complex;
b) allowing the DNL complex to bind to the antigen;
c) adding a targetable construct comprising (iii) at least one hapten; and (iv) a fluorescent probe, wherein the hapten binds to the DNL complex; and
d) detecting the fluorescently labeled target antigen by an intraoperative, intraperitoneal, laparoscopic, endoscopic or intravascular procedure.

2. The DNL complex for use according to claim 1, wherein the targetable construct is selected from the group consisting of IMP-448, IMP-449, IMP-460, IMP-461, IMP-462, IMP-467, IMP-468, IMP-470, IMP-485 and IMP-499.

3. The DNL complex for use according to claim 1, wherein the hapten is HSG or In-DTPA.

4. The DNL complex for use according to claim 1, wherein the antigen is a tumor-associated antigen, an autoimmune disease-associated antigen or an antigen produced or displayed by a pathogenic organism.

5. The DNL complex for use according to claim 4, wherein the tumor-associated antigen is selected from the group consisting of carbonic anhydrase IX, alpha-fetoprotein, α-actinin-4, A3, antigen specific for A33 antibody, ART-4, B7, Ba 733, BAGE, BrE3-antigen, CA125, CAMEL, CAP-1, CASP-8/m, , CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CXCR4, CXCR7, CXCL12, HIF-1a, colon-specific antigen-p (CSAp), CEA (CEACAM5), CEACAM6, c-met, DAM, EGFR, EGFRvIII, EGP-1, EGP-2, ELF2-M, Ep-CAM, Flt-1, Flt-3, folate receptor, G250 antigen, GAGE, gp100, GROB, HLA-DR, HM1.24, human chorionic gonadotropin (HCG) and its subunits, HER2/neu, HMGB-1, hypoxia inducible factor (HIF-1), HSP70-2M, HST-2, la, IGF-1R, IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5, MUC13, MUC16, MUM-1/2, MUM-3, NCA66, NCA95, NCA90, pancreatic cancer mucin, placental growth factor, p53, PLAGL2, prostatic acid phosphatase, PSA, PRAME, PSMA, P1GF, ILGF, ILGF-1R, IL-6, IL-25, RS5, RANTES, T101, SAGE, S100, survivin, survivin-2B, TAC, TAG-72, tenascin, TRAIL receptors, TNF-α, Tn antigen, Thomson-Friedenreich antigens, tumor necrosis antigens, TROP-2, VEGFR, ED-B fibronectin, WT-1, 17-1A-antigen, complement factors C3, C3a, C3b, C5a, C5, an angiogenesis marker, bcl-2, bcl-6, Kras, cMET, an oncogene marker and an oncogene product.

6. The DNL complex for use according to claim 1, wherein the antibody that binds to a target antigen is selected from the group consisting of hPAM4 (anti-mucin), hA20 (anti-CD20), hA19 (anti-CD19), hIMMU31 (anti-AFP), hLL1 (anti-CD74), hLL2 (anti-CD22), hMu-9 (anti-CSAp), hL243 (anti-HLA-DR), hMN-14 (anti-CEACAM5), hMN-15 (anti-CEACAM6), hRS7 (anti-EGP-1), hMN-3 (anti-CEACAM6), Ab124 (anti-CXCR4), Ab125 (anti-CXCR4), abciximab (anti-glycoprotein IIb/IIIa), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), trastuzumab (anti-ErbB2), abagovomab (anti-CA-125), adecatumumab (anti-EpCAM), atlizumab (anti-IL-6R), benralizumab (anti-CD125), CC49 (anti-TAG-72), AB-PG1-XG1-026 (anti-PSMA), D2/B (anti-PSMA), tocilizumab (anti-IL-6R), basiliximab (anti-CD25), daclizumab (anti-CD25), efalizumab (anti-CD11a), GA101 (anti-CD20), muromonab-CD3 (anti-CD3 receptor), natalizumab (anti-α4 integrin), omalizumab (anti-IgE), CDP571 (anti-TNF-α), infliximab (anti-TNF-a), certolizumab (anti-TNF-a), adalimumab (anti-TNF-a), belimumab (anti-B-cell activating factor), Alz 50 (anti-tau protein), gantenerumab (anti-amyloid protein), solanezumab (anti-amyloid protein), P4/D10 (anti-gp120), CR6261 (antiinfluenza), exbivirumab (anti-hepatitis B), felvizumab (anti-respiratory syncytial virus), foravirumab (anti-rabies virus), motavizumab (anti-respiratory syncytial virus), palivizumab (anti-respiratory syncytial virus), panobacumab (anti-Pseudomonas), rafivirumab (anti-rabies virus), regavirumab (anti-cytomegalovirus), sevirumab (anti-cytomegalovirus), tivirumab (anti-hepatitis B), and urtoxazumab (anti-E. coli).

7. The DNL complex for use according to claim 1, wherein the fluorescent probe is selected from the group consisting of Alexa 350, Alexa 430, AMCA, aminoacridine, BODIPY 630/650, BODIPY 650/665, BODIPY-FL, BODIPY-R6G, BODIPY-TMR, BODIPY-TRX, 5-carboxy-4',5'-dichloro-2',7'-dimethoxy fluorescein, 5-carboxy-2',4',5',7'-tetrachlorofluorescein, 5-carboxyfluorescein, 5-carboxyrhodamine, 6-carboxyrhodamine, 6-carboxytetramethyl amino, Cascade Blue, Cy2, Cy3, Cy5,6-FAM, dansyl chloride, fluorescein, HEX, 6-JOE, NBD (7-nitrobenz-2-oxa-1,3-diazole), Oregon Green 488, Oregon Green 500, Oregon Green 514, Pacific Blue, phthalic acid, terephthalic acid, isophthalic acid, cresyl fast violet, cresyl blue violet, brilliant cresyl blue, para-aminobenzoic acid, erythrosine, phthalocyanines, azomethines, cyanines, xanthines, succinylfluoresceins, rare earth metal cryptates, europium trisbipyridine diamine, a europium cryptate or chelate, diamine, dicyanins, La Jolla blue dye, allopycocyanin, allococyanin B, phycocyanin C, phycocyanin R, thiamine, phycoerythrocyanin, phycoerythrin R, REG, Rhodamine Green, rhodamine isothiocyanate, Rhodamine Red, ROX, TAMRA, TET, TRIT (tetramethyl rhodamine isothiol), Tetramethylrhodamine, and Texas Red.

8. The DNL complex for use according to claim 1, wherein the disease is selected from the group consisting of non-Hodgkin's lymphoma, acute lymphoid leukemia, chronic lymphoid leukemia, Burkitt lymphoma, Hodgkin's lymphoma, hairy cell leukemia, acute myeloid leukemia, chronic myeloid leukemia, T-cell lymphoma, T-cell leukemia, multiple myeloma, glioma, Waldenstrom's macroglobulinemia, carcinoma, melanoma, sarcoma, glioma and skin cancer, wherein preferably the carcinoma is a carcinoma of the oral cavity, gastrointestinal tract, pulmonary tract, lung, breast, ovary, prostate, uterus, endometrium, cervix, urinary bladder, pancreas, bone, brain, connective tissue, liver, gall bladder, kidney, skin, central nervous system or testes.

9. The DNL complex for use according to claim 1, wherein the disease is selected from the group consisting of acute immune thrombocytopenia, chronic immune thrombocytopenia, dermatomyositis, Sydenham's chorea, myasthenia gravis, systemic lupus erythematosus, lupus nephritis, rheumatic fever, polyglandular syndromes, bullous pemphigoid, pemphigus vulgaris, Type 1 diabetes, Type 2 diabetes, Henoch-Schonlein purpura, post-streptococcal nephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjögren's syndrome, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis, fibrosing alveolitis, organ transplant rejection and graft-versus-host disease.

10. The DNL complex for use according to claim 1, wherein the anti-hapten antibody and the anti-target antigen antibody are chimeric, humanized or human antibodies.

11. The DNL complex for use according to claim 1, wherein the anti-hapten antibody fragment and the anti-target antigen antibody fragment are selected from the group consisting of F(ab')₂, Fab, scFv or Fv antibody fragments.

12. Kit for use in a method of diagnosis of a disease or condition comprising detecting a fluorescently labeled target antigen associated with the disease or condition by an intraoperative, intraperitoneal, laparoscopic, endoscopic or intravascular procedure, wherein the kit comprises:
a) a DNL complex comprising (i) an anti-hapten antibody or antigen-binding fragment thereof conjugated to an AD moiety from an A-kinase anchor protein (AKAP); and (ii) a DDD moiety from human protein kinase A RIα, RIβ, RIIα or RIIβ, conjugated to an antibody or antigen-binding fragment thereof that binds to the target antigen associated with the disease or condition, wherein two copies of the DDD moiety form a dimer that binds to the AD moiety to form the DNL complex; and
b) a targetable construct comprising (iii) at least one hapten; and (iv) a fluorescent probe, wherein the hapten binds to the DNL complex.

13. Kit for use of claim 12, wherein the targetable construct is selected from the group consisting of IMP-448, IMP-449, IMP-460, IMP-461, IMP-462, IMP-467, IMP-468, IMP-470, IMP-485 and IMP-499.

## Patentansprüche

1. DNL-Komplex umfassend (i) einen anti-Hapten-Antikörper oder ein Antigen-bindendes Fragment davon, konjugiert an einen AD-Teil von einem A-Kinase-Anker-Protein (AKAP); und (ii) einen DDD-Teil von einer menschlichen Proteinkinase A RIα, RIβ, RIIα oder RIIβ, konjugiert an einen Antikörper oder ein Antigen-bindendes Fragment davon, der/das an ein Zielantigen bindet, das mit einer Erkrankung oder einem Zustand verbunden ist, wobei zwei Kopien des DDD-Teils ein Dimer bilden, das an den AD-Teil bindet, um den DNL-Komplex auszubilden, zur Verwendung in einem Verfahren für die Diagnose der Erkrankung oder des Zustandes umfassend Nachweisen des mit der Erkrankung oder dem Zustand verbundenen Zielantigens, wobei das Verfahren umfasst
a) Exponieren des Zielantigens gegenüber dem DNL-Komplex;
b) Erlauben, dass der DNL-Komplex an das Antigen bindet;
c) Hinzugeben eines targetierbaren Konstruktes umfassend (iii) wenigstens ein Hapten; und (iv) eine fluoreszierende Sonde, wobei das Hapten an den DNL-Komplex bindet;
d) Nachweisen des fluoreszenzmarkierten Zielantigens durch eine intraoperative, intraperitoneale, laparoskopische, endoskopische oder intravaskulare Prozedur.

2. DNL-Komplex zur Verwendung nach Anspruch 1, wobei das targetierbare Konstrukt ausgewählt ist aus der Gruppe bestehend aus IMP-448, IMP-449, IMP-460, IMP-461, IMP-462, IMP-467, IMP-468, IMP-470, IMP-485 und IMP-499.

3. DNL-Komplex zur Verwendung nach Anspruch 1, wobei das Hapten HSG oder In-DTPA ist.

4. DNL-Komplex zur Verwendung nach Anspruch 1, wobei das Antigen ein Tumorassoziiertes Antigen, ein mit einer Autoimmunerkrankung verbundenes Antigen oder ein Antigen ist, das von einem pathogenen Organismus produziert oder von diesem gezeigt wird.

5. DNL-Komplex zur Verwendung nach Anspruch 4, wobei das Tumor-assoziierte Antigen ausgewählt ist aus der Gruppe bestehend aus Carboanhydrase IX, alpha-Fetoprotein, α-Actinin-4, A3, A33-Antikörper-spezfischem Antigen, ART-4, B7, Ba 733, BAGE, BrE3-Antigen, CA125, CAMEL, CAP-1, CASP-8/m, , CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CXCR4, CXCR7, CXCL12, HIF-1a, Colon-spezifischem Antigen-p (CSAp), CEA (CEACAM5), CEACAM6, c-met, DAM, EGFR, EGFRvIII, EGP-1, EGP-2, ELF2-M, Ep-CAM, Flt-1, Flt-3, Folatrezeptor, G250-Antigen, GAGE, gp100, GROB, HLA-DR, HM1.24, menschlichem Choriongonadotropin (HCG) und seinen Untereinheiten, HER2/neu, HMGB-1, Hypoxie-induzierbarem Faktor (HIF-1), HSP70-2M, HST-2, Ia, IGF-1R, IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-23, IL-25, Insulin-ähnlichem Wachstumsfaktor-1 (IGF-1), KC4-Antigen, KS-1-Antigen, KS1-4, Le-Y, LDR/FUT, Macrophagenmigrationinhibierendem Faktor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5, MUC13, MUC16, MUM-1/2, MUM-3, NCA66, NCA95, NCA90, Pankreaskrebsmucin, Plazentawachstumsfaktor, p53, PLAGL2, saurer Prostataphosphatase, PSA, PRAME, PSMA, P1GF, ILGF, ILGF-1R, IL-6, IL-25, RS5, RANTES, T101, SAGE, S100, Survivin, Survivin-2B, TAC, TAG-72, Tenascin, TRAIL-Rezeptoren, TNF-α, Tn-Antigen, Thomson-Friedenreich-Antigenen, Tumornekrose-Antigenen, TROP-2, VEGFR, ED-B-Fibronektin, WT-1, 17-1A-Antigen, Komplementfaktoren C3, C3a, C3b, C5a, C5, einem Angiogenese-Marker, bcl-2, bcl-6, Kras, cMET, einem Onkogen-Marker und ein Onkogen-Produkt.

6. DNL-Komplex zur Verwendung nach Anspruch 1, wobei der Antikörper, der an ein Zielantigen bindet, ausgewählt ist aus der Gruppe bestehend aus hPAM4 (anti-Mucin), hA20 (anti-CD20), hA19 (anti-CD19), hIMMU31 (anti-AFP), hLL1 (anti-CD74), hLL2 (anti-CD22), hMu-9 (anti-CSAp), hL243 (anti-HLA-DR), hMN-14 (anti-CEACAM5), hMN-15 (anti-CEACAM6), hRS7 (anti-EGP-1), hMN-3 (anti-CEACAM6), Ab124 (anti-CXCR4), Ab125 (anti-CXCR4), Abciximab (anti-Glycoprotein IIb/IIIa), Alemtuzumab (anti-CD52), Bevacizumab (anti-VEGF), Cetuximab (anti-EGFR), Gemtuzumab (anti-CD33), Ibritumomab (anti-CD20), Panitumumab (anti-EGFR), Rituximab (anti-CD20), Tositumomab (anti-CD20), Trastuzumab (anti-ErbB2), Abagovomab (anti-CA-125), Adecatumumab (anti-EpCAM), Atlizumab (anti-IL-6R), Benralizumab (anti-CD125), CC49 (anti-TAG-72), AB-PG1-XG1-026 (anti-PSMA), D2/B (anti-PSMA), Tocilizumab (anti-IL-6R), Basiliximab (anti-CD25), Daclizumab (anti-CD25), Efalizumab (anti-CD11a), GA101 (anti-CD20), Muromonab-CD3 (anti-CD3-Rezeptor), Natalizumab (anti-α4-Integrin), Omalizumab (anti-IgE), CDP571 (anti-TNF-α), Infliximab (anti-TNF-α), Certolizumab (anti-TNF-α), Adalimumab (anti-TNF-α), Belimumab (anti-B-Zell aktivierenden Faktor), Alz 50 (anti-Tau-protein), Gantenerumab (anti-Amyloid-Protein), Solanezumab (anti-Amyloid-Protein), P4/D10 (anti-gp120), CR6261 (anti-Influenza), Exbivirumab (anti-Hepatitis B), Felvizumab (anti-Respiratory-syncytial-Virus), Foravirumab (anti-Tollwut-Virus), Motavizumab (anti-Respiratory-syncytial-Virus), Palivizumab (anti-Respiratory-syncytial-Virus), Panobacumab (anti-Pseudomonas), Rafivirumab (anti-Tollwut-Virus), Regavirumab (anti-Cytomegalovirus), Sevirumab (anti-Cytomegalovirus), Tivirumab (anti-Hepatitis B), und Urtoxazumab (anti-E. coli).

7. DNL-Komplex zur Verwendung nach Anspruch 1, wobei die fluoreszierende Sonde ausgewählt ist aus der Gruppe bestehend aus Alexa 350, Alexa 430, AMCA, Aminoacridin, BODIPY 630/650, BODIPY 650/665, BODIPY-FL, BODIPY-R6G, BODIPY-TMR, BODIPY-TRX, 5-Carboxy-4,5'-dichlor-2',7'-dimethoxyfluorescein, 5-Carboxy-2',4',5',7'-tetrachlorfluorescein, 5-Carboxyfluorescein, 5-Carboxyrhodamine, 6-Carboxyrhodamine, 6-Carboxytetramethylamino, Cascade Blue, Cy2, Cy3, Cy5,6-FAM, Dansylchlorid, Fluorescein, HEX, 6-JOE, NBD (7-Nitrobenz-2-oxa-1,3-diazol), Oregon Green 488, Oregon Green 500, Oregon Green 514, Pacific Blue, Phthalsäure, Terephthalsäure, Isophthalsäure, Kresyl-Schnellviolett, Kresylblauviolett, Brilliantkresylblau, para-Aminobenzoesäure, Erythrosin, Phthalcyanine, Azomethine, Cyanine, Xanthine, Succinylfluoresceine, Kryptaten seltener Erden, Europium-Trisbipyridindiamin, einem Europiumkryptat oder -Chelat, Diamin, Dicyanine, La Jolla-blauer Farbstoff, Allopycocyanin, allococyanin B, Phycocyanin C, phycocyanin R, Thiamin, Phycoerythrocyanin, Phycoerythrin R, REG, Rhodamingrün, Rhodamin Isothiocyanat, Rhodaminrot, ROX, TAMRA, TET, TRIT (Tetramethylrhodaminisothiol), Tetramethylrhodamin und Texasrot.

8. DNL-Komplex zur Verwendung nach Anspruch 1, wobei die Erkrankung ausgewählt ist aus der Gruppe bestehend aus Non-Hodgkin's Lymphom, akuter lymphoider Leukämie, chronischer lymphoider Leukämie, Burkitt-Lymphom, Hodgkin's Lymphom, Haarzell-Leukämie, akuter myeloischer Leukämie, chronischer myeloischer Leukämie, T-Zelllymphom, T-Zell-Leukämie, multiplem Myelom, Gliom, Waldenström'sche Makroglobulinämie, Karzinom, Melanom, Sarkom, Gliom-und Hautkrebs, wobei beorzugterweise das Karzinom ein Karzinom der Mundhöhle, des Gastrointestinaltraktes, des Lungensystems, der Lunge, der Brust, der Ovarien, der Prostata, des Uterus, des Endometriums, des Cervix, der Harnblase, des Pankreas, des Knochens, des Gehirns, des Bindegewenes, der Leber, der Gallenblase, der Niere, der Haut, des zentralen Nervensystems oder der Hoden ist.

9. DNL-Komplex zur Verwendung nach Anspruch 1, wobei die Erkrankung ausgewählt ist aus der Gruppe bestehend aus akuter Immune-Thrombozytopenie, chronischer Immun-Thrombozytopenie, Dermatomyositis, Sydenham'scher Chorea, Myasthenia gravis, systematischem Lupus erythematodes, Lupusnephritis, rheumatischem Fieber, polyglandulären Syndromen, bullösem Pemphigoid, Pemphigus vulgaris, Type 1-Diabetes, Type 2-Diabetes, Purpura Schoenlein-Hennoch, post-streptococcaler Nephritis, Erythema nodosum, Takayasu-Syndrom, Addison'che Krankheit, rheumatoider Arthritis, multipler Sklerose, Sarkoidose, Colitis ulcerosa, multiformem Erythem, IgA-Nephropathie, Polyarteriitis nodosa, Spondylarthritis ancylopoetica, Goodpasture-Syndrom, Thromboangitis obliterans, Sjögrensches Syndrom, primär biliäre Zirrhose, Hashimoto-Syndrom, Thyreotoxikose, Skleroderma, chronischer aktiver Hepatitis, akuter multipler Muskelentzündung/Dermatomyositis, Polychondritis, Pemphigus vulgaris, Wegener'scher Granulomatose, membranöser Nephropathie, amyotropher Lateralsklerose, Tabes dorsalis, Riesenzellenarteritis/Polymyalgie, perniziöser Anämie, schnellem progessivem nephritische Sydrom, Psoriasis, fibrosierender Alveolitis, Organtransplantatabstoßung und Graft-versus-Host-Disease.

10. DNL-Komplex zur Verwendung nach Anspruch 1, wobei der anti-Hapten-Antikörper und der anti-Zielantigen-Antikörper chimäre, humanisierte oder humane Antikörper sind.

11. DNL-Komplex zur Verwendung nach Anspruch 1, wobei das anti-Hapten-Antikörperfragment und das anti-Zielantigen-Antikörperfragment ausgewählt sind aus der Gruppe bestehend aus F(ab')₂, Fab, scFv oder Fv-Antikörperfragmente.

12. Kit zur Verwendung in einem Verfahren zur Diagnose einer Erkrankung oder eines Zustandes umfassend Nachweisen eines fluoreszenzmarkierten Zielantigens, das mit der Erkrankung oder dem Zustand verbunden ist, durch eine intraoperative, intraperitoneale, laparoskopische, endoskopische oder intravaskulare Prozedur, wobei der Kit umfasst:
a) einen DNL-Komplex umfassend (i) einen anti-Hapten-Antikörper oder ein Antigen-bindendes Fragment davon, konjugiert an einen AD-Teil von einem A-Kinase-Anker-Protein (AKAP); und (ii) einen DDD-Teil von einer menschlichen Protein-Kinase A RIα, RIβ, RIIα oder RIIβ, konjugiert an einen Antikörper oder ein Antigen-bindendes Fragment davon, der/das an das mit der Erkrankung oder dem Zustand verbundende Zielantigen bindet, wobei zwei Kopien des DDD-Teils ein Dimer bilden, das an den AD-Teil bindet, um den DNL-Komplex auszubilden; und
b) ein targetierbares Konstrukt umfassend (iii) wenigstens ein Hapten; und (iv) eine Ffluoreszierende Sonde, wobei das Hapten an den DNL-Komplex bindet.

13. Kit zur Verwendung nach Anspruch 12, wobei das tragetierbare Konstrukt ausgwählt ist aus der Gruppe bestehend aus IMP-448, IMP-449, IMP-460, IMP-461, IMP-462, IMP-467, IMP-468, IMP-470, IMP-485 und IMP-499.

## Revendications

1. Complexe DNL comprenant (i) un anticorps anti-haptène ou l'un de ses fragments de liaison de l'antigène conjugué à une fraction AD issue d'une protéine d'ancrage aux protéines kinases A (AKAP) ; et (ii) une fraction DDD issue de la protéine kinase A humaine RIα, RIβ, RIIα ou RIIβ, conjuguée à un anticorps ou l'un de ses fragments de liaison de l'antigène qui se lie à un antigène cible associé à une maladie ou une affection, dans lequel deux copies de la fraction DDD forment un dimère qui se lie à la fraction AD pour former le complexe DNL, pour une utilisation dans un procédé de diagnostic de la maladie ou de l'affection comprenant la détection de l'antigène cible associé à la maladie ou l'affection, dans lequel le procédé comprend
a. l'exposition de l'antigène cible au complexe DNL ;
b. l'étape consistant à laisser le complexe DNL se lier à l'antigène ;
c. l'ajout d'une construction ciblable comprenant (iii) au moins un haptène ; et (iv) une sonde fluorescente, dans lequel l'haptène se lie au complexe DNL ; et
d. la détection de l'antigène cible marqué par fluorescence par une procédure peropératoire, intrapéritonéale, laparoscopique, endoscopique ou intravasculaire.

2. Complexe DNL pour une utilisation selon la revendication 1, dans lequel la construction ciblable est choisie dans le groupe constitué de IMP-448, IMP-449, IMP-460, IMP-461, IMP-462, IMP-467, IMP-468, IMP-470, IMP-485 et IMP-499.

3. Complexe DNL pour une utilisation selon la revendication 1, dans lequel l'haptène est la HSG ou l'In-DTPA.

4. Complexe DNL pour une utilisation selon la revendication 1, dans lequel l'antigène est un antigène associé à une tumeur, un antigène associé à une maladie auto-immune ou un antigène produit ou présenté par un organisme pathogène.

5. Complexe DNL pour une utilisation selon la revendication 4, dans lequel l'antigène associé à une tumeur est choisi dans le groupe constitué de l'anhydrase carbonique IX, l'alpha-fcetoprotéine, l'α-actinine 4, A3, l'antigène spécifique de l'anticorps A33, ART-4, B7, Ba 733, BAGE, l'antigène BrE3, CA125, CAMEL, CAP-1, CASP-8/m, CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CXCR4, CXCR7, CXCL12, HIF-1a, l'antigène p spécifique du côlon (CSAp), CEA (CEACAM5), CEACAM6, c-met, DAM, EGFR, EGFRvIII, EGP-1, EGP-2, ELF2-M, Ep-CAM, Flt-1, Flt-3, le récepteur des folates, l'antigène G250, GAGE, gp100, GROB, HLA-DR, HM1.24, la gonadotrophine chorionique humaine (HCG) et ses sous-unités, HER2/neu, HMGB-1, le facteur inductible par hypoxie (HIF-1), HSP70-2M, HST-2, Ia, IGF-1R, IFN-γ, IFN-α, IFN-β, IL-2, IL-4R, IL-6R, IL-13R, IL-15R, IL-17R, IL-18R, IL-6, IL-8, IL-12, IL-15, IL-17, IL-18, IL-23, IL-25, le facteur de croissance de type insulinique 1 (IGF-1), l'antigène KC4, l'antigène KS-1, KS1-4, Le-Y, LDR/FUT, le facteur inhibiteur de la migration des macrophages (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5, MUC13, MUC16, MUM-1/2, MUM-3, NCA66, NCA95, NCA90, la mucine du cancer pancréatique, le facteur de croissance placentaire, p53, PLAGL2, la phosphatase acide prostatique, PSA, PRAME, PSMA, PIGF, ILGF, ILGF-1R, IL-6, IL-25, RS5, RANTES, T101, SAGE, S100, la survivine, la survivine 2B, TAC, TAG-72, la ténascine, les récepteurs de TRAIL, TNF-α, l'antigène Tn, les antigènes de Thomson-Friedenreich, les antigènes de nécrose tumorale, TROP-2, VEGFR, la fibronectine ED-B, WT-1, l'antigène 17-1A, les facteurs du complément C3, C3a, C3b, C5a, C5, un marqueur de l'angiogenèse, bcl-2, bcl-6, Kras, cMET, un marqueur oncogène et un produit oncogène.

6. Complexe DNL pour une utilisation selon la revendication 1, dans lequel l'anticorps qui se lie à un antigène cible est choisi dans le groupe constitué de hPAM4 (anti-mucine), hA20 (anti-CD20), hA19 (anti-CD19), hIMMU31 (anti-AFP), hLL1 (anti-CD74), hLL2 (anti-CD22), hMu-9 (anti-CSAp), hL243 (anti-HLA-DR), hMN-14 (anti-CEACAM5), hMN-15 (anti-CEACAM6), hRS7 (anti-EGP-1), hMN-3 (anti-CEACAM6), Ab124 (anti-CXCR4), Ab125 (anti-CXCR4), l'abciximab (anti-glycoprotéine IIb/IIIa), l'alemtuzumab (anti-CD52), le bévacizumab (anti-VEGF), le cétuximab (anti-EGFR), le gemtuzumab (anti-CD33), l'ibritumomab (anti-CD20), le panitumumab (anti-EGFR), le rituximab (anti-CD20), le tositumomab (anti-CD20), le trastuzumab (anti-ErbB2), l'abagovomab (anti-CA-125), l'adécatumumab (anti-EpCAM), l'atlizumab (anti-IL-6R), le benralizumab (anti-CD 125), CC49 (anti-TAG-72), AB-PG1-XG1-026 (anti-PSMA), D2/B (anti-PSMA), le tocilizumab (anti-IL-6R), le basiliximab (anti-CD25), le daclizumab (anti-CD25), l'éfalizumab (anti-CD11a), GA101 (anti-CD20), le muromonab-CD3 (anti-récepteur du CD3), le natalizumab (anti-intégrine α4), l'omalizumab (anti-IgE), CDP571 (anti-TNF-α), l'infliximab (anti-TNF-α), le certolizumab (anti-TNF-α), l'adalimumab (anti-TNF-α), le bélimumab (anti-facteur d'activation des lymphocytes B), Alz 50 (anti-protéine tau), le ganténérumab (anti-protéine amyloïde), le solanezumab (anti-protéine amyloïde), P4/D10 (anti-gp120), CR6261 (antigrippal), l'exbivirumab (anti-hépatite B), le felvizumab (anti-virus respiratoire syncytial), le foravirumab (anti-virus de la rage), le motavizumab (anti-virus respiratoire syncytial), le palivizumab (anti-virus respiratoire syncytial), le panobacumab (anti-Pseudomonas), le rafivirumab (anti-virus de la rage), le régavirumab (anti-cytomégalovirus), le sévirumab (anti-cytomégalovirus), le tivirumab (anti-hépatite B), et l'urtoxazumab (anti-E. coli).

7. Complexe DNL pour une utilisation selon la revendication 1, dans lequel la sonde fluorescente est choisie dans le groupe constitué de Alexa 350, Alexa 430, AMCA, l'aminoacridine, BODIPY 630/650, BODIPY 650/665, BODIPY-FL, BODIPY-R6G, BODIPY-TMR, BODIPY-TRX, la 5-carboxy-4',5'-dichloro-2',7'-diméthoxy-fluorescéine, la 5-carboxy-2',4',5',7'-tétrachlorofluorescéine, la 5- carboxyfluorescéine, la 5-carboxyrhodamine, la 6-carboxyrhodamine, 6-carboxytétraméthyl-amino, Cascade Blue, Cy2, Cy3, Cy5,6-FAM, le chlorure de dansyle, la fluorescéine, HEX, 6-JOE, NBD (7-nitrobenz-2-oxa-1,3-diazole), Oregon Green 488, Oregon Green 500, Oregon Green 514, Pacific Blue, l'acide phtalique, l'acide téréphtalique, l'acide isophtalique, le violet de crésyl acétate, le violet de crésyl bleu, le bleu de crésyl brillant, l'acide para-aminobenzoïque, l'érythrosine, les phtalocyanines, les azométhines, les cyanines, les xanthines, les succinylfluorescéines, les cryptates de métaux de terres rares, l'europium trisbipyridine diamine, un cryptate ou un chélateur d'europium, une diamine, les dicyanines, le colorant bleu de La Jolla, l'allopycocyanine, l'allococyanine B, la phycocyanine C, la phycocyanine R, la thiamine, la phycoérythrocyanine, la phycoérythrine R, REG, la Rhodamine verte, l'isothiocyanate de rhodamine, la Rhodamine rouge, ROX, TAMRA, TET, TRIT (tétraméthyl-rhodamine isothiol), la tétraméthylrhodamine, et le rouge Texas.

8. Complexe DNL pour une utilisation selon la revendication 1, dans lequel la maladie est choisie dans le groupe constitué du lymphome non hodgkinien, la leucémie lymphoïde aiguë, la leucémie lymphoïde chronique, le lymphome de Burkitt, le lymphome de Hodgkin, la leucémie à tricholeucocytes, la leucémie myéloïde aiguë, la leucémie myéloïde chronique, le lymphome à lymphocytes T, la leucémie à lymphocytes T, le myélome multiple, le gliome, la macroglobulinémie de Waldenström, le carcinome, le mélanome, le sarcome, le gliome et le cancer de la peau, dans lequel de préférence le carcinome est un carcinome de la cavité buccale, du tube digestif, du système pulmonaire, du poumon, du sein, de l'ovaire, de la prostate, de l'utérus, de l'endomètre, du col de l'utérus, de la vessie, du pancréas, des os, du cerveau, du tissu conjonctif, du foie, de la vésicule biliaire, du rein, de la peau, du système nerveux central ou des testicules.

9. Complexe DNL pour une utilisation selon la revendication 1, dans lequel la maladie est choisie dans le groupe constitué de l'immunothrombocytopénie aiguë, l'immunothrombocytopénie chronique, la dermatomyosite, la chorée de Sydenham, la myasthénie grave, le lupus érythémateux systémique, la néphrite lupique, le rhumatisme articulaire aigu, les syndromes polyglandulaires, la pemphigoïde bulleuse, le pemphigus vulgaire, le diabète de type 1, le diabète de type 2, le purpura de Henoch-Schonlein, la néphrite post-streptococcique, l'érythème noueux, l'artérite de Takayasu, la maladie d'Addison, la polyarthrite rhumatoïde, la sclérose en plaques, la sarcoïdose, la colite ulcéreuse, l'érythème multiforme, la néphropathie à IgA, la polyartérite noueuse, la spondylarthrite ankylosante, le syndrome de Goodpasture, la thromboangéite oblitérante, le syndrome de Sjögren, la cirrhose biliaire primaire, la thyroïdite de Hashimoto, la thyrotoxicose, la sclérodermie, l'hépatite active chronique, la polymyosite/dermatomyosite, la polychondrite, le pemphigus vulgaire, la granulomatose de Wegener, la néphropathie membraneuse, la sclérose latérale amyotrophique, le tabes dorsalis, l'artérite à cellules géantes/polymyalgie, l'anémie pernicieuse, la glomérulonéphrite à progression rapide, le psoriasis, l'alvéolite fibrosante, le rejet de greffe d'organe et la maladie du greffon contre l'hôte.

10. Complexe DNL pour une utilisation selon la revendication 1, dans lequel l'anticorps anti-haptène et l'anticorps anti-antigène cible sont des anticorps chimériques, humanisés ou humains.

11. Complexe DNL pour une utilisation selon la revendication 1, dans lequel le fragment d'anticorps anti-haptène et le fragment d'anticorps anti-antigène cible sont choisis dans le groupe constitué des fragments F(ab')₂, Fab, scFv ou Fv.

12. Kit pour une utilisation dans un procédé de diagnostic d'une maladie ou d'une affection comprenant la détection d'un antigène cible marqué par fluorescence associée à la maladie ou l'affection par une procédure peropératoire, intrapéritonéale, laparoscopique, endoscopique ou intravasculaire, dans lequel le kit comprend :
a. un complexe DNL comprenant (i) un anticorps anti-haptène ou l'un de ses fragments de liaison de l'antigène conjugué à une fraction AD issue d'une protéine d'ancrage aux protéines kinases A (AKAP) ; et (ii) une fraction DDD issue de la protéine kinase A humaine RIa, RIβ, RIIα ou RIIβ, conjuguée à un anticorps ou l'un de ses fragments de liaison de l'antigène qui se lie à un antigène cible associé à la maladie ou l'affection, dans lequel deux copies de la fraction DDD forment un dimère qui se lie à la fraction AD pour former le complexe DNL ; et
b. une construction ciblable comprenant (iii) au moins un haptène ; et (iv) une sonde fluorescente, dans lequel l'haptène se lie au complexe DNL.

13. Kit pour une utilisation selon la revendication 12, dans lequel la construction ciblable est choisie dans le groupe constitué de IMP-448, IMP-449, IMP-460, IMP-461, IMP-462, IMP-467, IMP-468, IMP-470, IMP-485 et IMP-499.
